# EUROPÄISCHE PATENTSCHRIFT

(11) **EP 0 552 493 B1**
(45) Veröffentlichungstag und Bekanntmachung des Hinweises auf die Patenterteilung: **11.09.1996**
(21) Anmeldenummer: 92121991.1
(22) Anmeldetag: 24.12.1992
(51) Int. Cl.: A01N 25/32, A01N 43/18

(54) **Thiochromenonderivate als Antidots und sie enthaltende herbizide Mittel**
Thiochromenone derivatives as antidotes and herbicidal agents containing them
Dérivés de la thiochromenone comme antidote et agents herbicides les contenant

(30) Priorität: 23.01.1992 DE 4201720
(43) Veröffentlichungstag der Anmeldung: 28.07.1993
(73) Patentinhaber: BASF Aktiengesellschaft, 67063 Ludwigshafen (DE)
(72) Erfinder: Hagen, Helmut, Dr., W-6710 Frankenthal (DE); Raatz, Peter, Dr., W-6700 Ludwigshafen (DE); Walter, Helmut, Dr., W-6719 Obrigheim (DE); Landes, Andreas, Dr., W-6703 Limburgerhof (DE)

(56) Entgegenhaltungen:
- EP-A- 456 069
- EP-A- 456 112
- EP-A- 456 118
- EP-A- 0 012 158
- EP-A- 0 134 198
- EP-A- 0 159 964
- EP-A- 0 459 140
- WO-A-92/10489
- RESEARCH DISCLOSURE, Nr. 147, Juli 1976, HAVANT GB '14712, Herbicidal antidotes'

## Beschreibung

Die vorliegende Erfindung betrifft herbizide Mittel, enthaltend mindestens ein Thiochromenon der Formel I 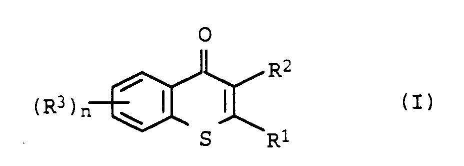 in der die Variablen folgende Bedeutung haben:
n
   1, 2, 3 oder 4, wobei die Reste R³ verschiedene Bedeutungen haben können, wenn n > 1 ist;
R¹
   Wasserstoff; Cyano; Halogen; C₅-C₁₆-Alkyl;
   C₁-C₄-Alkyl, das ein bis fünf Halogenatome oder einen der folgenden Reste tragen kann: Hydroxy, Mercapto, C₁-C₄-Alkoxy, C₁-C₄-Alkylthio, Phenyl, Naphthyl, Thienyl oder Pyridyl, wobei die aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
   Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
   eine Gruppe -XR⁴ oder eine Gruppe -COYR⁴, wobei
   - X: für Sauerstoff, Schwefel oder -NR⁵- steht,
   - Y: für Sauerstoff oder -NR⁵- steht,
   - R⁴: eine der folgenden Gruppen bedeutet:
   Wasserstoff; Formyl; C₁-C₁₆-Alkyl; C₃-C₇-Cycloalkyl; C₁-C₁₆-Alkylcarbonyl; C₃-C₇-Cycloalkylcarbonyl; C₁-C₁₆-Alkylsulfonyl; C₃-C₇-Cycloalkylsulfonyl;
   Phenyl, Naphthyl, Thienyl, Pyridyl, Phenylcarbonyl, Naphthylcarbonyl, Thienylcarbonyl, Pyridylcarbonyl, Phenylsulfonyl, Naphthylsulfonyl, Thienylsulfonyl oder Pyridylsulfonyl, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
   und
   - R⁵: für Wasserstoff; C₁-C₁₆-Alkyl, welches eine Hydroxy- oder C₁-C₄-Alkoxygruppe tragen kann, oder für
   Phenyl, Naphthyl, Thienyl oder Pyridyl steht, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
R²
   Wasserstoff; Cyano; Nitroso; Nitro; Halogen; C₅-C₁₆-Alkyl; C₁-C₄-Alkyl, C₁-C₄-Alkoxy oder C₁-C₄-Alkylthio, wobei diese Gruppen ein bis fünf Halogenatome oder einen der folgenden Reste tragen können: Hydroxy, Mercapto, C₁-C₄-Alkoxy, C₁-C₄-Alkylthio, Phenyl, Naphthyl, Thienyl oder Pyridyl, und wobei die aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
   Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
   eine Gruppe -NR⁴R⁵ oder eine Gruppe -COYR⁴, wobei
Y, R⁴ und R⁵ die vorstehend gegebenen Bedeutungen haben;
R³
   Wasserstoff; Cyano; Halogen; C₅-C₁₆-Alkyl; C₁-C₄-Alkyl, das ein bis fünf Halogenatome oder einen der folgenden Reste tragen kann: Hydroxy, Mercapto, C₁-C₄-Alkoxy, C₁-C₄-Alkylthio, Phenyl, Naphthyl, Thienyl oder Pyridyl, wobei die aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
   Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
   eine Gruppe -YR⁴, eine Gruppe -COYR⁴, eine Gruppe -COR⁶ oder eine Gruppe -SO₂R⁷, wobei
   - Y und R⁴: die vorstehend gegebenen Bedeutungen haben;
   - R⁶: eine der folgenden Gruppen bedeutet:
   Wasserstoff; C₁-C₁₆-Alkyl; C₃-C₇-Cycloalkyl;
   Phenyl, Naphthyl, Thienyl oder Pyridyl, wobei diese aromatischen Reste ihrerseits ein bis fünf Halogenatome und einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
   und
   - R⁷: für Wasserstoff; C₁-C₁₆-Alkyl; C₃-C₇-Cycloalkyl;
   Phenyl, Naphthyl, Thienyl oder Pyridyl steht, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
   oder für eine Gruppe -NR⁴R⁵ steht, in der die Reste R⁴ und R⁵ die vorstehend gegebenen Bedeutungen haben,
   und mindestens einen herbiziden Wirkstoff aus

A) der Gruppe der Cyclohexenon-Derivate der allgemeinen Formel II 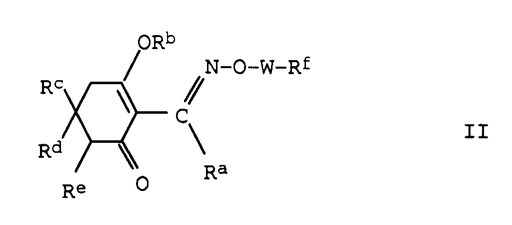 in der die Substituenten folgende Bedeutung haben:
   R^{a}
      eine C₁-C₆-Alkylgruppe;
   R^{b}
      Wasserstoff, das Äquivalent eines landwirtschaftlich brauchbaren Kations, eine C₂-C₈-Alkylcarbonylgruppe, eine C₁-C₁₀-Alkylsulfonylgruppe, eine C₁-C₁₀-Alkylphosphonylgruppe oder die Benzoyl-, Benzolsulfonyl- oder Benzolphosphonylgruppe, wobei die drei letztgenannten Gruppen noch je 1 bis 5 Halogenatome tragen können;
   R^{c}
      Wasserstoff, die Cyanogruppe, die Formylgruppe, eine C₁-C₆-Alkylgruppe, eine C₁-C₄-Alkoxy-C₁-C₆-alkyl- oder C₁-C₄-Alkylthio-C₁-C₆-alkylgruppe, eine Phenoxy-C₁-C₆-alkyl-, Phenylthio-C₁-C₆-alkyl-, Pyridyloxy-C₁-C₆-alkyl- oder Pyridylthio-C₁-C₆-alkylgruppe, wobei die Phenyl- und Pyridylringe jeweils noch einen bis drei Reste tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Alkenyl, C₃-C₆-Alkenyloxy, C₃-C₆-Alkinyl, C₃-C₆-Alkinyloxy und -NR^{g}R^{h}, wobei
      - R^{g}: Wasserstoff, C₁-C₄-Alkyl, C₃-C₆-Alkenyl, C₃-C₆-Alkinyl, C₁-C₆-Acyl oder Benzoyl, das einen bis drei Reste tragen kann, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy und C₁-C₄-Alkylthio und
      - R^{h}: Wasserstoff, C₁-C₄-Alkyl, C₃-C₆-Alkenyl oder C₃-C₆-Alkinyl bedeuten;
   eine C₃-C₇-Cycloalkyl- oder eine C₅-C₇-Cycloalkenylgruppe, wobei diese Gruppen noch einen bis drei Reste tragen können, ausgewählt aus einer Gruppe bestehend aus Hydroxyl, Halogen, C₁-C₄-Alkyl, partiell oder vollständig halogeniertem C₁-C₄-Alkyl, C₁-C₄-Alkoxy, C₁-C₄-Alkylthio, Benzylthio, C₁-C₄-Alkylsulfonyl, C₁-C₄-Alkylsulfenyl und C₁-C₄-Alkylsulfinyl,
   ein 5-gliedriger gesättigter Heterocyclus, der ein oder zwei Sauerstoff- oder Schwefelatome oder ein Sauerstoff- und ein Schwefelatom als Heteroatome enthält, und der noch einen bis drei Reste tragen kann, ausgewählt aus einer Gruppe bestehend aus C₁-C₄-Alkyl, partiell oder vollständig halogeniertem C₁-C₄-Alkyl, C₁-C₄-Alkoxy und C₁-C₄-Alkylthio,
   ein 6- oder 7-gliedriger gesättigter oder ein- oder zweifach ungesättigter Heterocyclus, der ein oder zwei Sauerstoff- oder Schwefelatome oder ein Sauerstoff und ein Schwefelatom als Heteroatome enthält, und der noch einen bis drei Reste tragen kann, ausgewählt aus einer Gruppe bestehend aus Hydroxyl, Halogen, C₁-C₄-Alkyl, partiell oder vollständig halogeniertem C₁-C₄-Alkyl, C₁-C₄-Alkoxy und C₁-C₄-Alkylthio,
   ein 5-gliedriger Heteroaromat, enthaltend ein bis drei Heteroatome, ausgewählt aus einer Gruppe bestehend aus ein oder zwei Stickstoffatomen und einem Sauerstoff- oder Schwefelatom, wobei der Heteroaromat noch einen bis drei Reste tragen kann, ausgewählt aus einer Gruppe bestehend aus Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₂-C₆-Alkenyl, C₂-C₆-Alkenyloxy, C₃-C₆-Alkinyloxy und C₁-C₄-Alkoxy-C₁-C₄-alkyl,
   eine Phenyl- oder Pyridylgruppe, die jeweils noch einen bis drei Reste tragen kann, ausgewählt aus einer Gruppe bestehend aus Nitro, Cyano, Formyl, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Alkenyl, C₃-C₆-Alkenyloxy, C₃-C₆-Alkinyl, C₃-C₆-Alkinyloxy und -NR^{k}R^{l}, wobei R^{k} und R^{l} die oben genannte Bedeutung haben;
   R^{d}
      Wasserstoff, die Hydroxylgruppe oder, wenn R^{c} für eine C₁-C₆-Alkylgruppe steht, eine C₁-C₆-Alkylgruppe;
   R^{e}
      Wasserstoff, Halogen, die Cyanogruppe, eine C₁-C₄-Alkoxycarbonylgruppe oder eine C₁-C₄-Alkylketoximgruppe;
   W
      eine C₁-C₆-Alkylen-, C₃-C₆-Alkenylen- oder C₃-C₆-Alkinylenkette, die jeweils noch ein bis drei Reste tragen können, ausgewählt aus einer Gruppe bestehend aus ein bis drei C₁-C₃-Alkylsubstituenten, ein bis drei Halogenatomen und einem Methylensubstituenten;
   eine C₃-C₆-Alkylen- oder C₄-C₆-Alkenylenkette, die beide noch eine bis drei C₁-C₃-Alkylreste tragen können, wobei jeweils eine Methylengruppe der Ketten durch ein Sauerstoff- oder Schwefelatom, eine Sulfoxid- oder Sulfongruppe oder eine Gruppe -N(Rⁱ)- substituiert sein kann, wobei
   - Rⁱ: für Wasserstoff, C₁-C₄-Alkyl, C₃-C₆-Alkenyl oder C₃-C₆-Alkinyl steht;
   - R^{f}: Wasserstoff; die Vinylgruppe;
   eine Gruppe -CH=CH-Z, wobei Z für Cyano, Halogen, einen C₁-C₄-Alkylrest, einen partiell oder vollständig halogenierten C₁-C₄-Alkylrest, einen C₃-C₆-Cycloalkylrest, der seinerseits noch einen bis drei Substituenten tragen kann, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy; den Carboxylrest, einen C₁-C₈-Alkorycarbonylrest, den Benzyloxycarbonylrest, den Phenyl-, Thienyl- oder Pyridylrest, wobei diese drei aromatischen Reste jeweils noch einen bis drei Substituenten tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Cycloalkyl wobei der Cycloalkylsubstituent seinerseits noch einen bis drei Reste tragen kann, ausgewählt aus einer Gruppe bestehend aus Halogen, C₁-C₄-Alkyl, partiell oder vollständig halogeniertem C₁-C₄-Alkyl und C₁-C₄-Alkoxy, steht;
   die Ethinylgruppe, die einen der folgenden Reste tragen kann: einen C₁-C₄-Alkyl- oder C₃-C₆-Cycloalkylrest, die beide noch einen bis drei Substituenten tragen können, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy, oder den Phenyl-, Thienyl- oder Pyridylrest, wobei die aromatischen Reste jeweils noch einen bis drei Substituenten tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy und C₁-C₄-Alkylthio;
   die Phenylgruppe, eine Halogenphenylgruppe, eine Dihalogenphenylgruppe, eine 5-gliedrige heteroaromatische Gruppe mit ein bis drei Heteroatomen, ausgewählt aus einer Gruppe bestehend aus ein bis drei Stickstoffatomen und einem Sauerstoff- oder Schwefelatom, oder eine 6-gliedrige heteroaromatische Gruppe mit ein bis vier Stickstoffatomen, die nicht alle gleichzeitig benachbart sein können, wobei die Phenyl- und Heteroarylgruppen noch einen bis drei Reste tragen können, ausgewählt aus einer Gruppe bestehend aus Nitro, C₁-C₄-Alkoxyresten, C₁-C₄-Alkylthioresten, partiell oder vollständig halogenierten C₁-C₄-Alkoxyresten, Resten Z oder einem Rest -NR^{k}R^{l}, wobei
   - R^{k}: Wasserstoff, C₁-C₄-Alkyl, C₃-C₆-Alkenyl oder C₃-C₆-Alkinyl und
   - R^{l}: Wasserstoff, C₁-C₄-Alkyl, C₃-C₆-Alkenyl, C₃-C₆-Alkinyl, C₁-C₆-Acyl oder Benzoyl, das noch einen bis drei Substituenten tragen kann, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy und C₁-C₄-Alkylthio,
   bedeuten,
oder
B) der Gruppe der 2-(4-Heteroaryloxy)- oder 2-(4-Aryloxy)-phenoxycarbonsäurederivate der Formel III 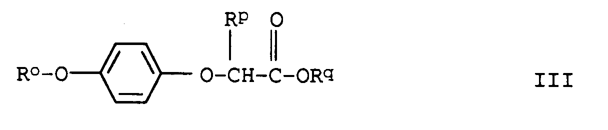 in der die Substituenten folgende Bedeutung haben:
   R^{o}
      die Phenylgruppe, die Pyridylgruppe, eine Benzoxazylgruppe, eine Benzthiazylgruppe oder eine Benzpyrazinylgruppe, wobei diese aromatischen Ringsysteme bis zu zwei der folgenden Reste tragen können: Halogen, Nitro, C₁-C₄-Alkyl, partiell oder vollständig halogeniertes C₁-C₄-Alkyl und/oder partiell oder vollständig halogeniertes C₁-C₄-Alkoxy;
   R^{p}
      Wasserstoff oder die Methylgruppe;
   R^{q}
      Wasserstoff, eine C₁-C₄-Alkylgruppe, eine C₃-C₄-Alkenylgruppe, eine C₃-C₄-Alkinylgruppe, eine C₁-C₄-Alkoxy-C₁-C₄-alkylgruppe, eine C₃-C₄-Alkylideniminooxy-C₂-C₃-alkylgruppe, eine Tetrahydrofuranylmethylgruppe, eine Isoxazolidingruppe oder das Äquivalent eines landwirtschaftlich brauchbaren Kations.

Äquivalent eines landwirtschaftlich brauchbaren Kations.

Außerdem betrifft die Erfindung Verfahren zur selektiven Bekämpfung von unerwünschtem Pflanzenwuchs auf Anbauflächen von Kulturpflanzen mit diesen herbiziden Mitteln.

Aus der Literatur sind Thiochromenone der Formel I, in denen wenigstens einer der Reste R¹, R² oder R³ für eine substituierte Aminogruppe steht, mit pharmazeutischer Wirkung bekannt (EP-A 159,964; GB-A 2,014,991). Die Herstellung von Thiochromenonen der Formel I ist außerdem in einer Arbeit von Bossert et al. (Ann. Chem., Bd. 680, S. 40f (1964)) beschrieben.

Eine antidotische oder antagonistische Wirkung der bekannten Verbindungen in Kombination mit herbiziden Wirkstoffen ist den genannten Druckschriften jedoch nicht zu entnehmen.

Um Kulturpflanzen vor der herbiziden Wirkung von
- Cyclohexenonderivaten aus der Gruppe der Verbindungen II und
- 2-(4-Heteroaryloxy)- oder 2-(4-Aryloxy)-phenoxyessigsäurederivaten aus der Gruppe der Verbindungen III
zu schützen, werden in der EP-A-O 459 140 bestimmte Cyanochinolin-Verbindungen mit antagonistischer Wirkung gelehrt.

Aufgabe der vorliegenden Erfindung war es, herbizide Mittel bereitzustellen, die eine gute Bekämpfung unerwünschter Pflanzen gewährleisten, ohne jedoch die Nutzpflanzen nennenswert zu schädigen oder deren Ernteertrag wesentlich herabzusetzen.

Gemäß dieser Aufgabe wurden die eingangs definierten herbiziden Mittel gefunden.

Des weiteren wurden Verfahren zur Behandlung von Pflanzenkulturen mit den antagonistisch wirksamen Verbindungen I und den Herbiziden II oder den Herbiziden III gefunden, wobei es unerheblich ist, ob die Verbindungen I und II oder I und III gemeinsam oder getrennt formuliert und ausgebracht werden und in welcher Reihenfolge die Applikation bei getrennter Ausbringung erfolgt.

Die herbiziden Mittel enthalten mindestens eine antagonistisch wirksame Verbindung I und mindestens eine Herbizid II oder ein Herbizid III.

Es können jedoch noch weitere antagonistisch oder herbizid wirksame Verbindungen in den erfindungsgemäßen herbiziden Mitteln enthalten sein.

Die Substituenten R¹ bis R³ der substituierten Thiochromenone haben im einzelnen die folgende Bedeutung:
n
   1, 2, 3 oder 4, wobei die Reste R³ verschiedene Bedeutungen haben können, wenn n > 1 ist;
R¹
   Wasserstoff; Cyano;
   Halogen wie Fluor, Chlor, Brom und Jod; unverzweigtes oder verzweigtes C₅-C₁₆-Alkyl; vorzusgweise Chlor, Brom,
   C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl, 2-Methylpropyl,
   wobei diese Gruppe ein bis fünf Halogenatome wie Fluor, Chlor, Brom und Jod, vorzugsweise Fluor, Chlor;
   oder einen der folgenden Reste tragen kann: Hydroxy, Mercapto,
   C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy, Ethoxy, 2-Methylpropyloxy,
   C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Ethylthio, Butylthio,
   Phenyl, Naphthyl, Thienyl oder Pyridyl, wobei die aromatischen Reste ihrerseits ein bis fünf Halogenatome wie Fluor, Chlor, Brom und Jod,
   und/oder einen bis drei der folgenden Substituenten tragen können:
   C₁-C₄-Alkyl wie Methyl, Ethyl, Propyl, 1-Methylethyl, Butyl, 1-Methylpropyl, 2-Methylpropyl und 1,1-Dimethylethyl,
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl,
   C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy, Ethoxy, 2-Methylpropyloxy,
   C₁-C₄-Halogenalkoxy, besonders C₁-C₄-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,
   C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Ethylthio,
   und C₁-C₄-Halogenalkylthio, besonders C₁-C₄-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, Phenyl, Naphthyl, Thienyl oder Pyridyl, wobei diese aromatischen Reste ihrerseits ein bis fünf Halogenatome wie Fluor, Chlor, Brom und Jod, vorzugsweise Fluor, Chlor;
   und/oder einen bis drei der folgenden Substituenten tragen können:
   C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl,
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl,
   C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy, Ethoxy, 2-Methylpropyloxy,
   C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,
   C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio,
   und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio,
   eine Gruppe -XR⁴ oder eine Gruppe -COYR⁴, wobei
   - X: für Sauerstoff, Schwefel und NR⁵ steht,
   - Y: für Sauerstoff und NR⁵ steht,
   - R⁴: eine der folgenden Gruppen bedeutet:
   Wasserstoff; Formyl;
   C₁-C₁₆-Alkyl, besonders C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl, Butyl, 2,2-Dimethylbutyl,
   C₃-C₇-Cycloalkyl wie Cyclopropyl, Cyclobutyl, Cyclopentyl, Cyclohexyl und Cycloheptyl, vorzugsweise Cyclopropyl, Cyclohexyl,
   C₁-C₁₆-Alkylcarbonyl, besonders C₁-C₆-Alkylcarbonyl wie Methylcarbonyl, Ethylcarbonyl, Propylcarbonyl, 1-Methylethylcarbonyl, Butylcarbonyl, 1-Methyl-propylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise Methylcarbonyl, Ethylcarbonyl, Butylcarbonyl,
   C₃-C₇-Cycloalkylcarbonyl wie Cyclopropylcarbonyl, Cyclobutylcarbonyl, Cyclopentylcarbonyl, Cyclohexylcarbonyl und Cycloheptylcarbonyl, vorzugsweise Cyclopropylcarbonyl, Cyclohexylcarbonyl,
   C₁-C₁₆-Alkylsulfonyl, besonders C₁-C₆-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, Hexylsulfonyl, 1,1-Dimethylpropylsulfonyl, 1,2-Dimethylpropylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl, vorzugsweise Methylsulfonyl, Ethylsulfonyl,
   C₃-C₇-Cycloalkylsulfonyl wie Cyclopropylsulfonyl, Cyclobutylsulfonyl, Cyclopentylsulfonyl, Cyclohexylsulfonyl und Cycloheptylsulfonyl,
   Phenyl, Naphthyl, Thienyl, Pyridyl, Phenylcarbonyl, Naphthylcarbonyl, Thienylcarbonyl, Pyridylcarbonyl, Phenylsulfonyl, Naphthylsulfonyl, Thienylsulfonyl oder Pyridylsulfonyl, wobei diese aromatischen Reste ihrerseits
   ein bis fünf Halogenatome wie Fluor, Chlor, Brom und Jod, vorzugsweise Phenyl, Fluor, Chlor;
   und/oder einen bis drei der folgenden Substituenten tragen können:
   C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl,
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl,
   C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy,
   C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,
   C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio,
   und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio,
   und
   - R⁵: für Wasserstoff;
   C₁-C₁₆-Alkyl, besonders C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl, Butyl,
   welches eine Hydroxy- oder C₁-C₄-Alkoxygruppe wie Methoxy, Ethoxy, Propyloxy, 1-Methylethoxy, Butyloxy, 1-Methylpropyloxy, 2-Methylpropyloxy und 1,1-Dimethylethoxy, tragen kann, oder für Phenyl, Naphthyl, Thienyl oder Pyridyl steht, wobei diese aromatischen Reste ihrerseits ein bis fünf Halogenatome wie Fluor, Chlor, Brom und Jod, vorzugsweise Fluor, Chlor;
   und/oder einen bis drei der folgenden Substituenten tragen können:
   C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl, 2-Methylpropyl,
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl,
   Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl,
   C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy, Ethoxy, 2-Methylpropyloxy,
   C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,
   C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio,
   und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio,
R²
   Wasserstoff; Cyano; Nitroso; Nitro;
   Halogen wie Fluor, Chlor, Brom und Jod, vorzugsweise Chlor, Brom;
   unverzweigtes oder verzweigtes C₅-C₁₆-Alkyl;
   C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl, Ethyl,
   C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy,
   oder C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio,
   wobei diese Gruppen ein bis fünf Halogenatome wie Fluor, Chlor, Brom und Jod, vorzugsweise Fluor, Chlor;
   oder einen der folgenden Reste tragen können: Hydroxy, Mercapto, C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy, Ethoxy,
   C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio,
   Phenyl, Naphthyl, Thienyl oder Pyridyl, wobei die aromatischen Reste ihrerseits ein bis fünf Halogenatome wie Fluor, Chlor, Brom und Jod, vorzugsweise Fluor, Chlor;
   und/oder einen bis drei der folgenden Substituenten tragen können:
   C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl,
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl,
   C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy,
   C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,
   C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio,
   und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio,
   Phenyl, Naphthyl, Thienyl oder Pyridyl, wobei diese aromatischen Reste ihrerseits ein bis fünf Halogenatome wie Fluor, Chlor, Brom und Jod, vorzugsweise Fluor, Chlor;
   und/oder einen bis drei der folgenden Substituenten tragen können: C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl,
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl,
   C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy,
   C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,
   C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio,
   und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio,
   eine Gruppe -NR⁴R⁵ oder eine Gruppe -COYR⁴, wobei
   Y, R⁴ und R⁵ im allgemeinen und im besonderen die vorstehend gegebene Bedeutung haben;
R³
   Wasserstoff; Cyano; Halogen wie Fluor, Chlor, Brom und Jod, vorzugsweise Chlor;
   unverzweigtes oder verzweigtes C₅-C₁₆-Alkyl;
   C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl,
   wobei diese Gruppe ein bis fünf Halogenatome wie Fluor, Chlor, Brom und Jod, vorzugsweise Fluor, Chlor;
   oder einen der folgenden Reste tragen kann:
   Hydroxy, Mercapto, C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy, Ethoxy,
   C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio, vorzugsweise Methylthio, Ethylthio,
   Phenyl, Naphthyl, Thienyl oder Pyridyl, wobei die aromatischen Reste ihrerseits ein bis fünf Halogenatome wie Fluor, Chlor, Brom und Jod, vorzugsweise Fluor, Chlor;
   und/oder einen bis drei der folgenden Substituenten tragen können: C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl,
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl,
   C₁-C₄-Alkoxy wie Methoxy, Ethoxy, Propyloxy, 1-Methylethoxy, Butyloxy, 1-Methylpropyloxy, 2-Methylpropyloxy und 1,1-Dimethylethoxy, vorzugsweise Methoxy, 2-Methylpropyloxy,
   C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,
   C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio,
   und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio,
   Phenyl, Naphthyl, Thienyl oder Pyridyl, wobei diese aromatischen Reste ihrerseits ein bis fünf Halogenatome wie Fluor, Chlor, Brom und Jod;
   und/oder einen bis drei der folgenden Substituenten tragen können: C₁-C₄-Alkyl wie Methyl, Ethyl, Propyl, 1-Methylethyl, Butyl, 1-Methylpropyl, 2-Methylpropyl und 1,1-Dimethylethyl;
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
   C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy;
   C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
   C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio;
   und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;
   eine Gruppe -YR⁴, eine Gruppe -COYR⁴, eine Gruppe -COR⁶ oder eine Gruppe -SO₂R⁷, wobei
   Y und R⁴ im allgemeinen und im besonderen die vorstehend gegebene Bedeutung haben;
   - R⁶: eine der folgenden Gruppen bedeutet:
   Wasserstoff;
   C₁-C₁₆-Alkyl, besonders C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl, Butyl,
   C₃-C₇-Cycloalkyl wie Cyclopropyl, Cyclobutyl, Cyclopentyl, Cyclohexyl und Cycloheptyl, vorzugsweise Cyclopropyl, Cyclohexyl,
   Phenyl, Naphthyl, Thienyl oder Pyridyl, wobei diese aromatischen Reste ihrerseits ein bis fünf Halogenatome wie Fluor, Chlor, Brom und Jod, vorzugsweise Fluor, Chlor;
   und/oder einen bis drei der folgenden Substituenten tragen können: C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl,
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl,
   C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy,
   C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,
   C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Methylthio, Ethylthio, Butylthio,
   und C₁-C₄-Halogenalkylthio₁ besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio,
   2,2,2-Trichlorethylthio und Pentafluorethylthio,
   und
   - R⁷: für Wasserstoff;
   C₁-C₁₆-Alkyl, besonders C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl,
   C₃-C₇-Cycloalkyl wie Cyclopropyl, Cyclobutyl, Cyclopentyl, Cyclohexyl und Cycloheptyl,
   Phenyl, Naphthyl, Thienyl oder Pyridyl steht, wobei diese aromatischen Reste ihrerseits ein bis fünf Halogenatome wie Fluor, Chlor, Brom und Jod, vorzugsweise Fluor, Chlor;
   und/oder einen bis drei der folgenden Substituenten tragen können: C₁-C₄-Alkyl wie Methyl, Ethyl, Propyl, 1-Methylethyl, Butyl, 1-Methylpropyl, 2-Methylpropyl und 1,1-Dimethylethyl, vorzugsweise Methyl,
   C₁-C₄-Halogenalkyl, besonders C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl,
   C₁-C₄-Alkoxy wie Methoxy, Ethoxy, Propyloxy, 1-Methylethoxy, Butyloxy, 1-Methyl-propyloxy, 2-Methylpropyloxy und 1,1-Dimethylethoxy,
   C₁-C₄-Halogenalkoxy, besonders C₁-C₂-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy,
   C₁-C₄-Alkylthio wie Methylthio, Ethylthio, Propylthio, 1-Methylethylthio, Butylthio, 1-Methyl-propylthio, 2-Methylpropylthio und 1,1-Dimethylethylthio,
   und C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio, oder für eine Gruppe -NR⁴R⁵ steht, in der die Reste R⁴ und R⁵ im allgemeinen und im besonderen die vorstehend gegebene Bedeutung haben,
   sowie die pflanzenverträglichen Salze derjenigen Verbindungen I, bei denen einer oder mehrere der Substituenten eine saure oder basische Gruppe bedeuten.

Als Antidots zur erfindungsgemäßen Verwendung in herbiziden Mitteln eignen sich besonders Thiochromenone der Formel I in denen der Index n den Wert 1 oder 2 hat.

Besonders geeignet sind auch solche Thiochromenone der Formel I, in denen der Rest R¹ die folgende Bedeutung hat:
Wasserstoff;
Halogen wie vorstehend im allgemeinen und im besonderen genannt;
C₁-C₂-Alkyl, wobei diese Gruppe ein bis fünf Halogenatome wie vorstehend im allgemeinen und im besonderen genannt, oder einen der folgenden Reste tragen kann: C₁-C₂-Alkoxy, C₁-C₂-Alkylthio, Phenyl, Naphthyl, Thienyl oder Pyridyl, und wobei die aromatischen Reste ihrerseits ein bis fünf Halogenatome wie vorstehend im allgemeinen und im besonderen genannt, und/oder einen bis drei der folgenden Substituenten tragen können: C₁-C₂-Alkyl, C₁-C₂-Halogenalkyl wie vorstehend im allgemeinen und im besonderen genannt, C₁-C₂-Alkoxy, C₁-C₂-Halogenalkoxy wie vorstehend im allgemeinen und im besonderen genannt, C₁-C₂-Alkylthio und C₁-C₂-Halogenalkylthio wie vorstehend im allgemeinen und im besonderen genannt;
Phenyl, Naphthyl, Thienyl oder Pyridyl, wobei diese aromatischen Reste ihrerseits ein bis fünf Halogenatome wie vorstehend im allgemeinen und im besonderen genannt, und/oder einen bis drei der folgenden Substituenten tragen können: C₁-C₂-Alkyl, C₁-C₂-Halogenalkyl wie vorstehend im allgemeinen und im besonderen genannt, C₁-C₂-Alkoxy, C₁-C₂-Halogenalkoxy wie vorstehend im allgemeinen und im besonderen genannt, C₁-C₂-Alkylthio und C₁-C₂-Halogenalkylthio wie vorstehend im allgemeinen und im besonderen genannt;
eine Gruppe -XR⁴ oder eine Gruppe -COYR⁴, wobei
- X und Y: für Sauerstoff und NR⁵ stehen,
- R⁴: eine der folgenden Gruppen bedeutet:
Wasserstoff; C₁-C₆-Alkyl wie vorstehend im allgemeinen und im besonderen genannt; C₃-C₇-Cycloalkyl wie vorstehend im allgemeinen und im besonderen genannt;
Phenyl, Naphthyl, Thienyl oder Pyridyl, wobei diese aromatischen Reste ihrerseits ein bis fünf Halogenatome wie vorstehend im allgemeinen und im besonderen genannt, und/oder einen bis drei der folgenden Substituenten tragen können: C₁-C₂-Alkyl, C₁-C₂-Halogenalkyl wie vorstehend im allgemeinen und im besonderen genannt, C₁-C₂-Alkoxy, C₁-C₂-Halogenalkoxy wie vorstehend im allgemeinen und im besonderen genannt, C₁-C₂-Alkylthio und C₁-C₂-Halogenalkylthio wie vorstehend im allgemeinen und im besonderen genannt;
und
- R⁵: für Wasserstoff oder für
C₁-C₆-Alkyl wie vorstehend im allgemeinen und im besonderen genannt, steht, welches eine Hydroxy- oder
C₁-C₄-Alkoxygruppe wie vorstehend im allgemeinen und im besonderen genannt, tragen kann.

Außerdem werden solche Thiochromenone der Formel I bevorzugt, in denen der Rest R² die folgende Bedeutung hat:
Wasserstoff;
Halogen wie vorstehend im allgemeinen und im besonderen genannt,
C₁-C₂-Alkyl, C₁-C₂-Alkoxy oder C₁-C₂-Alkylthio, wobei diese Gruppen ein bis fünf Halogenatome wie vorstehend im allgemeinen und im besonderen genannt, oder einen der folgenden Reste tragen können: C₁-C₂-Alkoxy, C₁-C₂-Alkylthio, Phenyl, Naphthyl, Thienyl oder Pyridyl, und wobei die aromatischen Reste ihrerseits ein bis fünf Halogenatome und/oder einen bis drei der folgenden Substituenten tragen können: C₁-C₂-Alkyl, C₁-C₂-Halogenalkyl wie vorstehend im allgemeinen und im besonderen genannt, C₁-C₂-Alkoxy, C₁-C₂-Halogenalkoxy wie vorstehend im allgemeinen und im besonderen genannt, C₁-C₂-Alkylthio und C₁-C₂-Halogenalkylthio wie vorstehend im allgemeinen und im besonderen genannt;
Phenyl, Naphthyl, Thienyl oder Pyridyl, wobei diese aromatischen Reste ihrerseits ein bis fünf Halogenatome wie vorstehend im allgemeinen und im besonderen genannt, und/oder einen bis drei der folgenden Substituenten tragen können: C₁-C₂-Alkyl, C₁-C₂-Halogenalkyl wie vorstehend im allgemeinen und im besonderen genannt, C₁-C₂-Alkoxy, C₁-C₂-Halogenalkoxy wie vorstehend im allgemeinen und im besonderen genannt, C₁-C₂-Alkylthio und C₁-C₂-Halogenalkylthio, wie vorstehend im allgemeinen und im besonderen genannt;
eine Gruppe -NR⁴R⁵ oder eine Gruppe -COYR⁴, wobei
- Y: für Sauerstoff und NR⁵ steht,
- R⁴: eine der folgenden Gruppen bedeutet:
Wasserstoff;
C₁-C₆-Alkyl wie vorstehend im allgemeinen und im besonderen genannt;
C₃-C₇-Cycloalkyl wie vorstehend im allgemeinen und im besonderen genannt;
Phenyl, Naphthyl, Thienyl oder Pyridyl, wobei diese aromatischen Reste ihrerseits ein bis fünf Halogenatome wie vorstehend im allgemeinen und im besonderen genannt, und/oder einen bis drei der folgenden Substituenten tragen können: C₁-C₂-Alkyl, C₁-C₂-Halogenalkyl wie vorstehend im allgemeinen und im besonderen genannt, C₁-C₂-Alkoxy, C₁-C₂-Halogenalkoxy wie vorstehend im allgemeinen und im besonderen genannt, C₁-C₂-Alkylthio und C₁-C₂-Halogenalkylthio wie vorstehend im allgemeinen und im besonderen genannt;
und
- R⁵: für Wasserstoff oder für
C₁-C₆-Alkyl wie vorstehend im allgemeinen und im besonderen genannt, steht, welches eine Hydroxy- oder C₁-C₄-Alkoxygruppe wie vorstehend im allgemeinen und im besonderen genannt, tragen kann.

Des weiteren sind im Hinblick auf ihre Verwendung als Antidots auch solche Thiochromenon Derivate der Formel I bevorzugt, in denen der Rest R³ die folgende Bedeutung hat:

Wasserstoff; Halogen wie vorstehend im allgemeinen und im besonderen genannt;

C₁-C₄-Alkyl wie vorstehend im allgemeinen und im besonderen genannt, wobei diese Gruppe ein bis fünf Halogenatome wie vorstehend im allgemeinen und im besonderen genannt, oder einen der folgenden Reste tragen kann: C₁-C₂-Alkoxy, C₁-C₂-Alkylthio, Phenyl, Naphthyl, Thienyl oder Pyridyl, und wobei die aromatischen Reste ihrerseits ein bis fünf Halogenatome wie vorstehend im allgemeinen und im besonderen genannt, und/oder einen bis drei der folgenden Substituenten tragen können: C₁-C₂-Alkyl, C₁-C₂-Halogenalkyl wie vorstehend im allgemeinen und im besonderen genannt, C₁-C₂-Alkoxy, C₁-C₂-Halogenalkoxy wie vorstehend im allgemeinen und im besonderen genannt, C₁-C₂-Alkylthio und C₁-C₂-Halogenalkylthio wie vorstehend im allgemeinen und im besonderen genannt;
eine Gruppe -YR⁴, eine Gruppe -COYR⁴, eine Gruppe -COR⁶ oder eine Gruppe -SO₂R⁷, wobei
- Y: für Sauerstoff oder -NR⁵- steht,
- R⁴: eine der folgenden Gruppen bedeutet:
Wasserstoff;
C₁-C₆-Alkyl wie vorstehend im allgemeinen und im besonderen genannt;
C₃-C₇-Cycloalkyl wie vorstehend im allgemeinen und im besonderen genannt;
Phenyl, Naphthyl, Thienyl oder Pyridyl, wobei diese aromatischen Reste ihrerseits ein bis fünf Halogenatome wie vorstehend im allgemeinen und im besonderen genannt, und/oder einen bis drei der folgenden Substituenten tragen können: C₁-C₂-Alkyl, C₁-C₂-Halogenalkyl wie vorstehend im allgemeinen und im besonderen genannt, C₁-C₂-Alkoxy, C₁-C₂-Halogenalkoxy wie vorstehend im allgemeinen und im besonderen genannt, C₁-C₂-Alkylthio und C₁-C₂-Halogenalkylthio wie vorstehend im allgemeinen und im besonderen genannt;
- R⁵: für Wasserstoff oder für
C₁-C₆-Alkyl wie vorstehend im allgemeinen und im besonderen genannt, steht, welches eine Hydroxy- oder C₁-C₄-Alkoxygruppe tragen kann;
- R⁶: eine der folgenden Gruppen bedeutet:
Wasserstoff;
C₁-C₆-Alkyl wie vorstehend im allgemeinen und im besonderen genannt;
C₃-C₇-Cycloalkyl wie vorstehend im allgemeinen und im besonderen genannt;
Phenyl, Naphthyl, Thienyl oder Pyridyl, wobei diese aromatischen Reste ihrerseits ein bis fünf Halogenatome wie vorstehend im allgemeinen und im besonderen genannt, und/oder einen bis drei der folgenden Substituenten tragen können: C₁-C₂-Alkyl, C₁-C₂-Halogenalkyl, wie vorstehend im allgemeinen und im besonderen genannt, C₁-C₂-Alkoxy, C₁-C₂-Halogenalkoxy, wie vorstehend im allgemeinen und im besonderen genannt, C₁-C₂-Alkylthio und C₁-C₂-Halogenalkylthio, wie vorstehend im allgemeinen und im besonderen genannt,
und
- R⁷: für Wasserstoff;
C₁-C₆-Alkyl wie vorstehend im allgemeinen und im besonderen genannt;
C₃-C₇-Cycloalkyl wie vorstehend im allgemeinen und im besonderen genannt;
Phenyl, Naphthyl, Thienyl oder Pyridyl steht, wobei diese aromatischen Reste ihrerseits ein bis fünf Halogenatome wie vorstehend im allgemeinen und im besonderen genannt,
und/oder einen bis drei der folgenden Substituenten tragen können: C₁-C₂-Alkyl, C₁-C₂-Halogenalkyl, wie vorstehend im allgemeinen und im besonderen genannt, C₁-C₂-Alkoxy, C₁-C₂-Halogenalkoxy, wie vorstehend im allgemeinen und im besonderen genannt, C₁-C₂-Alkylthio und C₁-C₂-Halogenalkylthio wie vorstehend im allgemeinen und im besonderen genannt;
oder für eine Gruppe -NR⁴R⁵ steht, in der die Reste R⁴ und R⁵ die vorstehend gegebene Bedeutung haben.

Insbesondere werden für die Verwendung in herbiziden Mitteln solche Thiochromenone der Formel I bevorzugt, in denen der Index n den Wert 1 oder 2 hat und die Substituenten die folgende Bedeutung haben:
R¹
   Wasserstoff;
   Halogen wie vorstehend im allgemeinen und im besonderen genannt;
   C₁-C₂-Alkyl, wobei diese Gruppe ein bis fünf Halogenatome wie vorstehend im allgemeinen und im besonderen genannt, oder eine C₁-C₂-Alkoxygruppe tragen kann;
   Phenyl, welches ein bis fünf Halogenatome wie vorstehend im allgemeinen und im besonderen genannt, und/oder einen bis drei der folgenden Substituenten tragen kann: C₁-C₂-Alkyl, C₁-C₂-Alkoxy und C₁-C₂-Alkylthio;
   eine Gruppe -XR⁴ oder eine Gruppe -COYR⁴, wobei
   - X und Y: für Sauerstoff und NR⁵ stehen,
   - R⁴: eine der folgenden Gruppen bedeutet:
   Wasserstoff;
   C₁-C₆-Alkyl wie vorstehend im allgemeinen und im besonderen genannt;
   C₃-C₇-Cycloalkyl wie vorstehend im allgemeinen und im besonderen genannt;
   Phenyl, welches ein bis fünf Halogenatome wie vorstehend im allgemeinen und im besonderen genannt, und/oder einen bis drei der folgenden Substituenten tragen kann: C₁-C₂-Alkyl, C₁-C₂-Alkoxy und C₁-C₂-Alkylthio;
   und
   - R⁵: für Wasserstoff oder für C₁-C₆-Alkyl wie vorstehend im allgemeinen und im besonderen genannt, steht;
R²
   Wasserstoff;
   Halogen wie vorstehend im allgemeinen und im besonderen genannt;
   C₁-C₂-Alkyl oder C₁-C₂-Alkoxy, wobei diese Gruppen ein bis fünf Halogenatome oder einen C₁-C₂-Alkoxyrest tragen können; Phenyl, welches ein bis fünf Halogenatome wie vorstehend im allgemeinen und im besonderen genannt, und/oder einen bis drei der folgenden Substituenten tragen kann: C₁-C₂-Alkyl, C₁-C₂-Alkoxy und C₁-C₂-Alkylthio;
   eine Gruppe -NR⁴R⁵ oder eine Gruppe -COYR⁴, wobei
   Y, R⁴ und R⁵ die vorstehend gegebene Bedeutung haben,
R³
   Wasserstoff;
   Halogen wie vorstehend im allgemeinen und im besonderen genannt;
   C₁-C₄-Alkyl wie vorstehend im allgemeinen und im besonderen genannt, wobei diese Gruppe ein bis fünf Halogenatome wie vorstehend im allgemeinen und im besonderen genannt, oder einen C₁-C₂-Alkoxyrest tragen kann;
   eine Gruppe -YR⁴, eine Gruppe -COYR⁴, eine Gruppe -COR⁶ oder eine Gruppe -SO₂R⁷, wobei
   - Y und R⁴: die vorstehend gegebene Bedeutung haben, und
   - R⁶ und R⁷: unabhängig voneinander für die folgenden Gruppen stehen:
   C₁-C₆-Alkyl wie vorstehend im allgemeinen und im besonderen genannt;
   C₃-C₇-Cycloalkyl wie vorstehend im allgemeinen und im besonderen genannt;
   Phenyl, welches ein bis fünf Halogenatome wie vorstehend im allgemeinen und im besonderen genannt, und/oder einen bis drei der folgenden Substituenten tragen kann: C₁-C₂-Alkyl, C₁-C₂-Alkoxy und C₁-C₂-Alkylthio.

Die substituierten Thiochromenone der Formel I eignen sich als Antidots, um herbizide Wirkstoffe für Kulturpflanzen wie Kulturhirse, Reis, Mais, Getreidearten (Weizen, Roggen, Gerste, Hafer), Baumwolle, Zuckerrüben, Zuckerrohr und Soja verträglicher zu machen. Sie wirken antagonistisch auf Herbizide verschiedenster Stoffklassen wie Triazine, Phenylharnstoffderivate, Carbamate, Thiolcarbamate, Halogenacetanilide, Benzoesäurederivate sowie insbesondere Halogenphenoxyessigsäureester, substituierte Phenoxyphenoxyessigsäureester, Phenoxyphenoxypropionsäureester und Cyclohexenonderivate.

Herbizid wirksame Cyclohexenon-Derivate II sind beispielsweise aus EP-A 228 598, EP-A 230 235, EP-A 238 021, EP-A 368 227, US-A 4 432 786, DE-A 24 39 104, EP-A 456 068, EP-A 456 069, EP-A 456 112 und EP-A 456 118 bekannt oder können nach den in diesen Schriften beschriebenen Verfahren erhalten werden. Sie dienen vorwiegend zur Bekämpfung unerwünschter Gräser in dicotylen Kulturen und in Gräsern, die nicht zur Familie der Gramineen zählen. In Abhängigkeit der Substituenten und der Dosierung der Verbindungen des Typs II bei ihrer Anwendung können diese Cyclohexenone auch zur selektiven Bekämpfung von unerwünschten Gräsern in Gramineen-Kulturen wie Weizen und Reis eingesetzt werden.

Als herbizide Wirkstoffe II kommen sowohl die reinen Enantiomeren als auch Racemate oder Diastereoisomerengemische von Cyclohexanon-Derivaten II in Betracht.

Die Cyclohexenon-Derivate II können in mehreren tautomeren Formen geschrieben werden, die alle von der Erfindung umfaßt werden.

Im Hinblick auf die bestimmungsgemäße Verwendung der erfindungsgemäßen herbiziden Mittel kommen Cyclohexenonderivate der Formel II in Betracht, wobei die Substituenten im einzelnen folgende Bedeutung haben:
R^{a}
   eine unverzweigte oder verzweigte C₁-C₆-Alkylgruppe wie Methyl, Ethyl, Propyl, Isopropyl, n-Butyl, sec. -Butyl, i-Butyl, tert.-Butyl, n-Pentyl oder n-Hexyl, insbesondere Ethyl und Propyl;
R^{b}
   Wasserstoff;
   das Äquivalent eines landwirtschaftlich brauchbaren Kations, beispielweise ein Alkalimetallkation wie Natrium oder Kalium, ein Äquivalent eines Erdalkalimetallkations wie Calzium, Magnesium und Barium, Mangan-, Kupfer-, Zink- oder Eisenkationen, Ammoniumkationen mit gewünschtenfalls einem bis drei Substituenten, ausgewählt aus einer Gruppe bestehend aus drei C₁-C₄-Alkylresten, drei Hydroxy-C₁-C₄-alkylresten, und einem Phenyl- oder Benzylrest, wie Tetraalkyl- und Benzyltrialkylammoniumkationen, Phosphoniumkationen, Sulfoniumkationen wie Trialkylsulfoniumkationen oder Sulfoxoniumkationen;
   eine C₂-C₈-Alkylcarbonyloxygruppe wie Methylcarbonyloxy, Ethylcarbonyloxy, n-Propylcarbonyloxy, 1-Methylethylcarbonsäure, n-Butylcarbonyloxy, 1-Methylpropylcarbonyloxy, 2-Methylpropylcarbonyloxy, 1,1-Dimethylethylcarbonyloxy, n-Pentylcarbonyloxy, 1-Methylbutylcarbonyloxy, 2-Methylbutylcarbonyloxy, 3-Methylbutylcarbonyloxy, 1,1-Dimethylpropylcarbonyloxy, 1,2-Dimethylpropylcarbonyloxy, 2,2-Dimethylpropylcarbonyloxy, 1-Ethylpropylcarbonyloxy, n-Hexylcarbonyloxy, 1-Methylpentylcarbonyloxy, 2-Methylpentylcarbonyloxy, 3-Methylpentylcarbonyloxy, 4-Methylpentylcarbonyloxy, 1,1-Dimethylbutylcarbonyloxy, 1,2-Dimethylbutylcarbonyloxy, 1,3-Dimethylbutylcarbonyloxy, 2,2-Dimethylbutylcarbonyloxy, 2,3-Dimethylbutylcarbonyloxy, 3,3-Dimethylbutylcarbonyloxy, 1-Ethylbutylcarbonyloxy, 2-Ethylbutylcarbonyloxy, 1,1,2-Trimethylpropylcarbonyloxy, 1,2,2-Trimethylpropylcarbonyloxy, 1-Ethyl-1-methylpropylcarbonyloxy und 1-Ethyl-2-methylpropylcarbonyloxy;
   die Benzoylgruppe oder ein am Phenylkern durch ein bis fünf Halogenatome substituiertes Derivat;
   eine unverzweigte oder verzweigte C₁-C₁₀-Alkylsulfonylgruppe, insbesondere eine C₁-C₆-Alkylsulfonylgruppe wie Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl, 1,1-Dimethylethylsulfonyl, n-Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, n-Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
   die Benzolsulfonylgruppe und ein am Phenylkern durch ein bis fünf Halogenatome substituiertes Derivat;
   eine unverzweigte oder verzweigte C₁-C₁₀-Alkylphosphonylgruppe, insbesondere eine C₁-C₆-Alkylphosphonylgruppe wie Methylphosphonyl, Ethylphosphonyl, n-Propylphosphonyl, 1-Methylethylphosphonyl, n-Butylphosphonyl, 1-Methylpropylphosphonyl, 2-Methylpropylphosphonyl, 1,1-Dimethylethylphosphonyl, n-Pentylphosphonyl, 1-Methylbutylphosphonyl, 2-Methylbutylphosphonyl, 3-Methylbutylphosphonyl, 1,1-Dimethylpropylphosphonyl, 1,2-Dimethylpropylphosphonyl, 2,2-Dimethylpropylphosphonyl, 1-Ethylpropylphosphonyl, Hexylphosphonyl, 1-Methylpentylphosphonyl, 2-Methylpentylphosphonyl, 3-Methylpentylphosphonyl, 4-Methylpentylphosphonyl, 1,1-Dimethylbutylphosphonyl, 1,2-Dimethylhutylphosphonyl, 1,3-Dimethylbutyphosphonyl, 2,2-Dimethylbutylphosphonyl, 2,3-Dimethylbutylphosphonyl, 3,3-Dimethylbutylphosphonyl, 1-Ethylbutylphosphonyl, 2-Ethylbutyphosphonyl, 1,1,2-Trimethylpropylphosphonyl, 1,2,2-Trimethylpropylphosphonyl, 1-Ethyl-1-methylpropylphosphonyl und 1-Ethyl-2-methylpropylphosphonyl;
   die Benzolphosphylgruppe oder ein am Phenylkern durch ein bis fünf Halogenatome substituiertes Derivat;
R^{c}
   Wasserstoff; die Cyanogruppe; die Formylgruppe;
   eine unverzweigte oder verzweigte C₁-C₆-Alkylgruppe wie vorstehend genannt, insbesondere Ethyl, n-Propyl und Isopropyl;
   eine C₁-C₄-Alkoxy-C₁-C₄-alkylgruppe wie Methoxymethyl, Ethoxymethyl Propoxymethyl, 1-Methoxyethyl, 2-Methoxyethyl, 1-Ethoxyethyl, 2-Ethoxyethyl, 2-Propoxyethyl, 1-Methoxypropyl, 2-Methoxypropyl, 3-Methoxypropyl, 1-Ethoxypropyl, 2-Ethoxypropyl, 3-Ethoxypropyl, 1-Methoxybutyl, 2-Methoxybutyl, 3-Methoxybutyl, 4-Methoxybutyl, 1-Ethoxybutyl, 2-Ethoxybutyl, 3-Ethoxybutyl, 4-Ethoxybutyl, 1,3-Dimethoxypropyl, vorzugsweise Methoxymethyl und 2-Ethoxy-ethyl;
   eine C₁-C₄-Alkylthio-C₁-C₄-alkylgruppe wie Methylthiomethyl, Ethylthiomethyl, 1-Methylthioethyl, 2-Methylthioethyl, 1-Ethylthioethyl, 2-Ethylthioethyl, 1-Methylthiopropyl, 2-Methylthiopropyl, 3-Methylthiopropyl, 1-Ethylthiopropyl, 2-Ethylthiopropyl, 3-Ethylthiopropyl, 1-Methylthiobutyl, 2-Methylthiobutyl, 3-Methylthiobutyl, 4-Methylthiobutyl, 1-Ethylthiobutyl, 2-Ethylthiobutyl, 3-Ethylthiobutyl, 4-Ethylthiobutyl, vorzugsweise 2-Ethylthiopropyl;
   eine Phenoxy-C₁-C₆-alkyl-, Phenylthio-C₁-C₆-alkyl-, Pyridyloxy-C₁-C₆-alkyl- oder Pyridylthio-C₁-C₆-alkylgruppe, wobei die Phenyl- und Pyridylringe noch einen bis drei Reste tragen können, ausgewählt aus einerGruppe, bestehend aus Nitro, Cyano, Halogen wie Fluor, Chlor, Brom und Jod, insbesondere Fluor und Chlor, unverzweigtem oder verzweigtem C₁-C₄-Alkyl wie Methyl, Ethyl, n-Propyl, Isopropyl, n-Butyl und tert.-Butyl, partiell oder vollständig halogeniertem C₁-C₄-Alkyl, insbesondere C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl, C₁-C₄-Alkoxy wie Methoxy, Ethoxy, n-Propoxy, Isopropoxy, n-Butoxy und tert.-Butoxy, vorzugsweise Methoxy und Ethoxy, partiell oder vollständig halogeniertem C₁-C₄-Alkoxy, insbesondere C₁-C₂-Halogenalkoxy wie Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy und Pentafluorethyloxy, insbesondere Trifluormethoxy, C₁-C₄-Alkylthio wie Methylthio, Ethylthio, n-Propylthio, Isopropylthio, n-Butylthio und tert.-Butylthio, vorzugsweise Methylthio und Ethylthio, C₃-C₆-Alkenyl, wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, vorzugsweise Prop-2-enyl und But-3-enyl, C₃-C₆-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 3,3-Dimethyl-2-butenyloxy, 1-Ethyl-2-butenyloxyoxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy, vorzugsweise Prop-2-enyloxy und But-2-enyloxy, C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 4-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl,1-Ethyl-3-butinyl, 2-Ethyl-3-butinyl und 1-Ethyl-1-methyl-2-propinyl, vorzugsweise Prop-2-inyl, C₃-C₆-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, vorzugsweise Prop-2-inyloxy,
   oder eine Gruppe NR^{g}R^{h}, wobei
R^{h}
   Wasserstoff, unverzweigtem oder verzweigtes C₁-C₄-Alkyl wie vorstehend genannt, C₃-C₆-Alkenyl wie vorstehend genannt, oder C₃-C₆-Alkinyl wie vorstehend genannt, vorzugsweise Wasserstoff,
   und
Rg
   Wasserstoff, unverzweigte oder verzweigtes C₁-C₄-Alkyl wie vorstehend genannt, C₃-C₆-Alkenyl wie vorstehend genannt,
   C₃-C₆-Alkinyl wie vorstehend genannt, C₁-C₆-Acyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, insbesondere Methylcarbonyl und 1,1-Dimethylethylcarbonyl;
   oder
   Benzoyl, das noch einen bis drei Substituenten tragen kann, ausgewählt aus einer Gruppe bestehend aus Nitro, Cyano, Halogen wie vorstehend genannt, insbesondere Fluor und Chlor, unverzweigte oder verzweigtes C₁-C₄-Alkyl wie vorstehend genannt, partiell oder vollständig halogeniertem C₁-C₄-Alkyl, insbesondere C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl;
   C₁-C₄-Alkoxy wie vorstehend genannt und C₁-C₄-Alkylthio wie vorstehend genannt,
   bedeuten;
   besonders bevorzugte Phenoxy-C₁-C₆-alkyl-, Phenylthio-C₁-C₆-alkyl-, Pyridyloxy-C₁-C₆-alkyl- oder Pyridylthio-C₁-C₆-alkylgruppen sind Phenoxymethyl, Phenoxyethyl, Phenoxypropyl, Phenoxybutyl, 4-Fluorphenoxy-ethyl, 2-(4-Fluorphenoxy)-propyl, 4-Trifluormethylphenoxy-ethyl, 2-(4-Trifluormethylphenoxy)-propyl, Phenylthiomethyl, Phenylthioethyl, Phenylthiopropyl, Phenylthiobutyl, 4-Fluorphenylthio-ethyl, 2-(4-Fluorphenylthio)propyl, 4-Trifluormethylphenylthio-ethyl, 2-(4-Trifluormethylphenylthio)propyl, insbesondere 4-Fluorphenylthio-ethyl und 4-Trifluormethylphenylthio-ethyl;
   eine C₃-C₇-Cycloalkyl- oder C₅-C₇-Cycloalkenylgruppe wie Cyclopropyl, Cyclobutyl, Cyclopentyl, Cyclohexyl, Cycloheptyl, Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl und Cyclohept-4-enyl, wobei die Carbocyclen jeweils noch einen bis drei Reste tragen können, ausgewählt aus einer Gruppe bestehend aus Hydroxyl, Halogen wie vorstehend genannt, insbesondere Fluor und Chlor, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, C₁-C₄-Alkylthio wie vorstehend genannt, insbesondere Methylthio, Ethylthio, Benzylthio, C₁-C₄-Alkylsulfonyl, wie Methylsulfonyl, Ethylsulfonyl, n-Propylsulfonyl, 1-Methylethyl-sulfonyl, n-Butylsulfonyl, 1-Methylpropylsulfonyl, 2-Methylpropylsulfonyl und 1,1-Dimethylethylsulfonyl, vorzugsweise Methylsulfonyl, sowie C₁-C₄-Alkylsulfinyl wie Methylsulfinyl, Ethylsulfinyl, Propylsulfinyl, 1-Methylethyl-sulfinyl, Butylsulfinyl, 1-Methylpropylsulfinyl, 2-Methylpropylsulfinyl und 1,1-Dimethylethylsulfinyl, vorzugsweise Methylsulfinyl,
   unter den substituierten C₃-C₇-Cycloalkyl- und C₅-C₇-Cycloalkenylgruppen sind 1-Methylthiocyclopropyl, 1-Ethylthiocyclopropyl, 4-Methylcyclohexyl, 4-Methylcyclohex-3-enyl, 3-Ethylthio-4-hydroxy-4-methylcyclohexyl, 3,4-Dihydroxycyclohexyl, insbesondere 1-Methylthiocyclopropyl, 1-Ethylthiocyclopropyl und 3,4-Dihydroxycyclohexyl, besonders bevorzugt;
   5-gliedriges Heterocycloalkyl wie Tetrahydrofuran-2-yl, Tetrahydrofuran-3-yl, Tetrahydrothien-2-yl, Tetrahydrothien-3-yl, Pyrrolidin-2-yl, Pyrrolidin-3-yl, Isoxazolidin-3-yl, Isoxazolidin-4-yl, Isoxazolidin-5-yl, Isothiazolidin-3-yl, Isothiazolidin-4-yl, Isothiazolidin-5-yl, Pyrazolidin-3-yl, Pyrazolidin-4-yl, Pyrazolidin-5-yl, Oxazolidin-2-yl, Oxazolidin-4-yl, Oxazolidin-5-yl, Thiazolidin-2-yl, Thiazolidin-4-yl, Thiazolidin-5-yl, Imidazolidin-2-yl, Imidazolidin-4-yl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin2-yl, 1,3,4-Thiadiazolidin-2-yl, Dioxolanyl, Dithiolanyl, insbesondere Tetrahydrofuran-2-yl, Tetrahydrofuran-3-yl und Dioxolanyl, wobei die Heterocyclen jeweils noch einen bis drei Reste tragen können, ausgewählt aus einer Gruppe bestehend aus unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, und C₁-C₄-Alkylthio wie vorstehend genannt, insbesondere Methylthio;
   ein 6- oder 7-gliedriger Heterocyclus wie Tetrahydrothiopyran-2-yl, Tetrahydrothiopyran-3-yl, Tetrahydrothiopyran-4-yl, Tetrahydropyran-2-yl, Tetrahydropyran-3-yl, Tetrahydropyran-4-yl, 5,6-Dihydro-2H-thiopyran-2-yl, 5,6-Dihydro-2H-thiopyran-3-yl, 5,6-Dihydro-2H-thiopyran-4-yl, 5,6-Dihydro-2H-thiopyran-5-yl, 5,6-Dihydro-2H-thiopyran-6-yl, 5,6-Dihydro-2H-pyran-2-yl, 5,6-Dihydro-2H-pyran-3-yl, 5,6-Dihydro-2H-pyran-4-yl, 5,6-Dihydro-2H-pyran-5-yl, 5,6-Dihydro-2H-pyran-6-yl und Dioxepan-5-yl, insbesondere Tetrahydrothiopyran-3-yl, Tetrahydropyran-3-yl und Tetrahydropyran-4-yl, wobei die Heterocyclen jeweils noch einen bis drei Reste tragen können, ausgewählt aus einer Gruppe bestehend aus Hydroxyl, Halogen wie vorstehend genannt, insbesondere Chlor und Brom, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, und C₁-C₄-Alkylthio wie vorstehend genannt, insbesondere Methylthio und Ethylthio;
   ein 5-gliedriger Heteroaromat, enthaltend ein bis drei Heteroatome, ausgewählt aus einer Gruppe bestehend aus ein oder zwei Stickstoffatomen und einem Sauerstoff- oder Schwefelatom, beispielsweise 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 5-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, insbesondere Isoxazolyl und Pyrazolyl, wobei der Heteroaromat noch einen bis drei Reste tragen kann, ausgewählt aus einer Gruppe bestehend aus Cyano, Halogen wie vorstehend genannt, insbesondere Fluor und Chlor, unverzweigtem oder verzweigtem C₁-C₄-Alkyl wie vorstehend genannt, insbesondere Methyl, Ethyl und Isopropyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, partiell oder vollständig halogeniertem C₁-C₄-Alkoxy wie vorstehend genannt, insbesondere Trifluormethoxy, C₁-C₄-Alkylthio wie vorstehend genannt, insbesondere Methylthio, C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere 1-Methylethenyl, C2-C6-Alkenyloxy wie wie Ethenyloxy, 1-Propenyloxy, 2-Propenyloxy, 1-Methylethenyloxy, 1-Butenyloxy, 2-Butenyloxy, 3-Butenyloxy, 1-Methyl-1-propenyloxy, 2-Methyl-1-propenyloxy, 1-Methyl-2-propenyloxy, 2-Methyl-2-propenyloxy, 1-Pentenyloxy, 2-Pentenyloxy, 3-Pentenyloxy, 4-Pentenyloxy, 1-Methyl-1-butenyloxy, 2-Methyl-1-butenyloxy, 3-Methyl-1-butenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-1-butenyloxy, 1,2-Dimethyl-2-butenyloxy, 1,2-Dimethyl-3-butenyloxy, 1,3-Dimethyl-1-butenyloxy, 1,3-Dimethyl-2-butenyloxy, 1,3-Dimethyl-3-butenyloxy, 2,2-Dimethyl-3-butenyloxy, 2,3-Dimethyl-1-butenyloxy, 2,3-Dimethyl-2-butenyloxy, 2,3-Dimethyl-3-butenyloxy, 3,3-Dimethyl-1-butenyloxy, 3,3-Dimethyl-2-butenyloxy, 1-Ethyl-1-butenyloxy, 1-Ethyl-2-butenyloxy, 1-Ethyl-3-butenyloxy, 2-Ethyl-1-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-1-propenyloxy und 1-Ethyl-2-methyl-2-propenyloxy, insbesondere Prop-2-enyloxy; C3-C6-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 1,1-Dimethyl-2-propinyloxy, 1-Ethyl-2-propinyloxy, 2-Hexinyloxy, 3-Hexinyloxy, 4-Hexinyl-oxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyl-oxy und 1-Ethyl-1-methyl-2-propinyloxy, insbesondere Prop-2-inyloxy, und C1-C4-Alkoxy-C1-C4-alkyl wie wie Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, Methoxyethyl, Ethoxyethyl, n-Propoxyethyl, (1-Methylethoxy)ethyl, n-Butoxyethyl, (1-Methylpropoxy)ethyl, (2-Methylpropoxy)ethyl, (1,1-Dimethylethoxy)ethyl, 3-(Methoxy)propyl, 2-(Methoxy)propyl und 2-(Ethoxy)propyl, vorzugsweise Methoxymethyl und Ethoxyethyl,
   eine Phenyl- oder Pyridylgruppe, die jeweils noch einen bis drei Reste tragen kann, ausgewählt aus einer Gruppe bestehend aus Nitro, Cyano, Formyl, Halogen wie vorstehend genannt, insbesondere Fluor und Chlor, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, partiell oder vollständig halogeniertem C₁-C₄-Alkoxy wie vorstehend genannt, insbesondere Trifluormethoxy, C₁-C₄-Alkylthio wie vorstehend genannt, insbesondere Methylthio, C₃-C₆-Alkenyl wie vorstehend genannt, insbesondere C₃-C₄-Alkenyl, vorzugsweise Prop-2-enyl, C₃-C₆-Alkenyloxy wie vorstehend genannt, insbesondere C₃-C₄-Alkenyloxy, vorzugsweise Prop-2-enyloxy, C₃-C₆-Alkinyl wie vorstehend genannt, insbesondere C₃-C₄-Alkinyl, vorzugsweise Prop-2-inyl, C₃-C₆-Alkinyloxy wie vorstehend genannt, insbesondere C₃-C₄-Alkinyloxy, vorzugsweise Prop-2-inyloxy und NR^{g}R^{h}, wobei R^{g} und R^{h} die oben genannte Bedeutung haben, vorzugsweise Wasserstoff, Acetyl und Benzoyl;
   besondere bevorzugte Phenyl- und Pyridylgruppen sind Phenyl, 4-Ethylphenyl, 4-Propargyloxyphenyl, 2,4,6-Trimethylphenyl, 4-Benzoylamino-3-fluorphenyl, 4-Formylphenyl und Pyridyl;
W
   eine C₁-C₆-Alkylen-, eine C₃-C₆-Alkenylen- oder eine C₃-C₆-Alkinylenkette wie Methylen, Ethylen, Propylen, Butylen, Pentylen, Hexylen, Propenylen, Prop-2-enylen, Butenylen, But-2-enylen, But-3-enylen, Pentenylen, Pent-2-enylen, Pent-3-enylen, Pent-4-enylen, Hex-1-enylen, Hex-2-enylen, Hex-3-enylen, Hex-4-enylen, Hex-5-enylen, Prop-2-inylen, But-2-inylen, But-3-inylen, Pent-2-inylen, Pent-3-inylen, Pent-4-inylen, Hex-2-inylen, Hex-3-inylen, Hex-4-inylen, Hex-5-inylen, wobei diese Gruppen noch ein bis drei Reste tragen können, ausgewählt aus einer Gruppe bestehend aus drei Halogenatomen wie vorstehend genannt, insbesondere Fluor und Chlor, drei C₁-C₃-Alkylsubstituenten wie Methyl, Ethyl, n-Propyl und Isopropyl, insbesondere Methyl und Ethyl, und einem Methylensubstituenten. Bei den ungesättigten Ketten können sowohl die cis- als auch die trans-Form auftreten. Besonders bevorzugt sind Propylen, Butylen, Prop-2-enylen, But-2-enylen, But-3-enylen und But-3-inylen;
   eine C₃-C₆-Alkylen- oder C₄-C₆-Alkenylengruppe, die beide noch einen bis drei Alkylreste tragen könen, wobei jeweils eine Methylengruppe durch ein Sauerstoff- oder Schwefelatom, eine Sulfoxid-, eine Sulfongruppe oder eine Gruppe -N(Rⁱ)-, mit Rⁱ Wasserstoff, unverzweigtes oder verzweigtes C₁-C₄-Alkyl wie vorstehend genannt, insbesondere Methyl und Ethyl, C₃-C₆-Alkenyl wie vorstehend genannt, insbesondere Prop-2-enyl und But-2-enyl, oder C₃-C₆-Alkinyl wie vorstehend genannt, insbesondere Prop-2-inyl und But-2-inyl, ersetzt sein kann, beispielsweise 3-Oxapropylen, 3-Azapropylen, 3-Thiapropylen, 3-Thiapropylen-3-oxid, 3-Thiapropylen-3,3-dioxid, 3-Oxabutylen, 3-Azabutylen, 3-Thiabutylen, 3-Thiabutylen-3-oxid, 3-Thiabutylen-3,3-dioxid, 4-Oxabutylen, 4-Azabutylen, 4-Thiabutylen, 4-Thiabutylen-4-oxid, 4-Thiabutylen-4,4-dioxid, 4-Oxabut-2-enylen, 4-Azabut-2-enylen, 4-Thiabut-2-enylen, 3-Oxapentylen, 3-Azapentylen, 3-Thiapentylen, 3-Thiapentylen-3-oxid, 3-Thiapentylen-3,3-dioxid, 4-Oxapentylen, 4-Azapentylen, 4-Thiapentylen, 4-Thiapentylen-4-oxid, 4-Thiapentylen-4,4-dioxid, 5-Oxapentylen, 5-Azapentylen, 5-Thiapentylen, 5-Thiapentylen-5-oxid, 5-Thiapentylen-5,5-dioxid, 5-Oxapent-3-enylen, 5-Azapent-3-enylen, 5-Thiapent-3-enylen, 3-Oxahexylen, 3-Azahexylen, 3-Thiahexylen, 3-Thiahexylen-3-oxid, 3-Thiahexylen-3,3-dioxid, 4-Oxahexylen, 4-Azahexylen, 4-Thiahexylen, 4-Thiahexylen-4-oxid, 4-Thiahexylen-4,4-dioxid, 5-Oxahexylen, 5-Azahexylen, 5-Thiahexylen, 5-Thiahexylen-5-oxid, 5-Thiahexylen-5,5-dioxid, 6-Oxahexylen, 6-Azahexylen, 6-Thiahexylen, 6-Thiahexylen-6-oxid, 6-Thiahexylen-6,6-dioxid, 6-Oxahex-4-enylen, 6-Azahex-4-enylen, 6-Thiahex-4-enylen.
   Bei den ungesättigten Ketten können die Doppelbindungen sowohl cis- als auch die trans-Konfiguration aufweisen.
   Besonders bevorzugt sind 3-Oxapropylen, 2-Methyl-3-oxapropylen, 3-Oxabutylen und 4-Oxabutylen;
R^{f}
   Wasserstoff; die Vinylgruppe;
   eine Gruppe -CH=CH-Z, wobei Z für Cyano;
   Halogen wie vorstehend genannt, insbesondere Fluor und Chlor;
   einen unverzweigten oder verzweigten C₁-C₄-Alkylrest wie vorstehend genannt, insbesondere Methyl und 1,1-Dimethylethyl;
   einen partiell oder vollständig halogenierten C₁-C₄-Alkylrest wie vorstehend genannt, insbesondere Difluormethyl, Trifluormethyl, 2,2,2-Trifluorethyl und Pentafluorethyl; einen C₃-C₆-Cycloalkylrest wie Cyclopropyl, Cyclobutyl, Cyclopentyl und Cyclohexyl, der seinerseits noch einen bis drei Substituenten tragen kann, ausgewählt aus einer Gruppe bestehend aus Hydroxyl, Halogen wie vorstehend genannt, insbesondere Fluor und Chlor, unverzweigtem oder verzweigtem C₁-C₄-Alkyl wie vorstehend genannt, insbesondere Methyl und Isopropyl,
   partiell oder vollständig halogeniertem C₁-C₄-Alkyl wie vorstehend genannt, insbesondere Trifluormethyl, und C₁-C₄-Alkoxy wie vorstehend genannt, insbesondere Methoxy;
   den Carboxylrest;
   einen C₁-C₈-Alkoxycarbonylrest wie Methoxycarbonyl, Ethoxycarbonyl, n-Propyloxycarbonyl, 1-Methyl-ethoxycarbonyl, n-Butoxycarbonyl, 1-Methylpropyloxycarbonyl, 2-Methylpropyloxycarbonyl, 1,1-Dimethylethoxycarbonyl, n-Pentoxycarbonyl, 1-Methylbutyloxycarbonyl, 2-Methylbutyloxycarbonyl, 3-ethylbutyloxycarbonyl, 2,2-Dimethylpropyloxycarbonyl, 1-Ethylpropyloxycarbonyl, n-Hexoxycarbonyl, 1,1-Dimethyl-propoxycarbonyl, 1,2-Dimethyl-propyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropoxycarbonyl, 1-Ethyl-1-methylpropyloxycarbonyl und 1-Ethyl-2-methylpropyloxycarbonyl, insbesondere Methoxycarbonyl;
   den Benzyloxycarbonylrest;
   den Phenyl-, Thienyl- oder Pyridylrest, wobei diese Reste jeweils noch einen bis drei Substituenten tragen können, ausgewählt aus einer Gruppe bestehend aus Nitro, Cyano, Halogen wie vorstehend genannt, insbesondere Fluor und Chlor, unverzweigtem oder verzweigtem C₁-C₄-Alkyl wie vorstehend genannt, insbesondere Methyl, Ethyl und 1-Methylethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy und Ethoxy, partiell oder vollständig halogeniertem C₁-C₄-Alkoxy wie vorstehend genannt, insbesondere Difluormethoxy und Trifluormethoxy, C₁-C₄-Alkylthio wie vorstehend genannt, insbesondere Methylthio, C₃-C₆-Cycloalkyl wie vorstehend genannt, wobei der Cycloalkylsubstituent seinerseits noch einen bis drei Reste tragen kann, ausgewählt aus einer Gruppe bestehend aus Halogen wie vorstehend genannt, insbesondere Fluor und Chlor, unverzweigtem oder verzweigtem C₁-C₄-Alkyl wie vorstehend genannt, insbesondere Methyl, partiell oder vollständig halogeniertem C₁-C₄-Alkyl wie vorstehend genannt, insbesondere Trifluormethyl, und C₁-C₄-Alkoxy wie vorstehend genannt, insbesondere Methoxy;
   steht;
   die Ethinylgruppe, die einen der folgenden Reste tragen kann:
   unverzweigtes oder verzweigtes C₁-C₄-Alkyl wie vorstehend genannt, insbesondere Methyl und Ethyl, wobei der Alkylrest seinerseits noch einen bis drei Substituenten tragen kann, ausgewählt aus einer Gruppe bestehend aus Hydroxyl, Halogen wie vorstehend genannt, insbesondere Fluor und Chlor, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, und C₁-C₄-Alkoxy wie vorstehend genannt, insbesondere Methoxy;
   C₃-C₆-Cycloalkyl wie Cyclopropyl, Cyclobutyl, Cyclopentyl und Cyclohexyl, wobei der Cycloalkylrest seinerseits noch einen bis drei Substituenten tragen kann, ausgewählt aus einer Gruppe bestehend aus Hydroxyl, Halogen wie vorstehend genannt, insbesondere Fluor und Chlor, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, und C₁-C₄-Alkoxy wie vorstehend genannt, insbesondere Methoxy,
   den Phenyl-, Thienyl- oder Pyridylrest, wobei jeder dieser drei aromatischen Reste noch einen bis drei Substituenten tragen kann, ausgewählt aus einer Gruppe bestehend aus Nitro, Cyano, Halogen wie vorstehend genannt, insbesondere Fluor und Chlor, unverzweigtem oder verzweigtem C₁-C₄-Alkyl wie vorstehend genannt, insbesondere Methyl und Ethyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy, partiell oder vollständig halogeniertem C₁-C₄-Alkoxy wie vorstehend genannt, insbesondere Trifluormethoxy und C₁-C₄-Alkylthio wie vorstehend genannt, insbesondere Methylthio;
   - die Phenylgruppe; eine Halogenphenylgruppe wie Fluorphenyl, Chlorphenyl und Bromphenyl; eine Dihalogenphenylgruppe wie Difluorphenyl, Dichlorphenyl, Dibromphenyl, Fluorchlorphenyl, Fluorbromphenyl und Chlorbromphenyl;
   - eine 5-gliedrige heteroaromatische Gruppe mit ein bis drei Heteroatomen, ausgewählt aus einer Gruppe bestehend aus ein bis drei Stickstoffatomen und einem Sauerstoff- oder Schwefelatom wie Furanyl, Thienyl, Pyrrolyl, Isoxazolyl, Isothiazolyl, Pyrazolyl, Oxazolyl, Thiazolyl, Imidazolyl, 1,2,4-Oxadiazolyl, 1,2,4-Thiadiazolyl, 1,2,4-Triazolyl, 1,3,4-Oxadiazolyl, 1,3,4-Thiadiazolyl und 1,3,4-Triazolyl, insbesondere Furanyl und Thienyl;
   eine 6-gliedrige heteroaromatische Gruppe mit ein bis vier Stickstoffatomen als Heteroatomen wie Pyridyl, Pyrimidyl, Pyrazyl, Pyridazinyl, Triazyl, Tetrazyl, insbesondere Pyridyl und Pyrimidyl;
   wobei die Phenyl- und Heteroarylgruppen noch einen bis drei der folgende Reste tragen können, im Falle der Heteroarylreste jedoch höchstens so viele, wie substituierbare C-Atome vorhanden sind:
   Nitro;
   C1-C4-Alkoxyreste wie vorstehend genannt, insbesondere Methoxy;
   C1-C4-Alkylthioreste wie vorstehend genannt, insbesondere Methylthio;
   partiell oder vollständig halogenierte C1-C4-Alkoxyreste, insbesondere C1-C2-Halogenalkoxyreste wie vorstehend genannt, vorzugsweise Trifluormethoxy;
   Resten Z und einem Rest -NR^{k}R^{l} mit
   - R^{k}: Wasserstoff, unverzweigtem oder verzweigtem C₁-C₄-Alkyl wie vorstehend genannt, insbesondere Methyl und Ethyl, C₃-C₆-Alkenyl wie vorstehend genannt, insbesondere Prop-2-en-1-yl oder C₃-C₆-Alkinyl wie vorstehend genannt, insbesondere Prop-2-in-1-yl, und
   - R^{l}: Wasserstoff, unverzweigtem oder verzweigtem C₁-C₄-Alkyl wie vorstehend genannt, insbesondere Methyl und Ethyl, C₃-C₆-Alkenyl wie vorstehend genannt, insbesondere Prop-2-en-1-yl, C3-C6-Alkinyl wie vorstehend genannt, insbesondere Prop-2-in-1-yl, unverzweigtem oder verzweigtem C₁-C₆-Acyl wie Acetyl, Propionyl und Butyryl, oder Benzoyl, das noch einen bis drei Substituenten tragen kann, ausgewählt aus einer Gruppe bestehend aus Nitro, Cyano, Halogen wie vorstehend genannt, insbesondere Fluor und Chlor, unverzweigtem oder verzweigtem C₁-C₄-Alkyl wie vorstehend genannt, insbesondere Methyl, partiell oder vollständig halogeniertem C₁-C₄-Alkyl wie vorstehend genannt, insbesondere Trifluormethyl, C₁-C₄-Alkoxy wie vorstehend genannt, insbesondere Methoxy und Ethoxy, und C₁-C₄-Alkylthio wie vorstehend genannt, insbesondere Methylthio;
   Bei mehreren Resten Z können die Substituenten gleich oder verschieden sein.

Ganz besonders bevorzugte Cyclohexenon-Derivate der Formel II, deren Kulturpflanzenverträglichkeit durch substituierte Thiochromenone I verbessert werden kann, sind den folgenden Tabellen 1 bis 9 zu entnehmen:

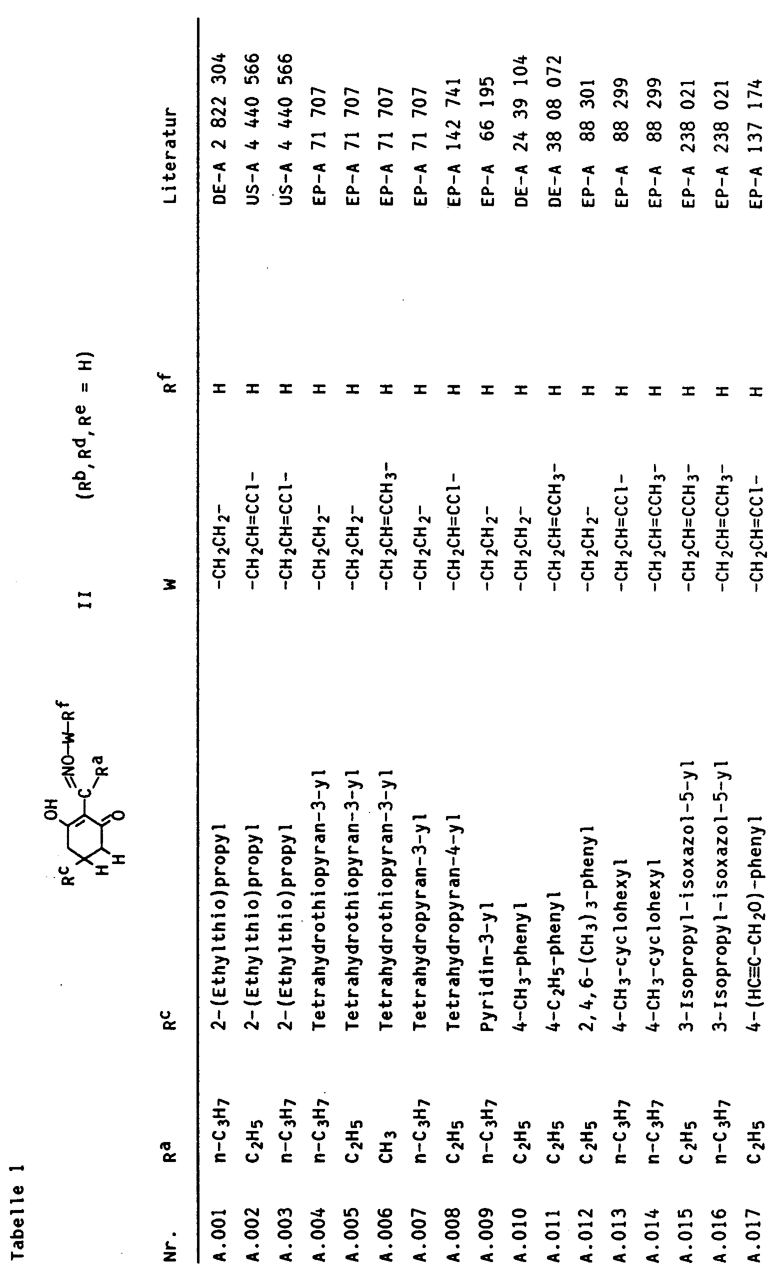

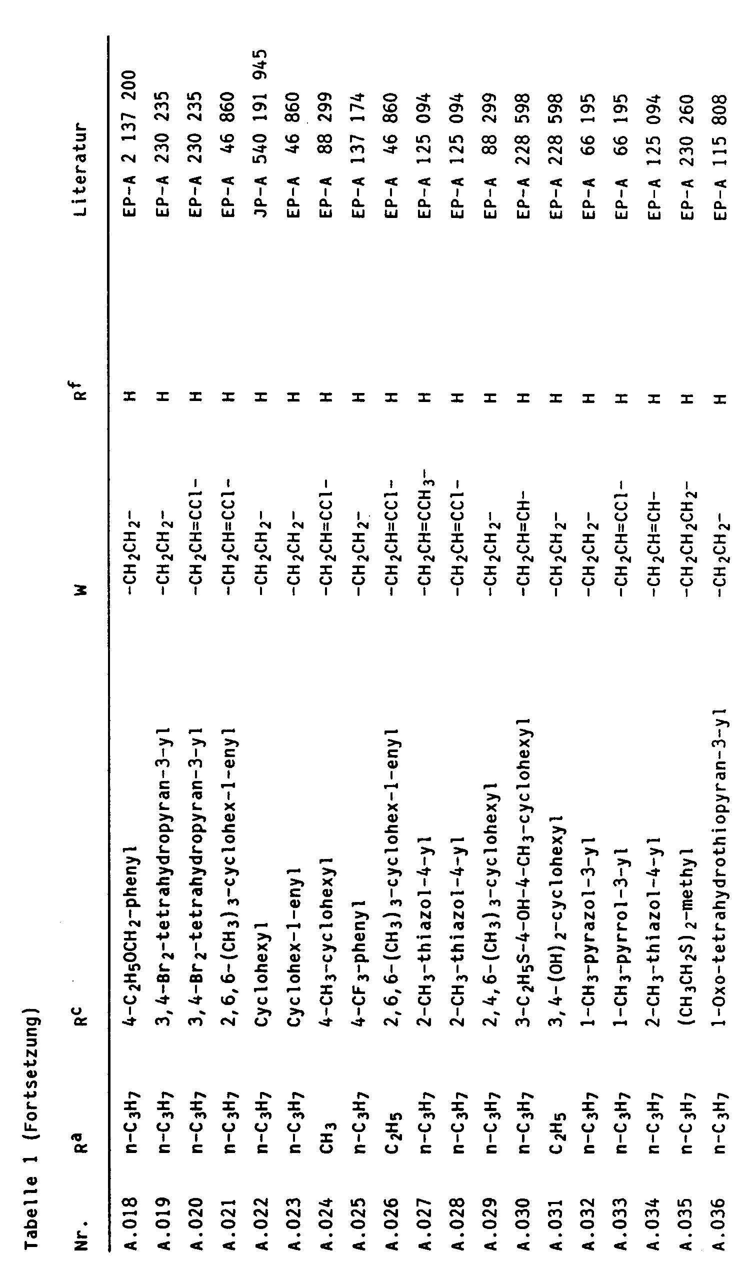

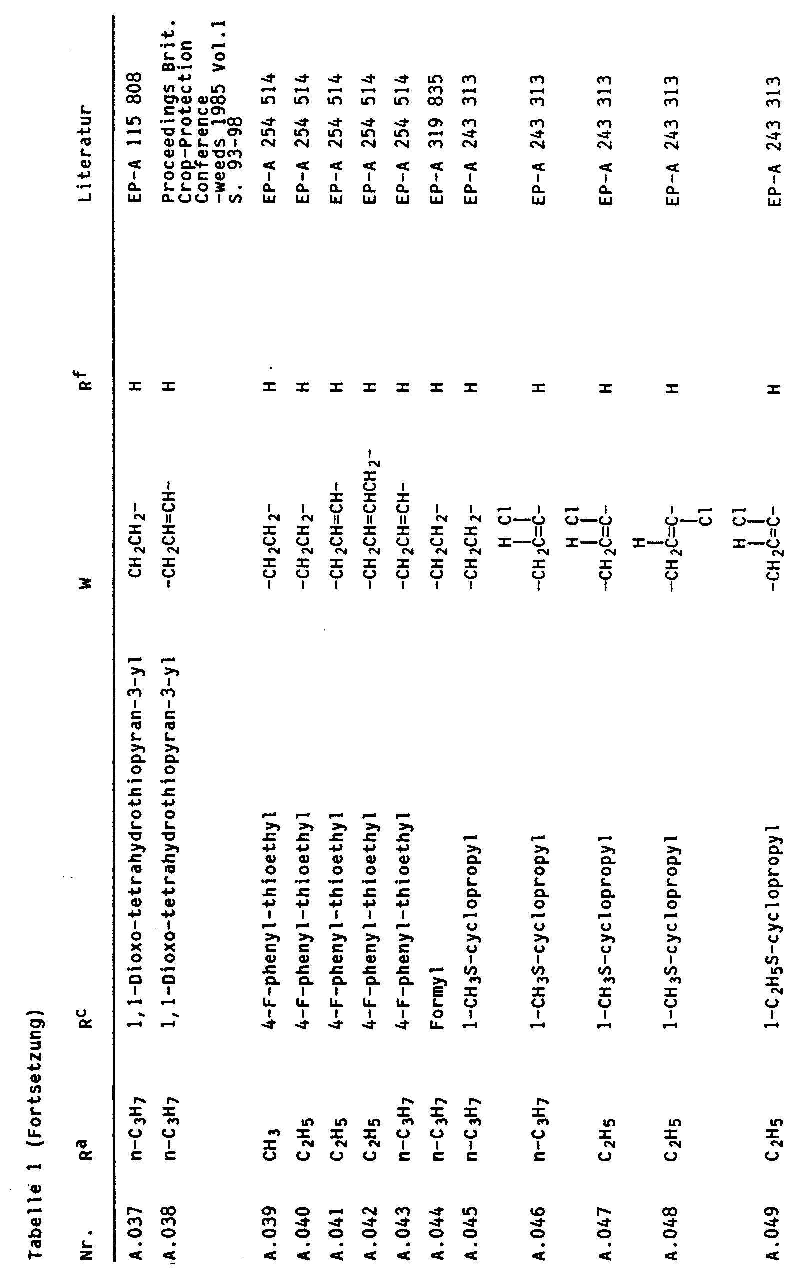

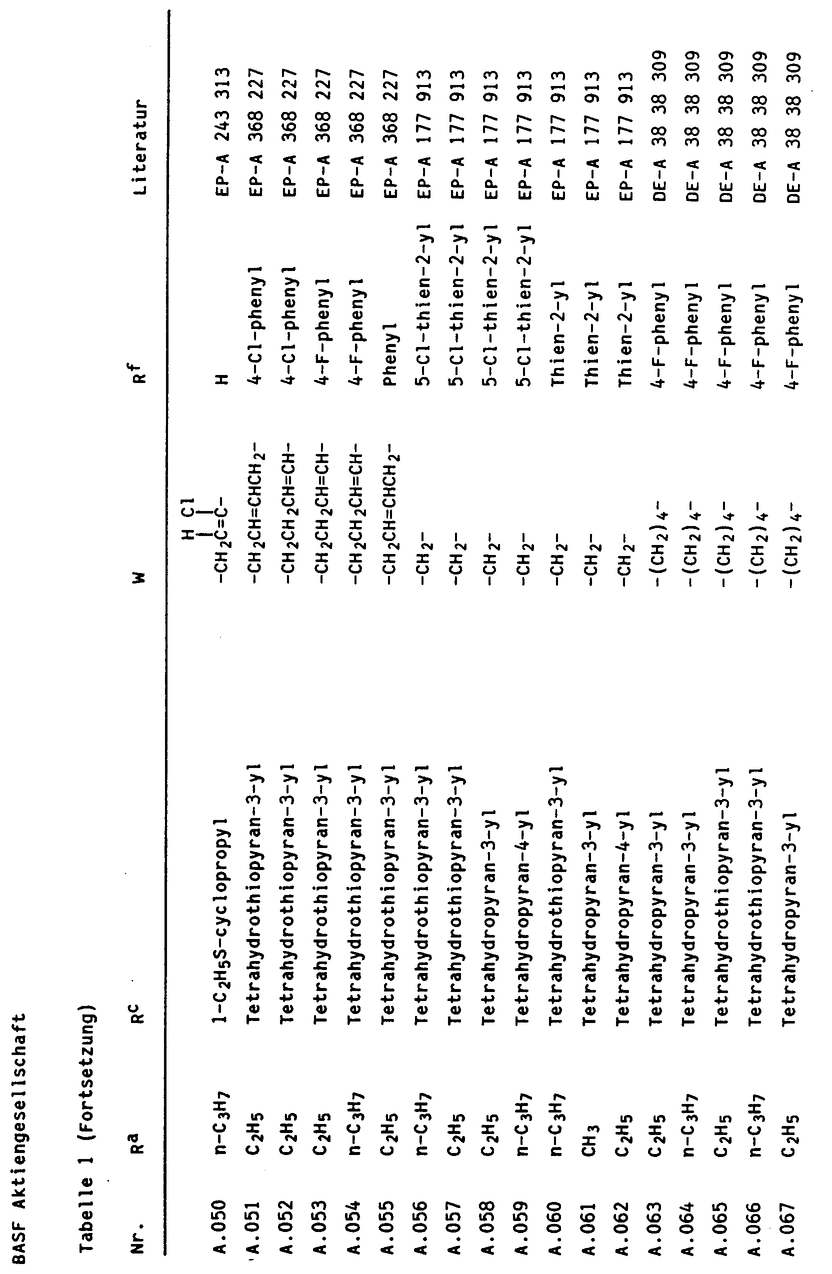

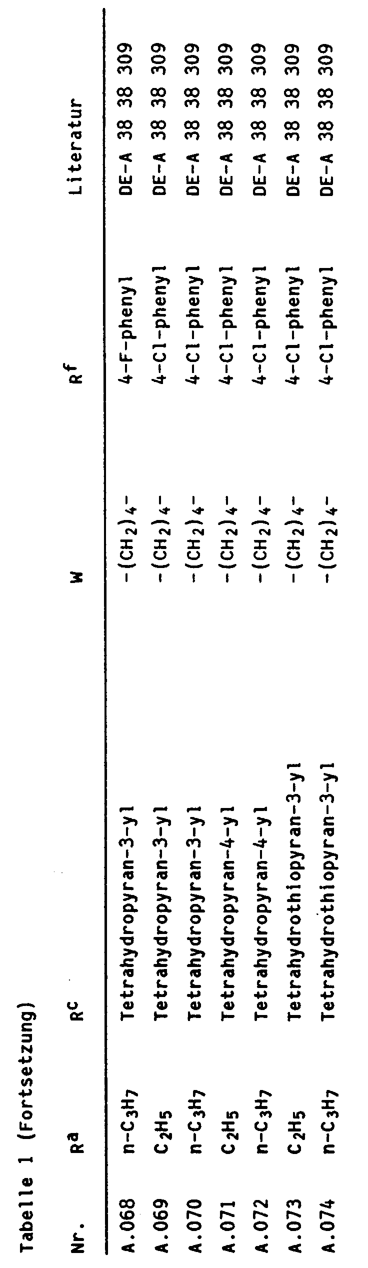

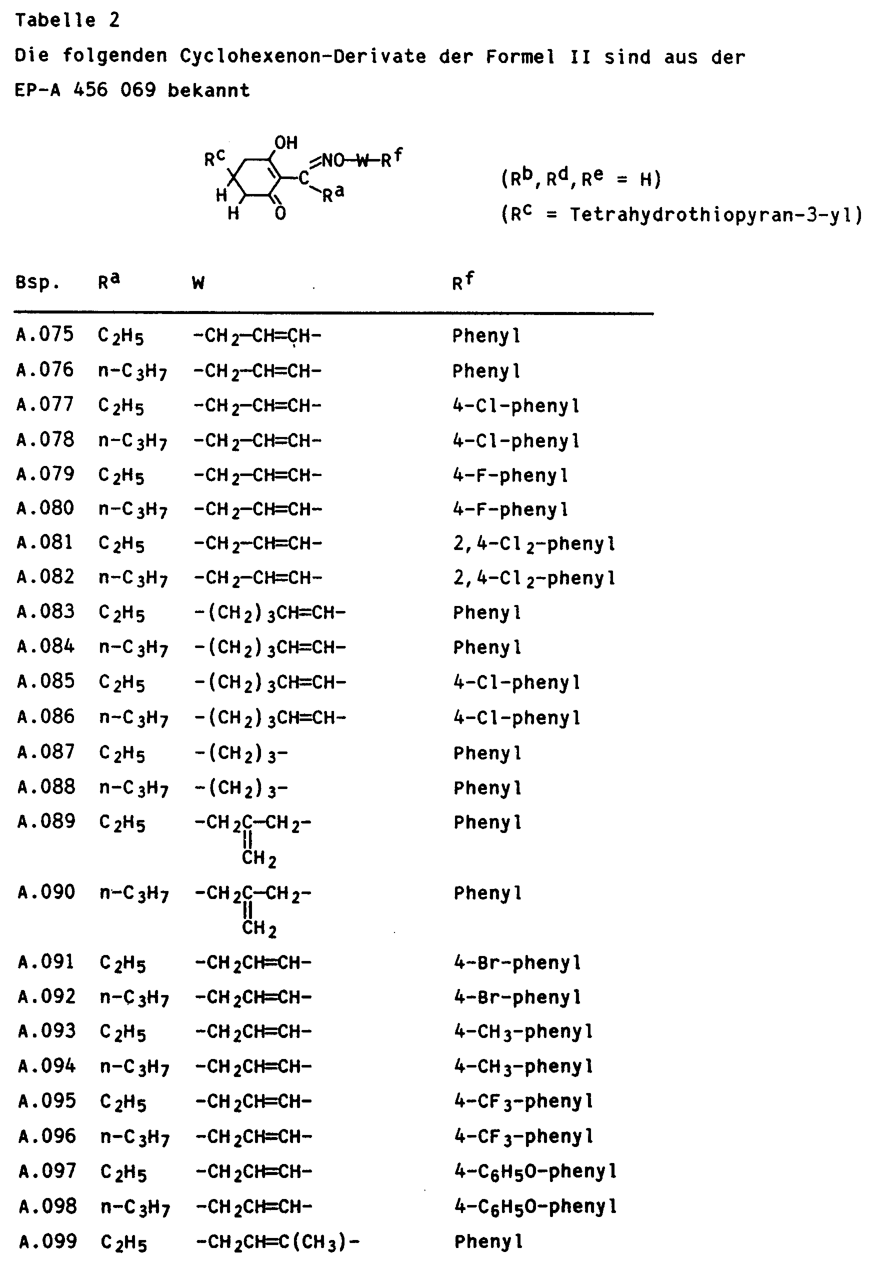

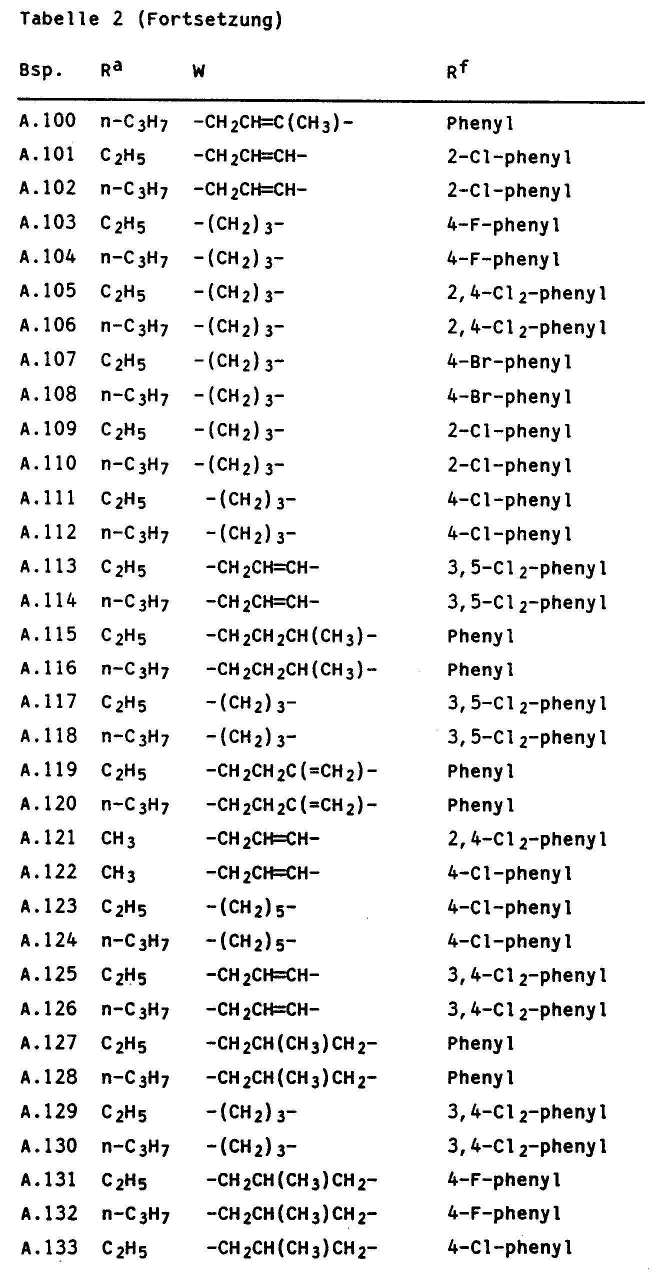

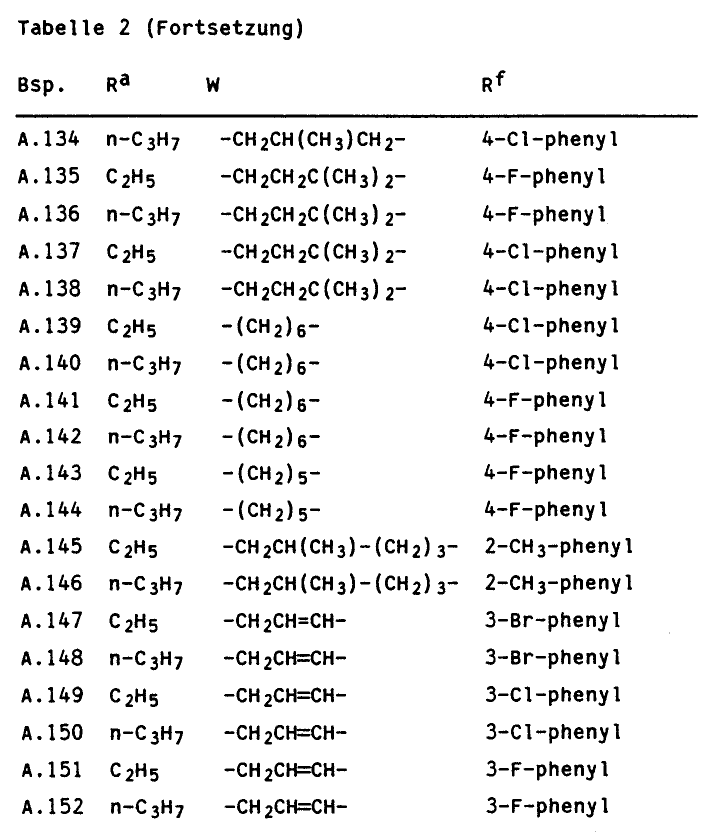

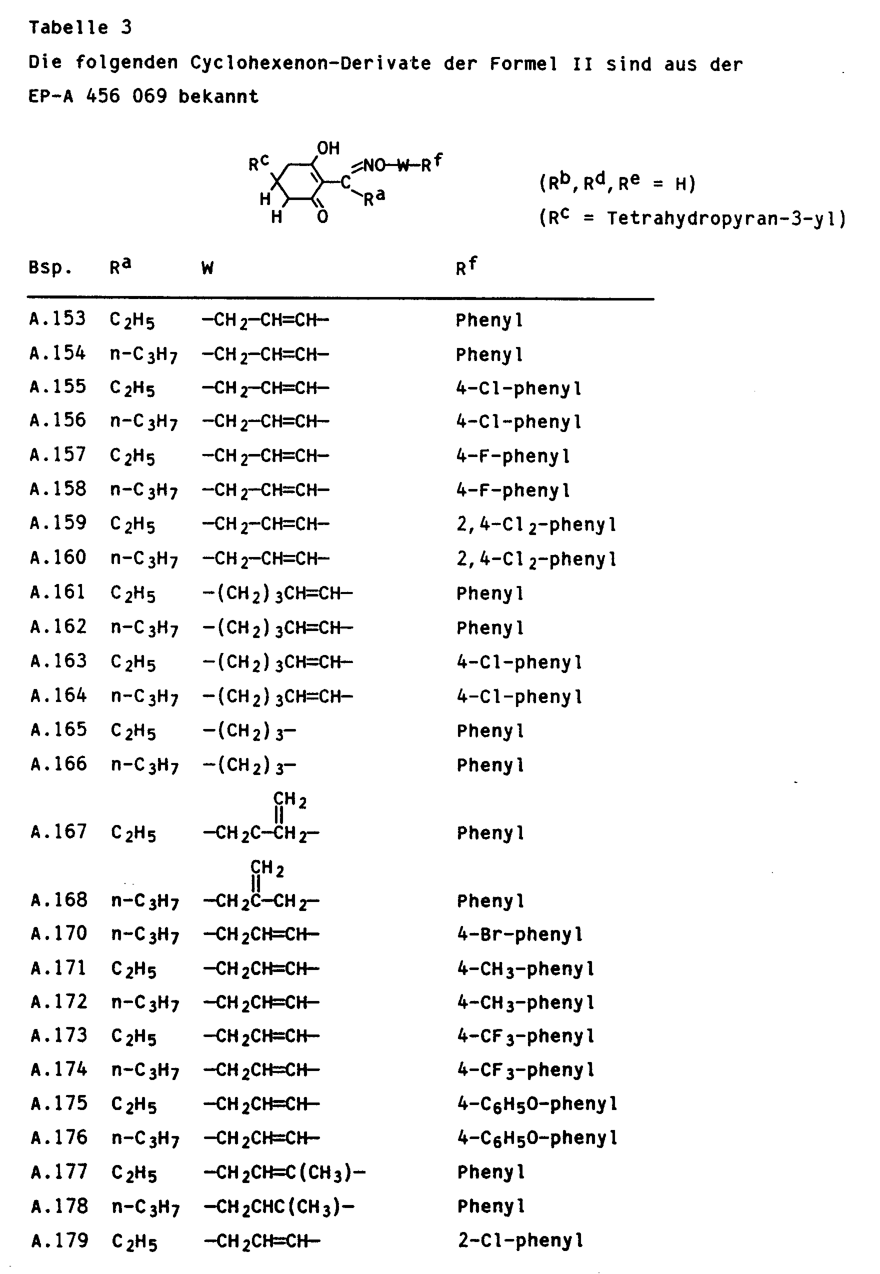

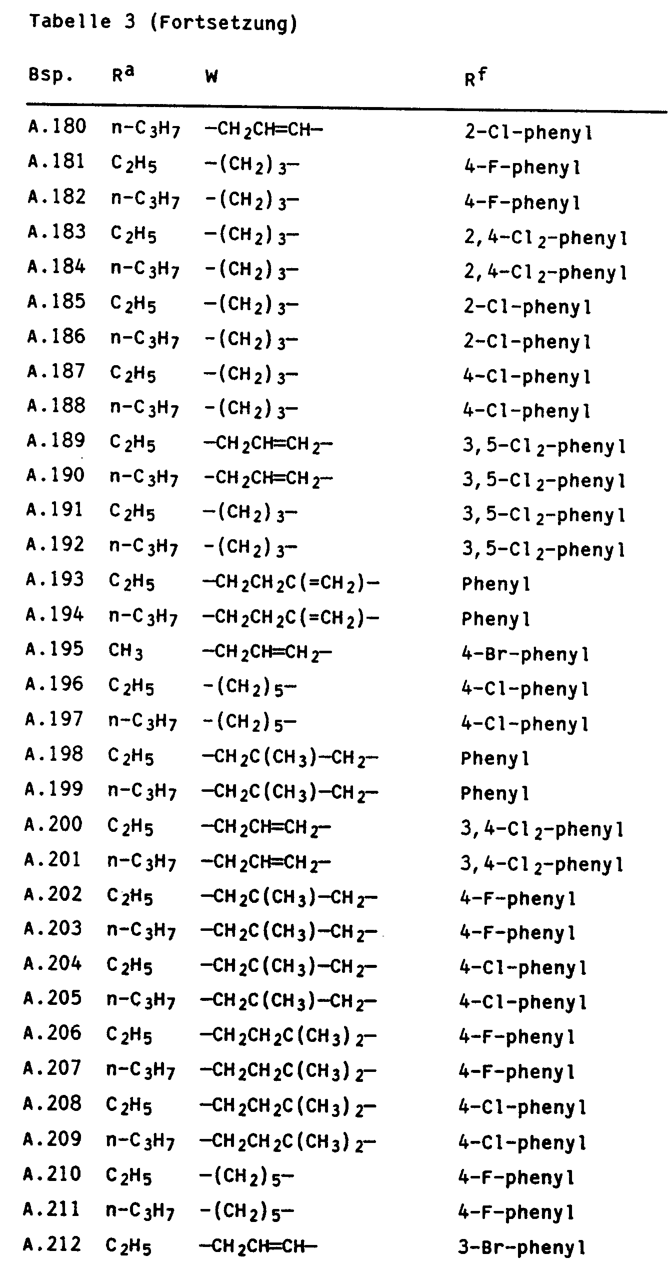

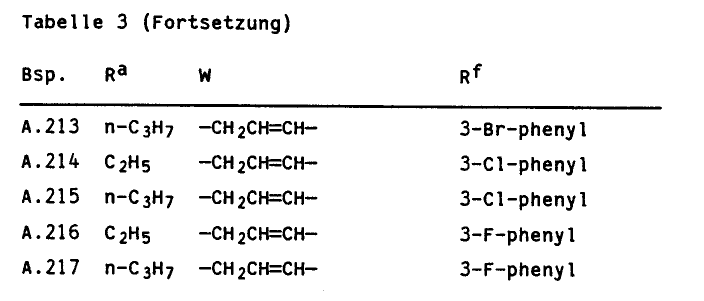

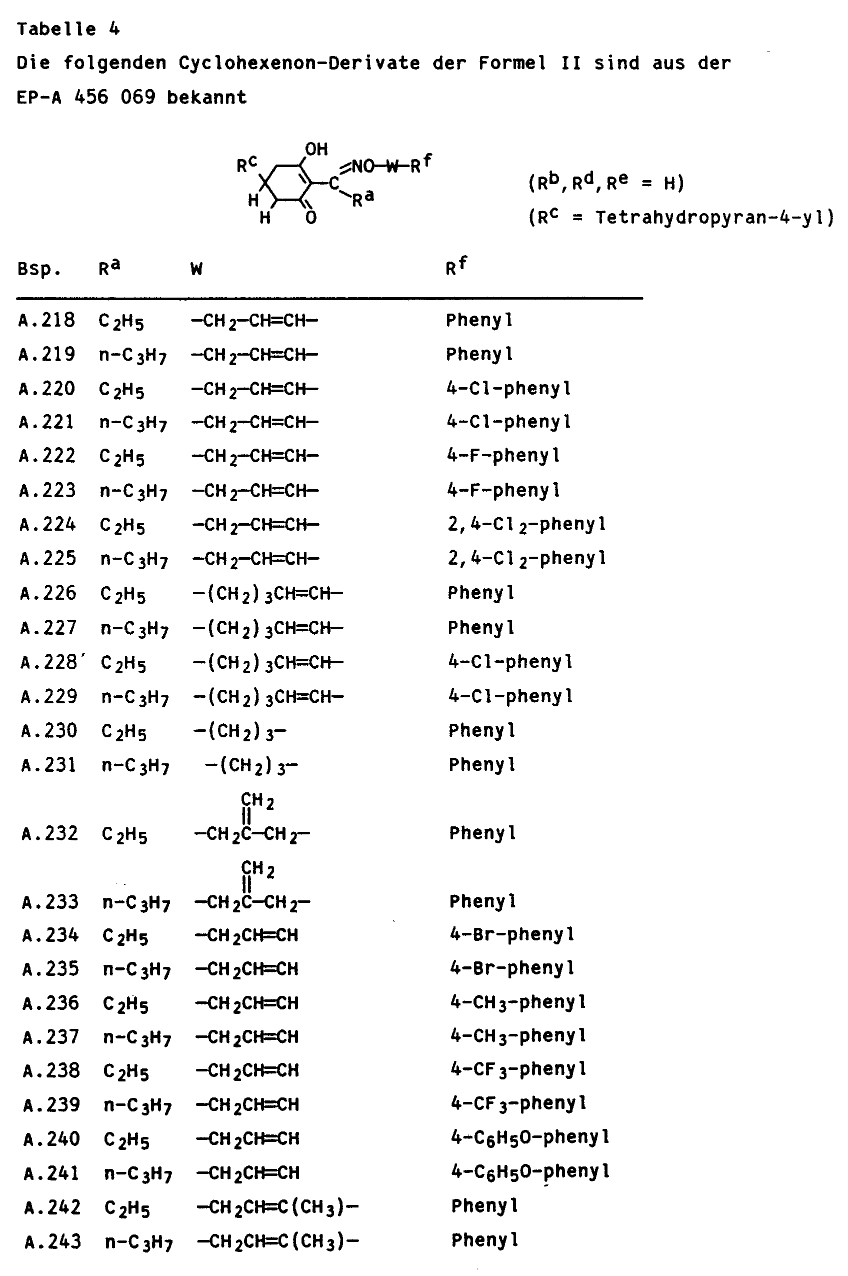

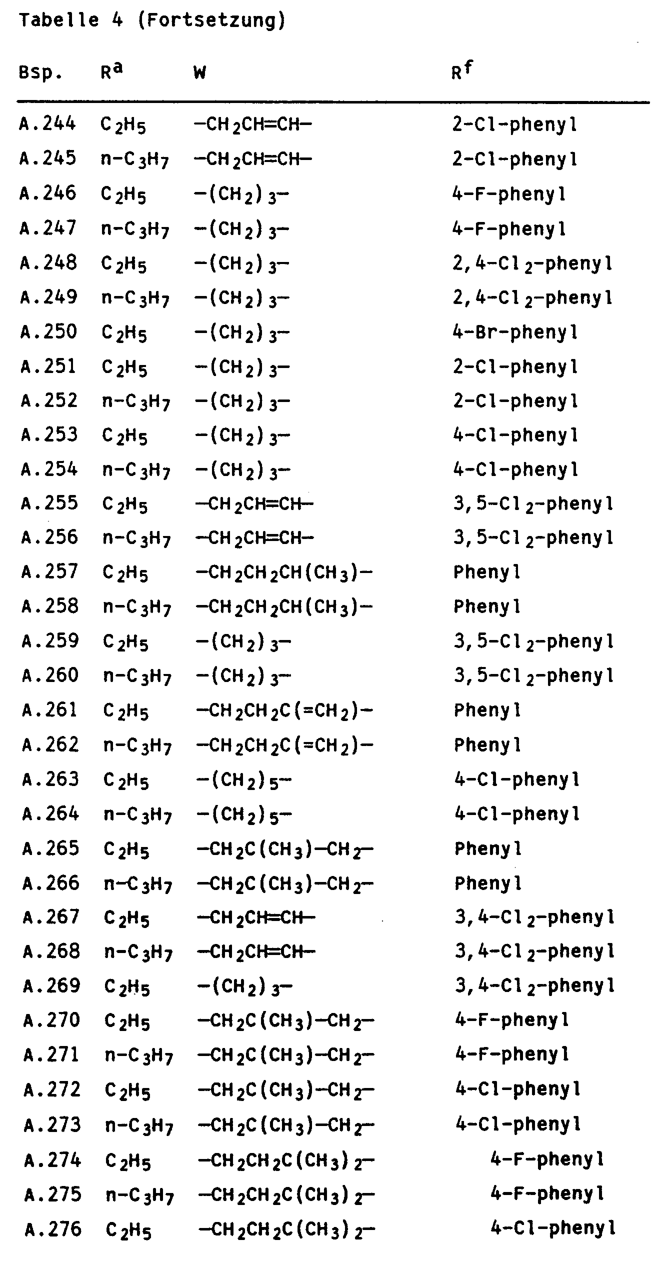

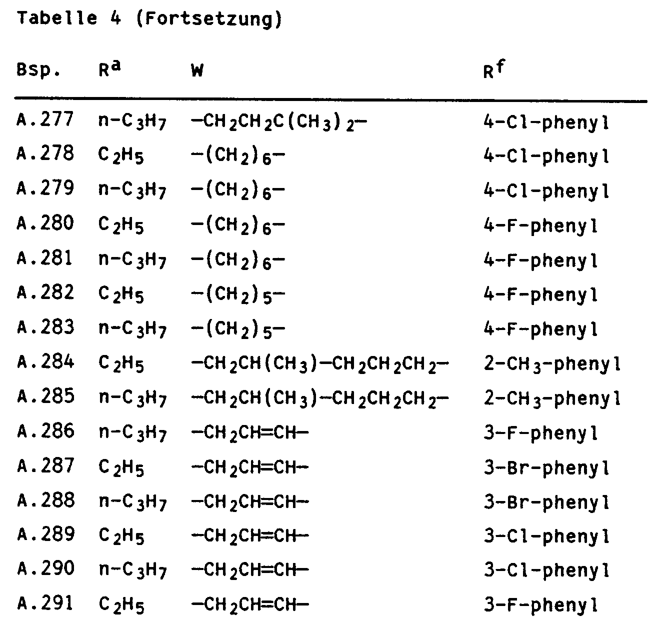

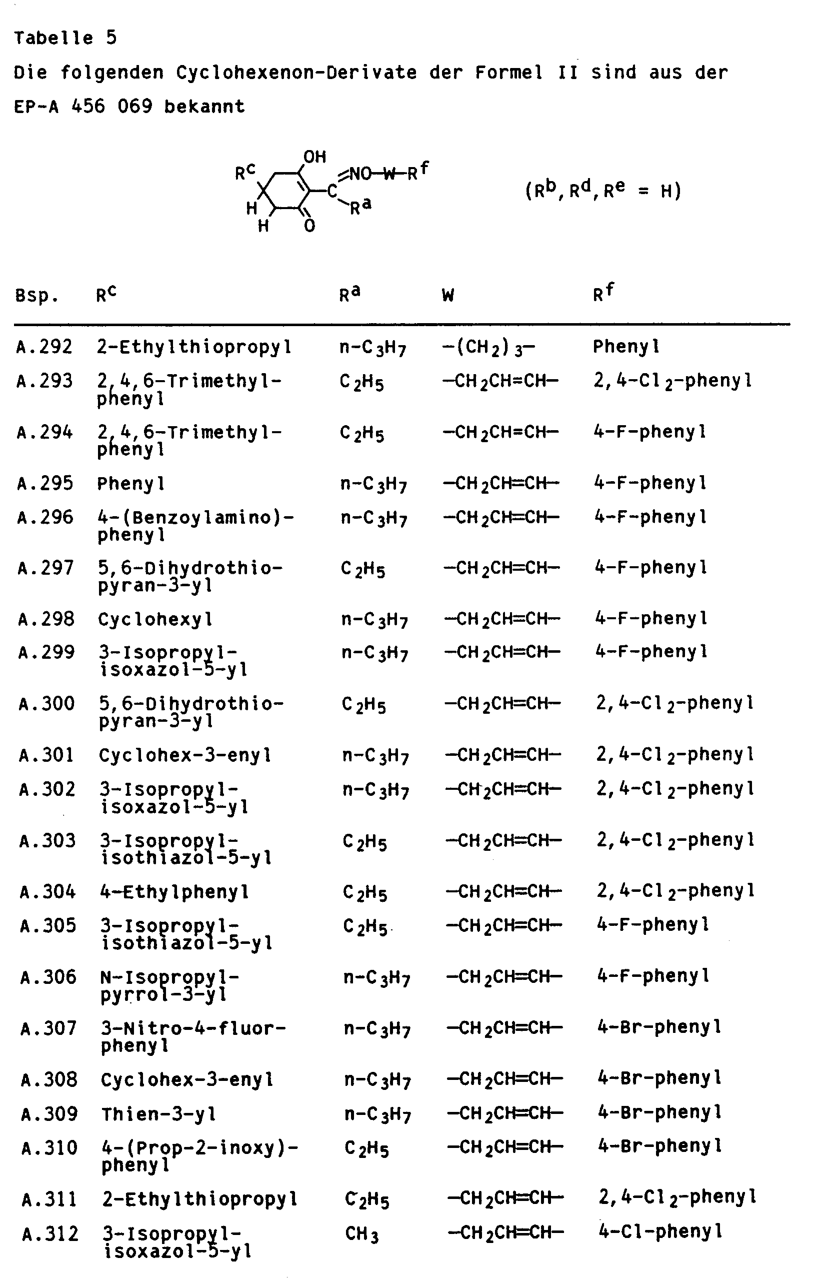

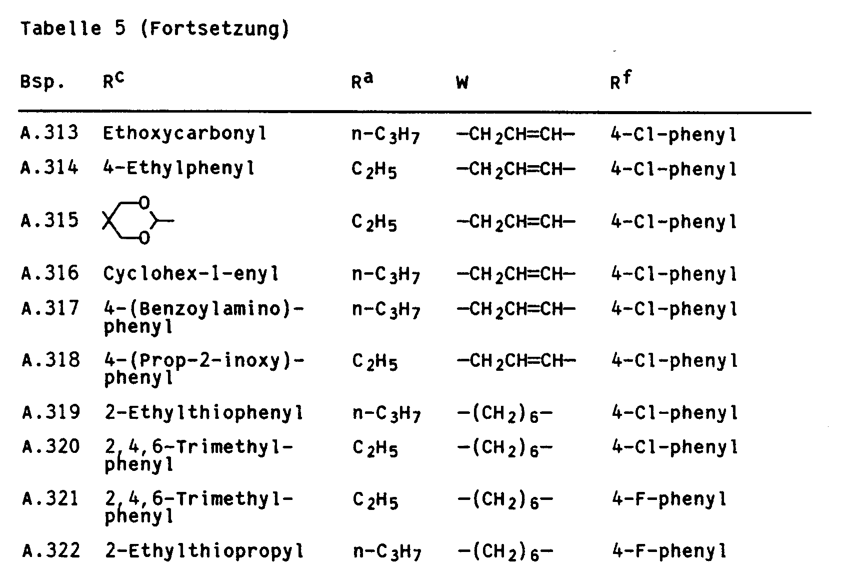

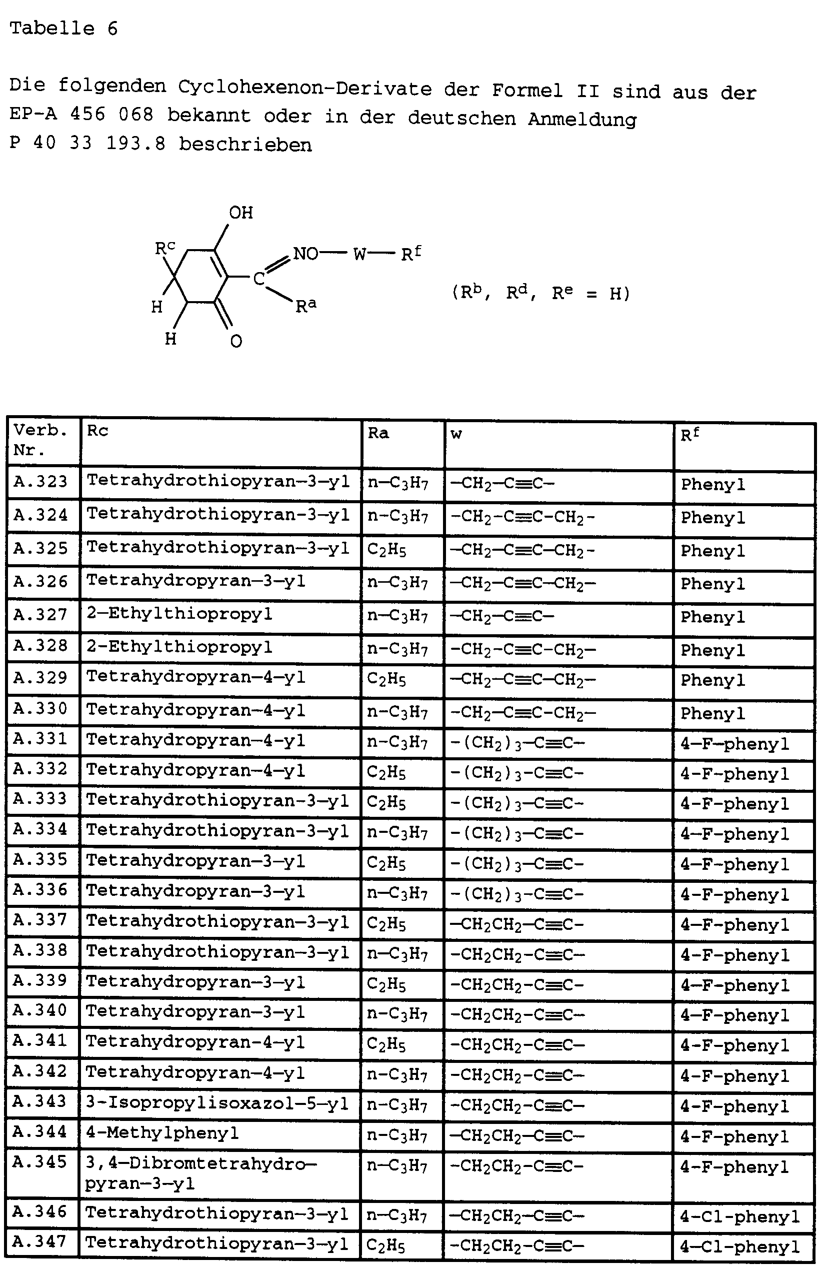

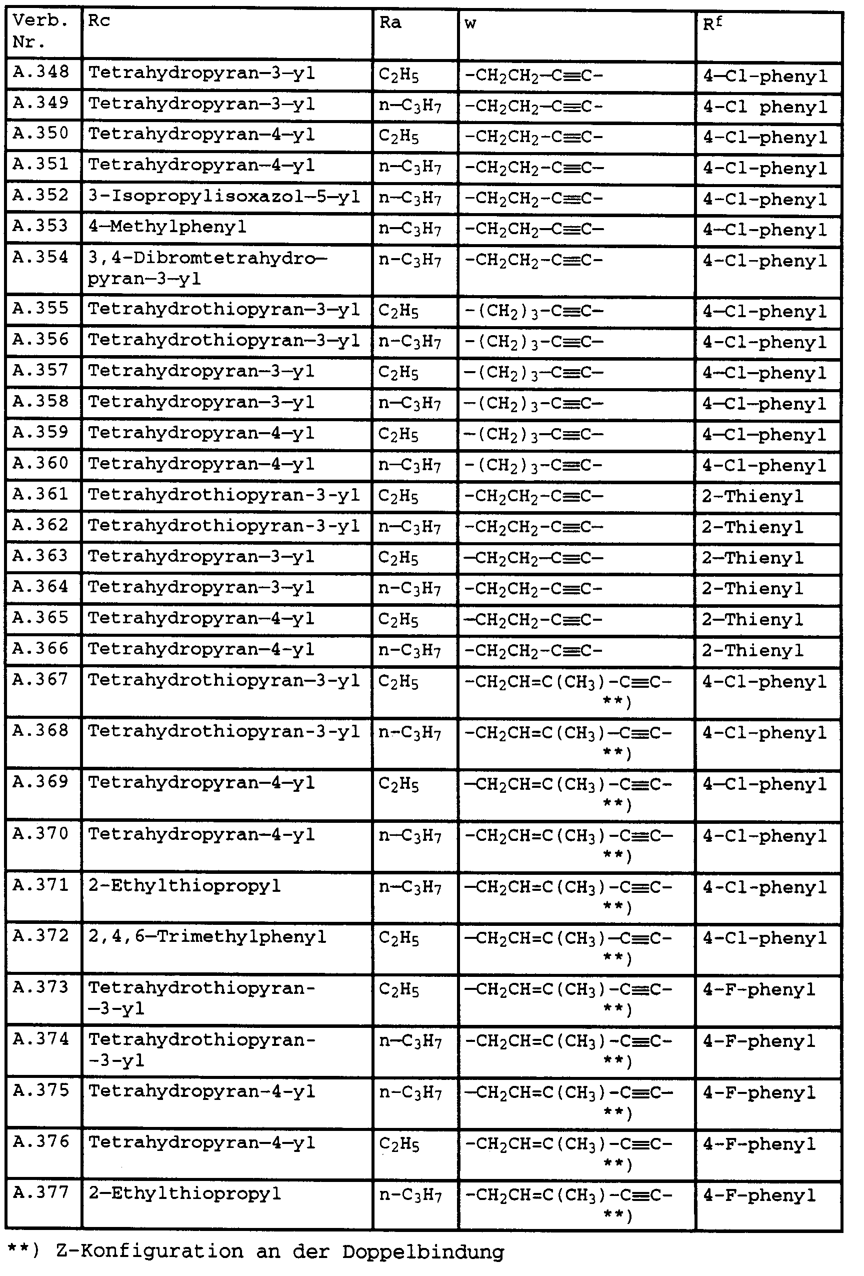

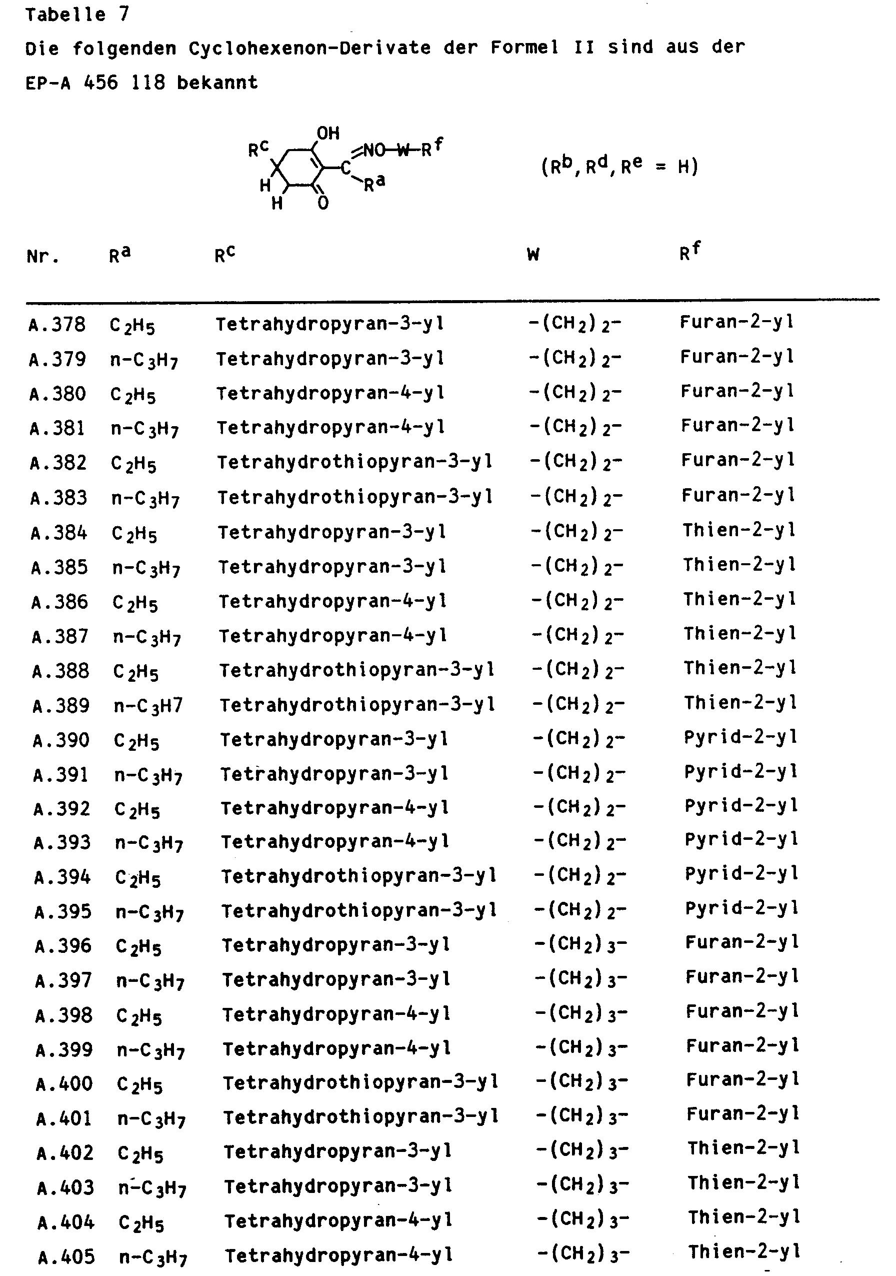

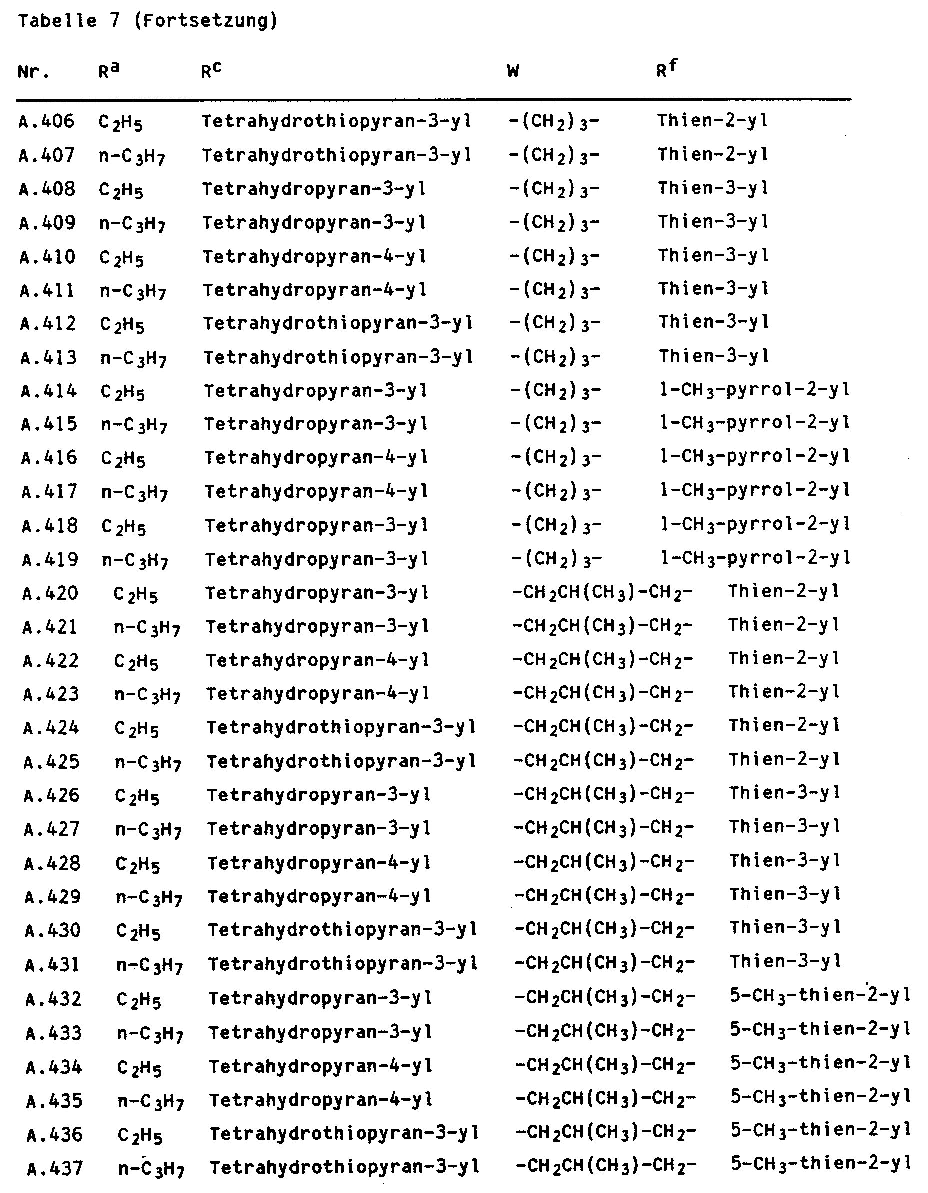

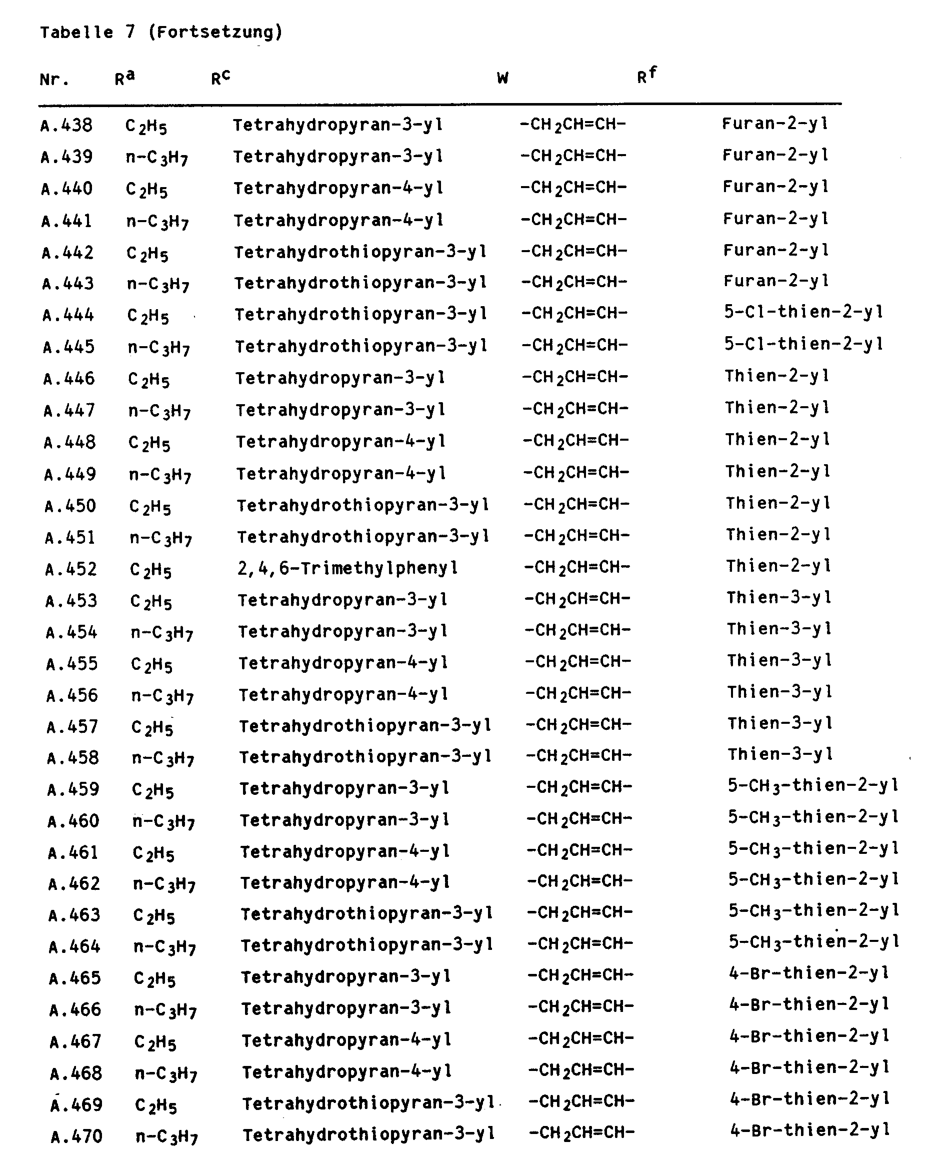

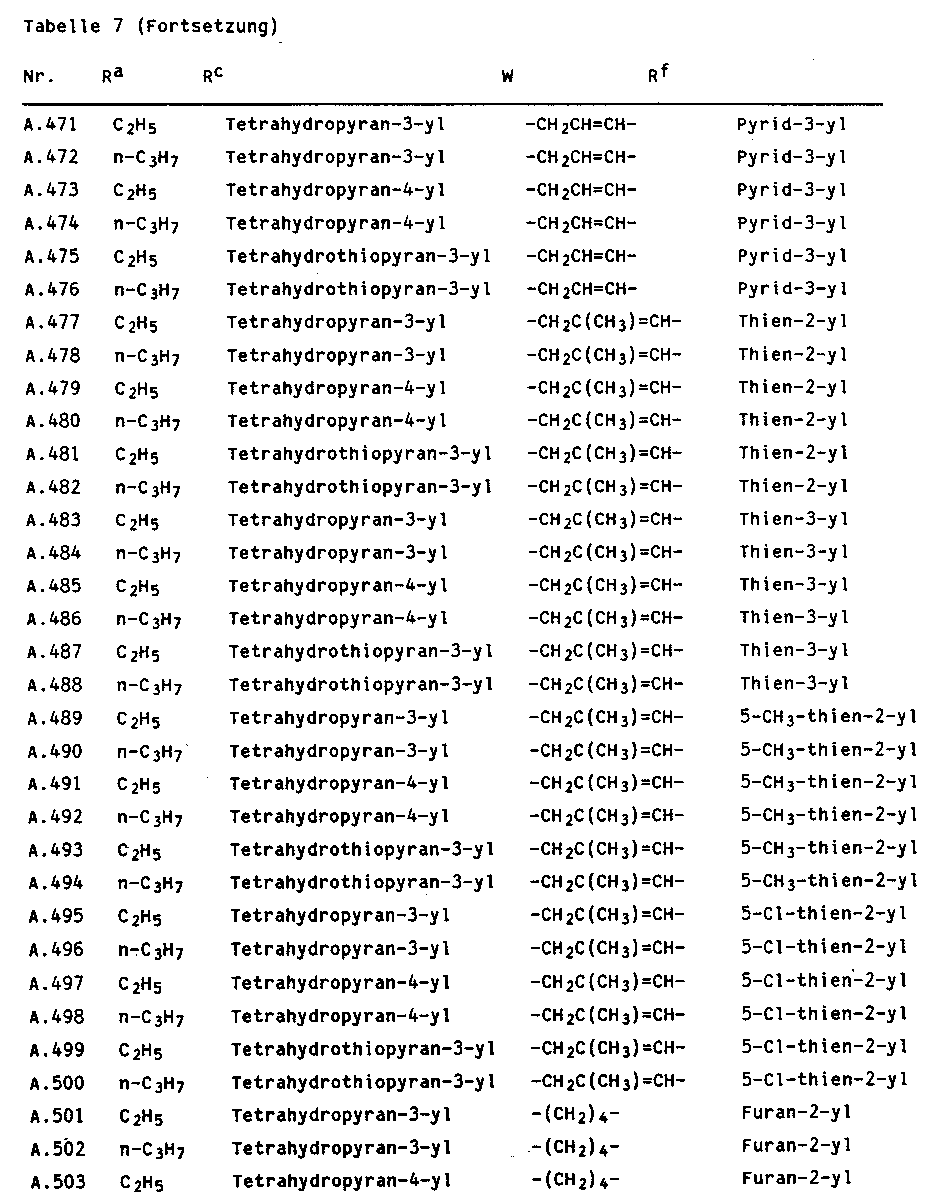

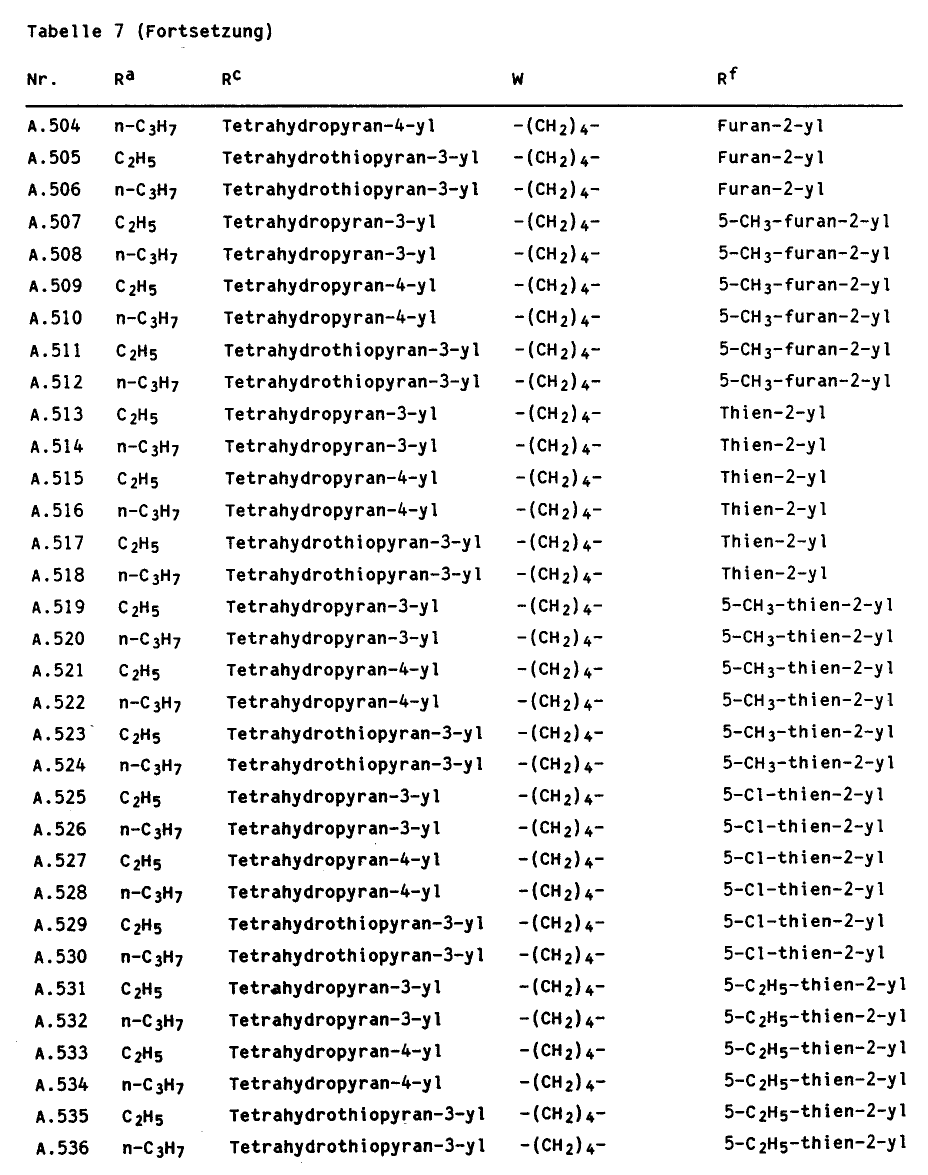

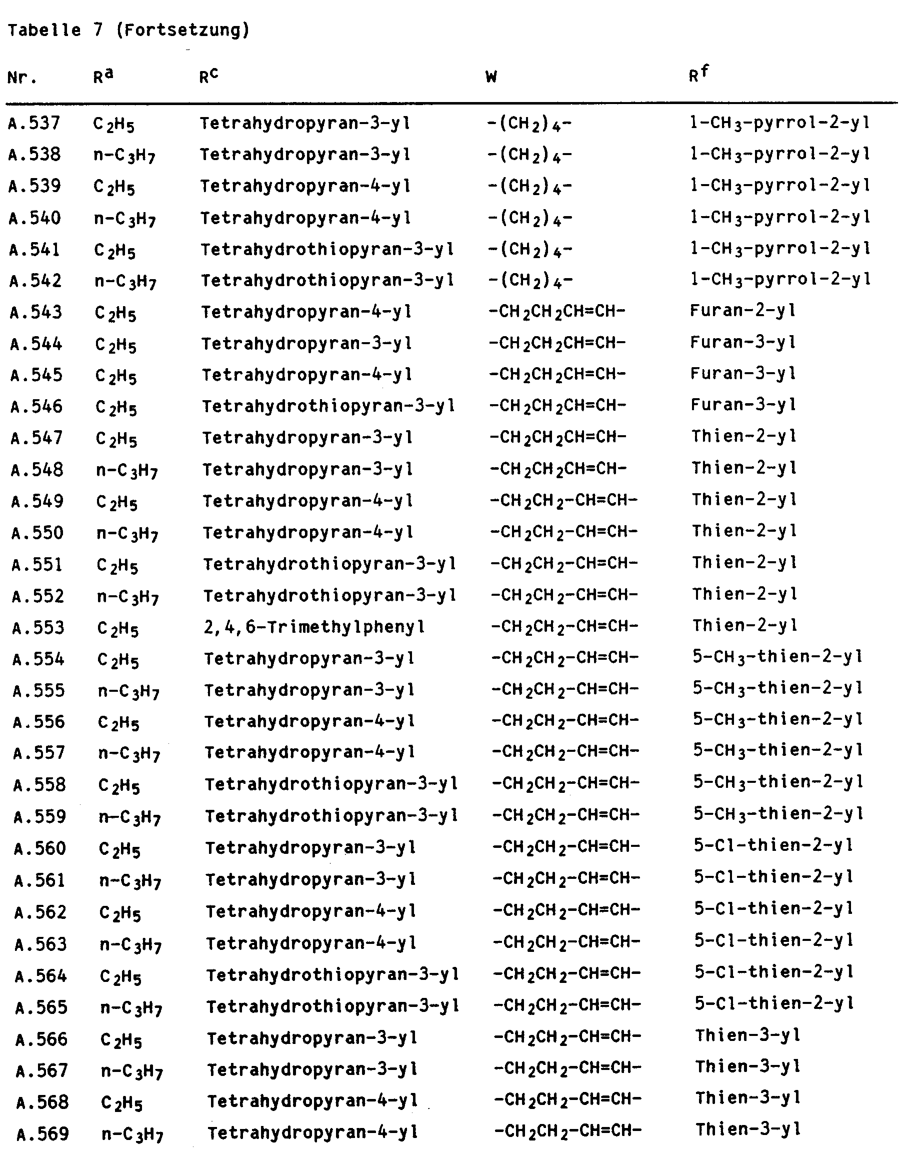

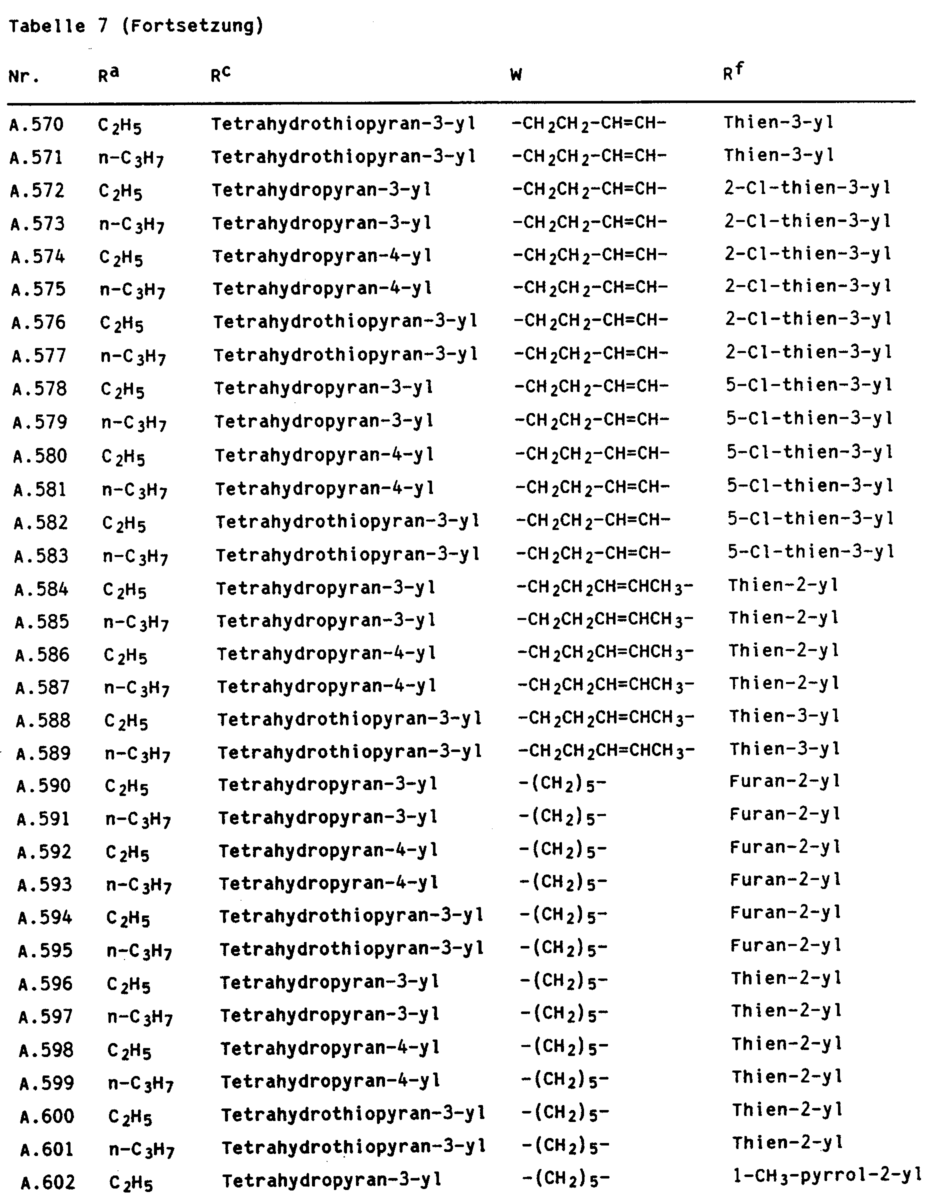

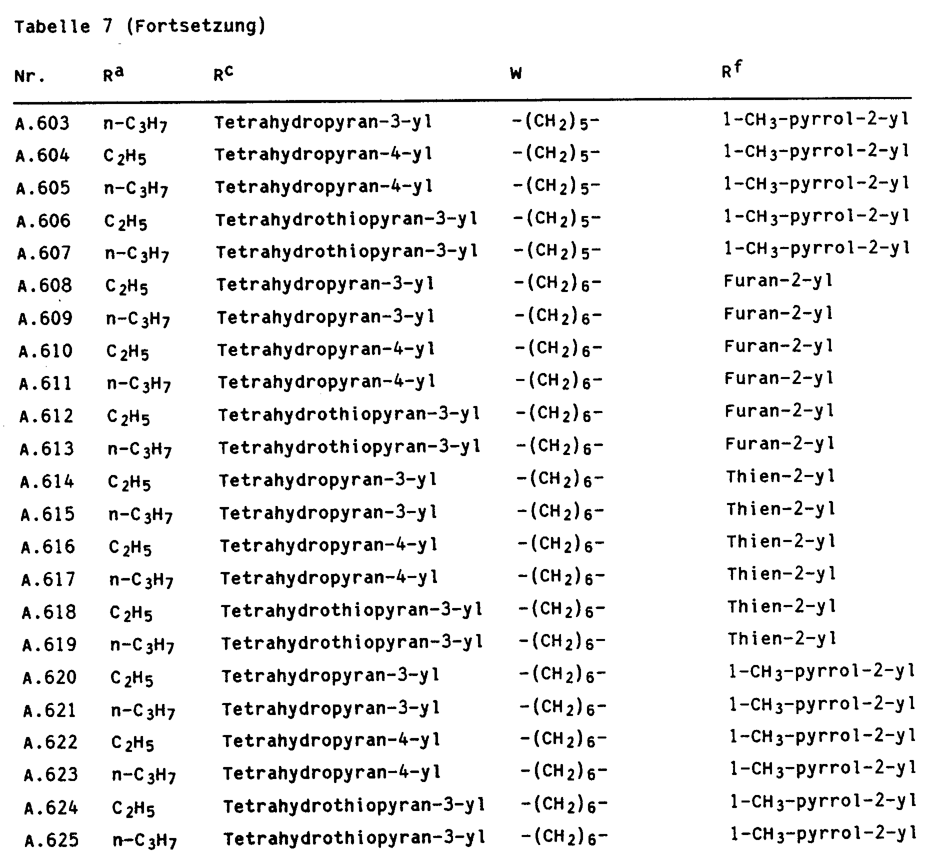

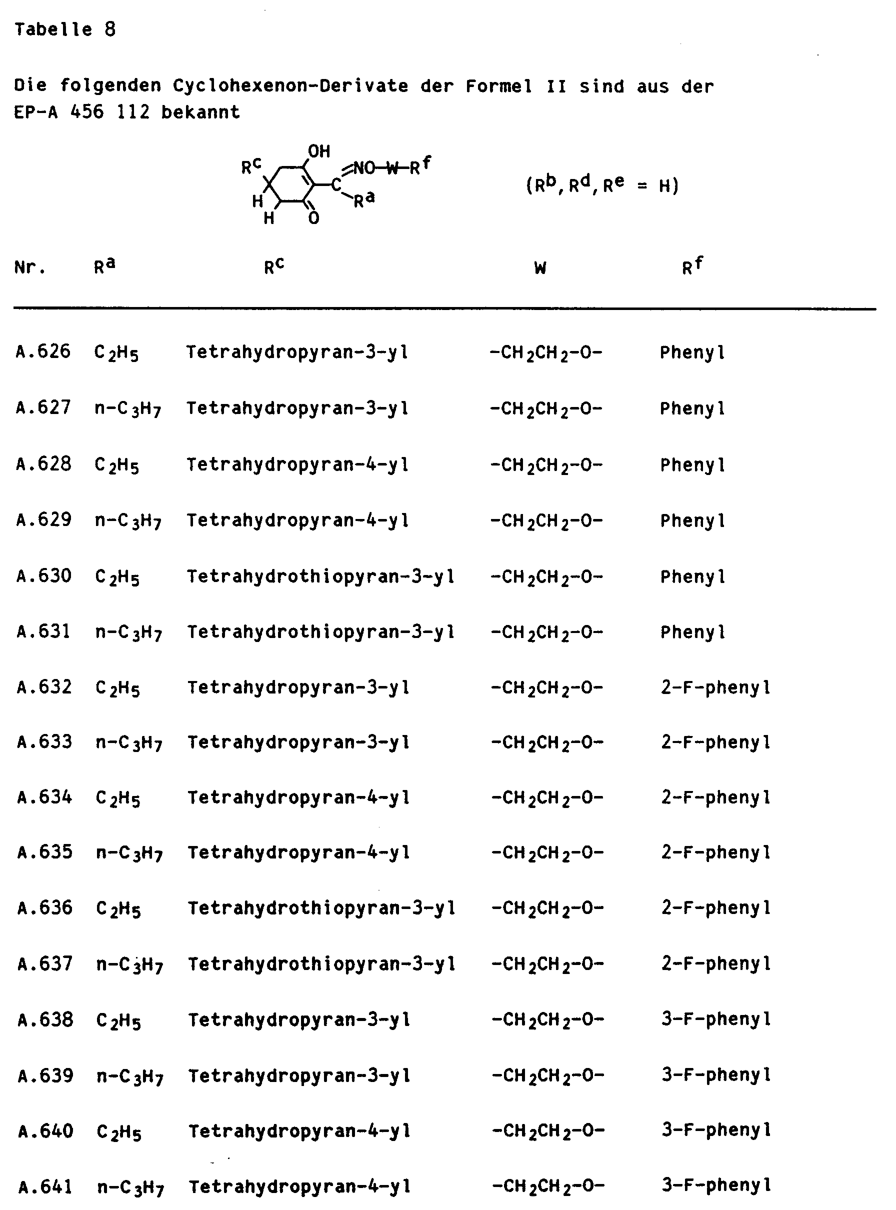

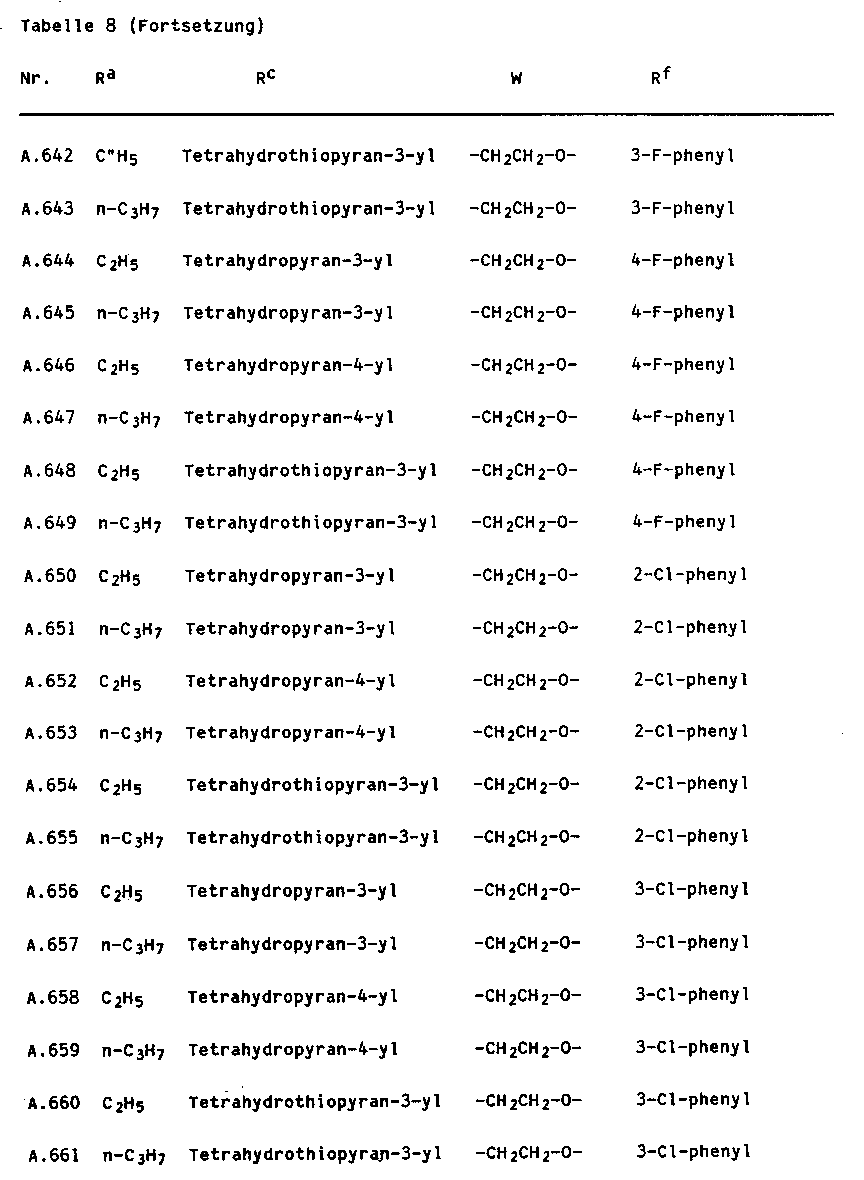

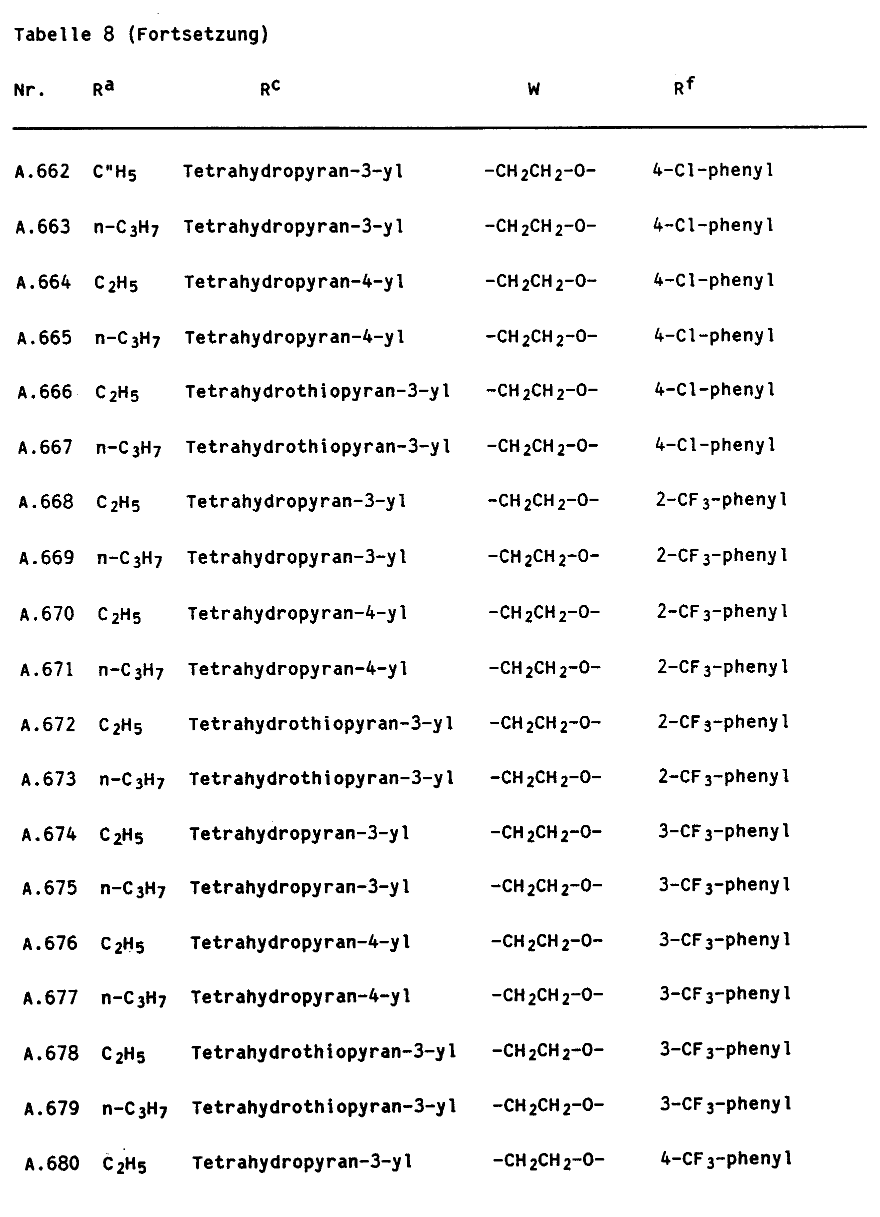

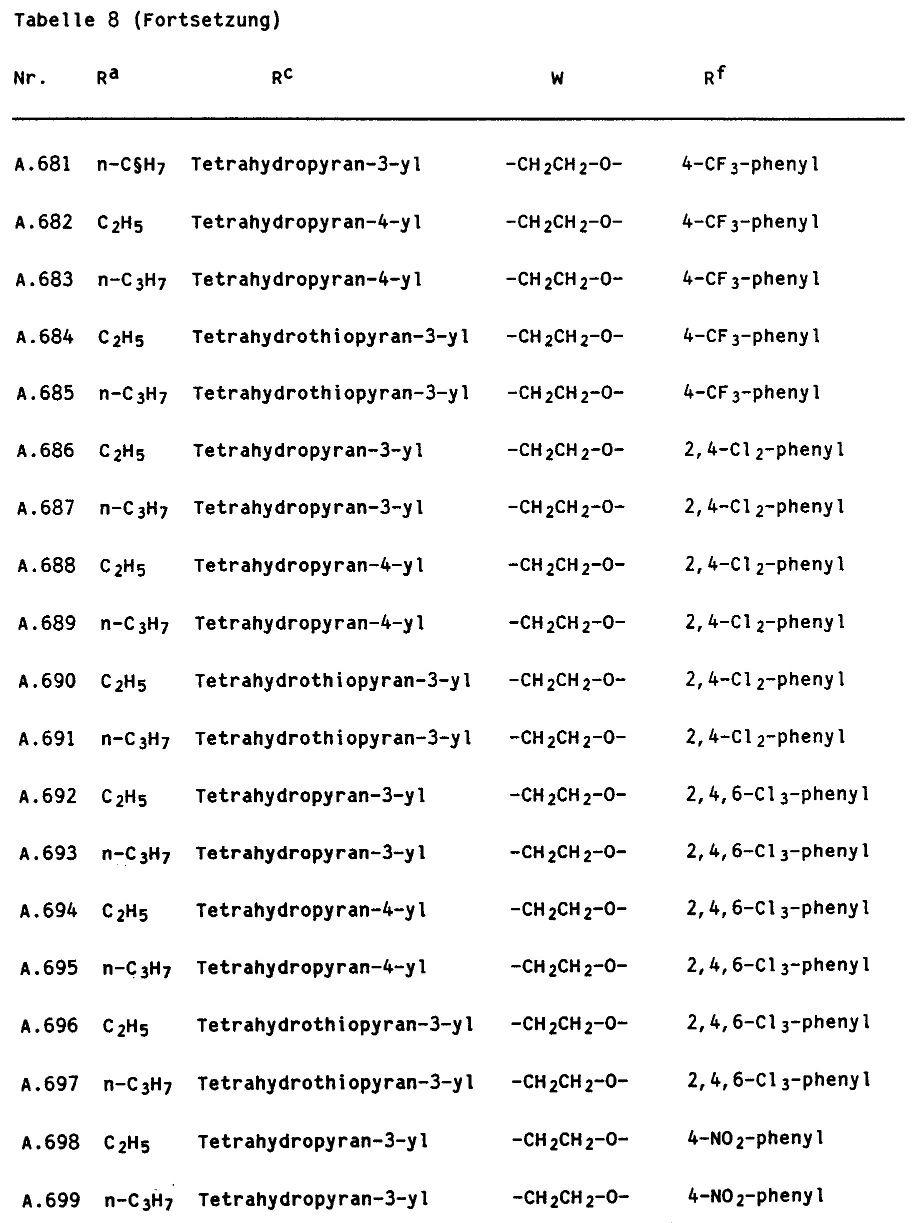

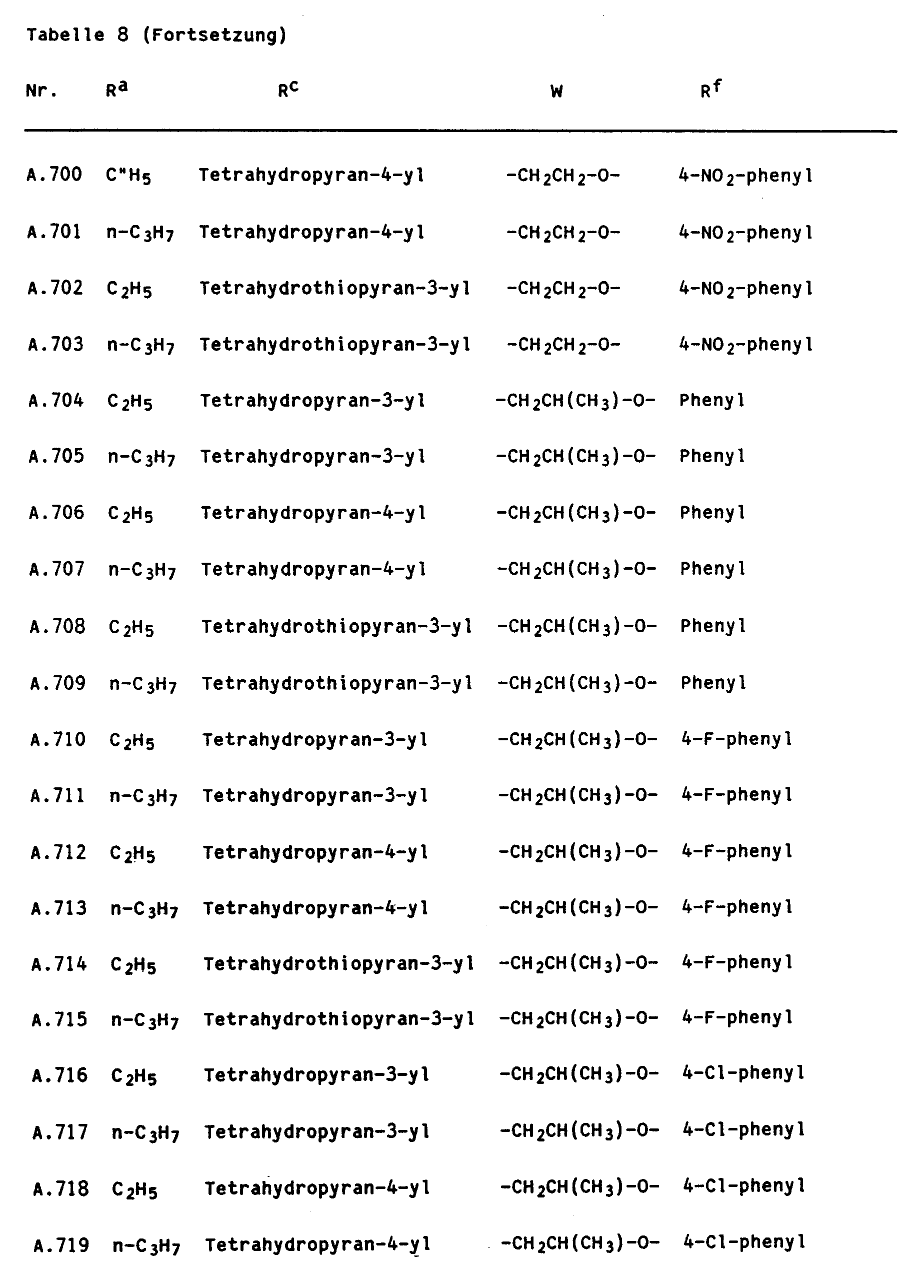

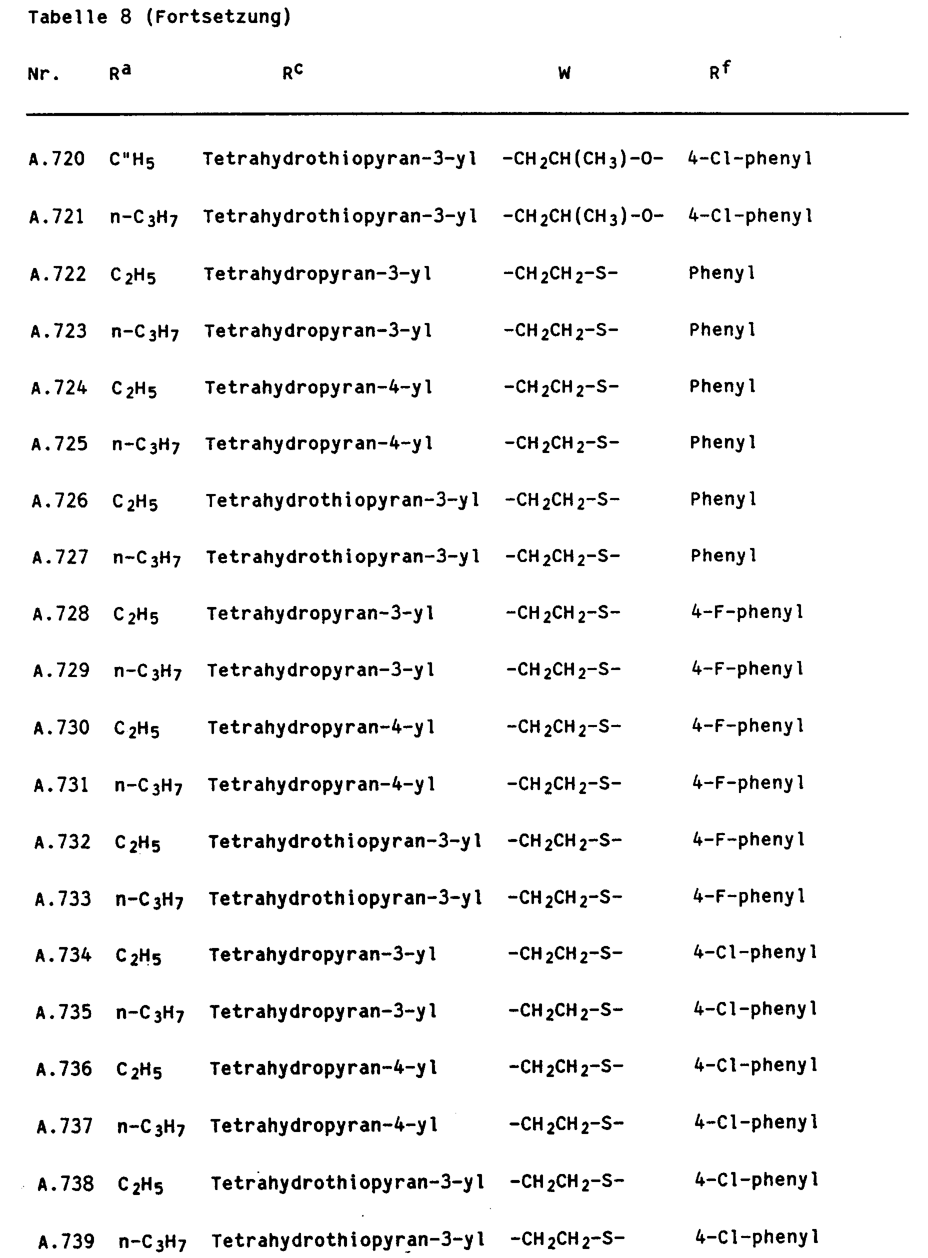

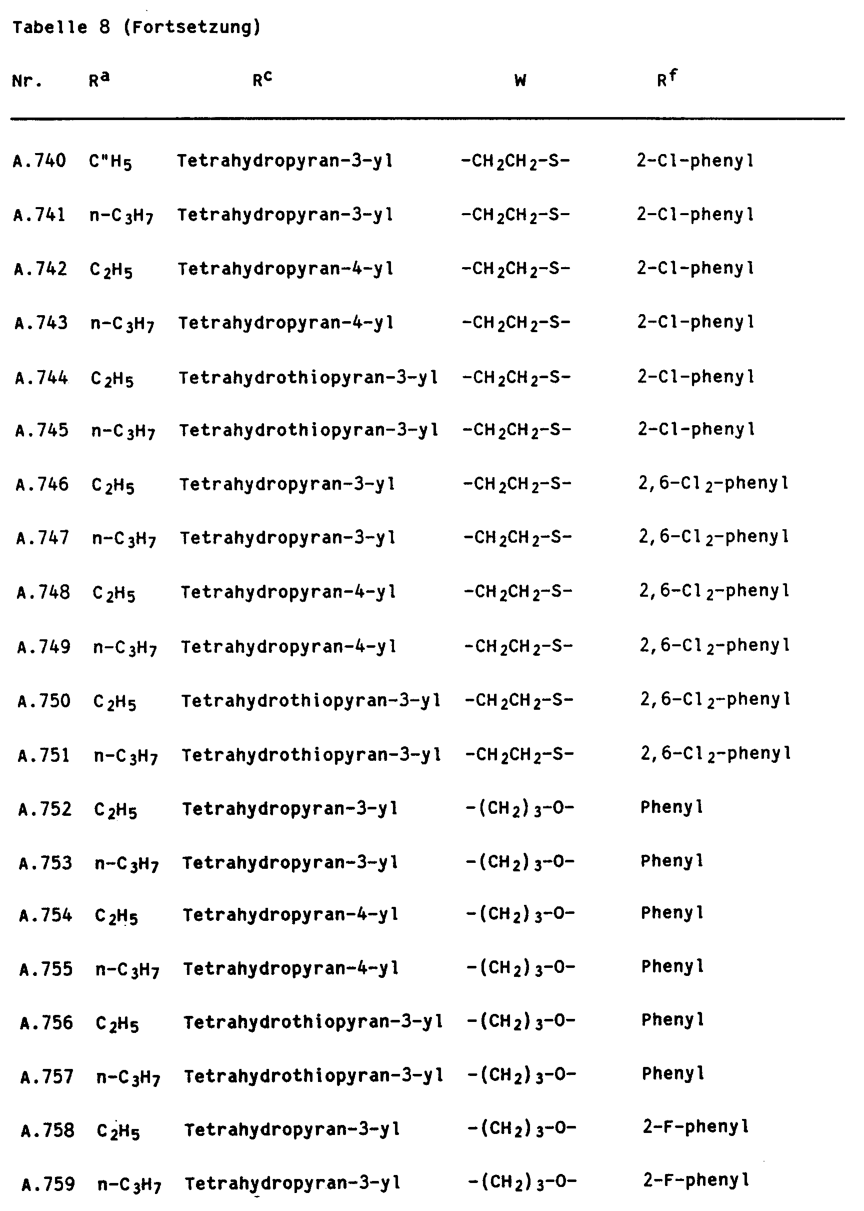

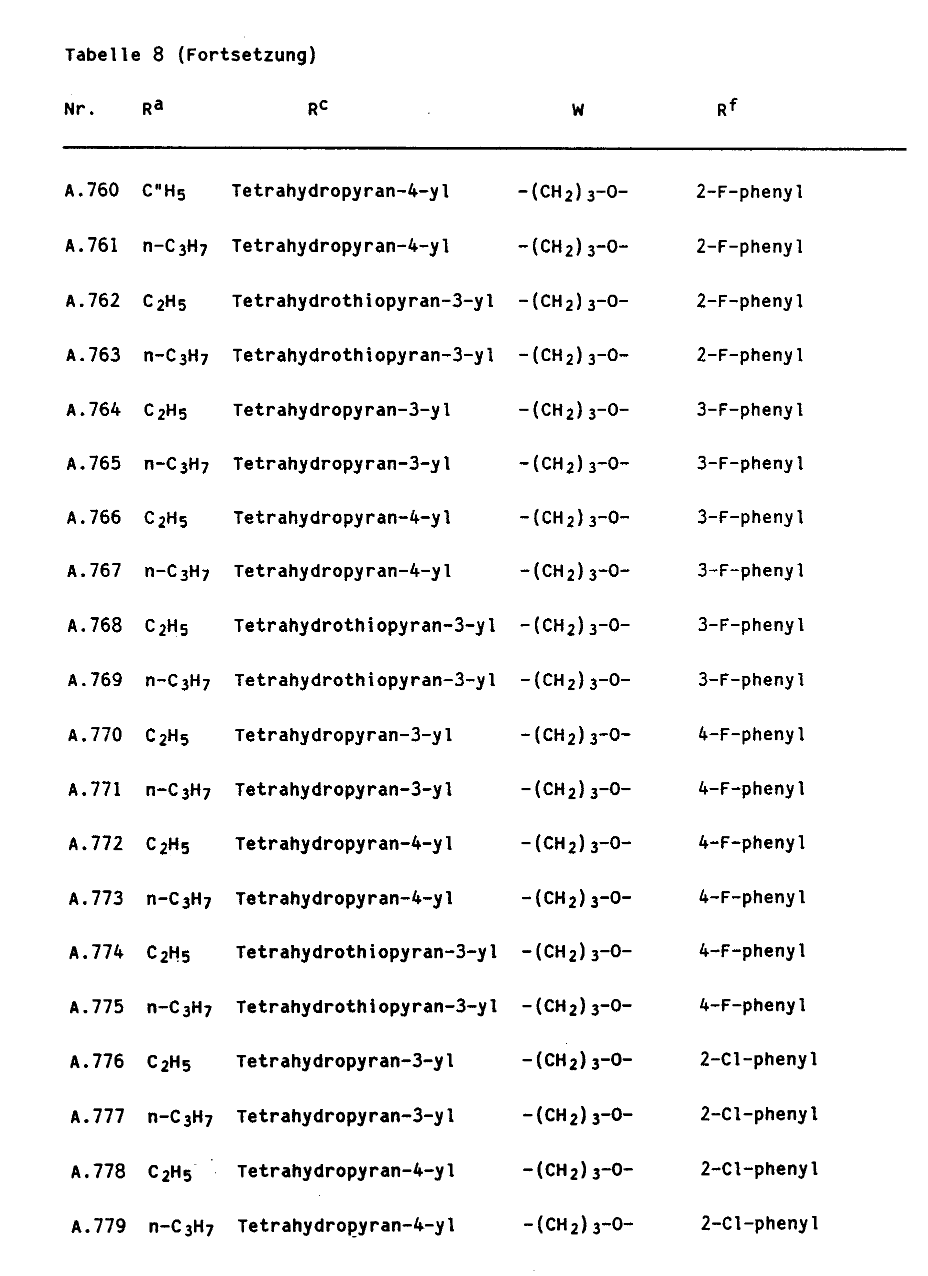

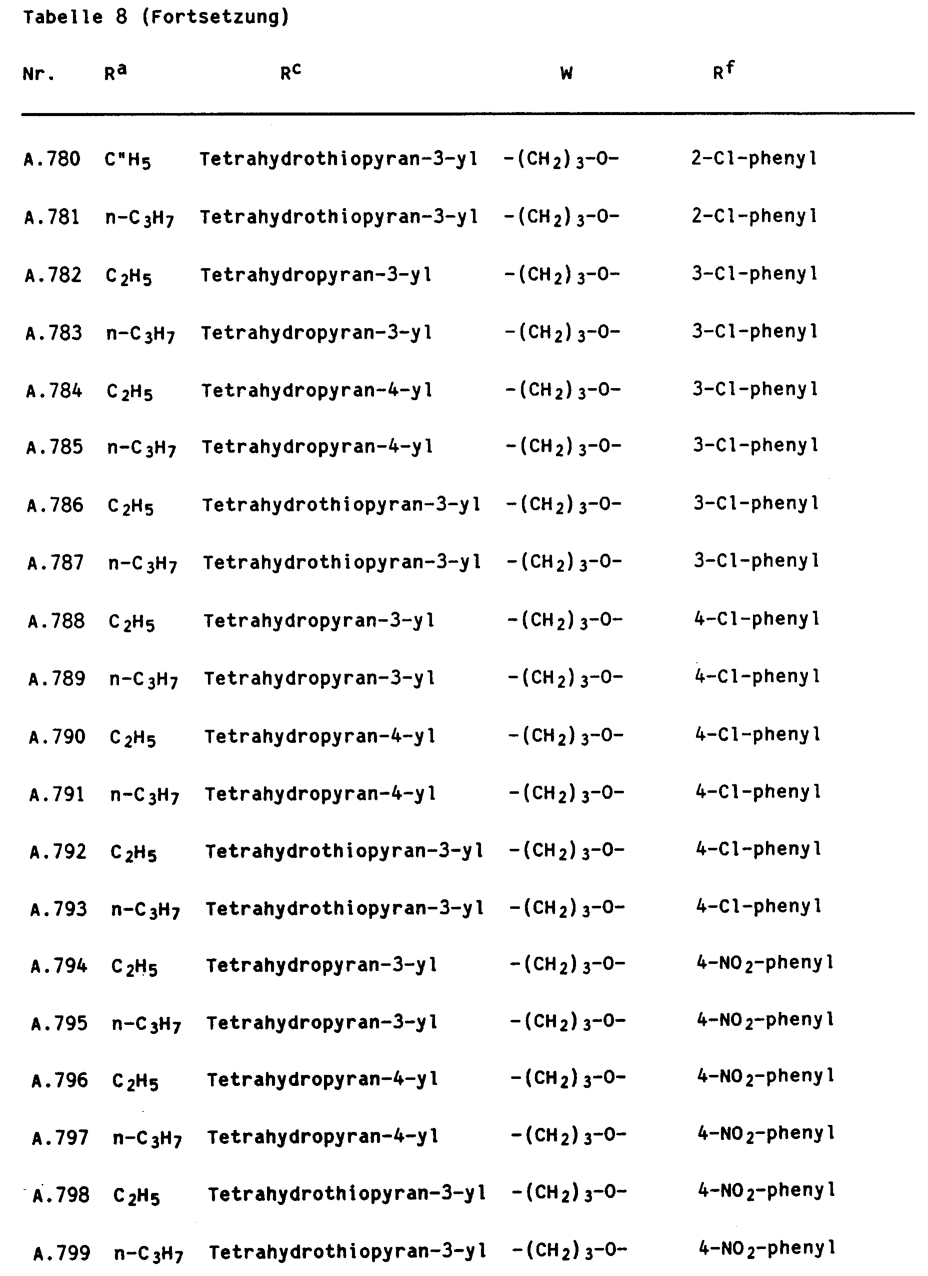

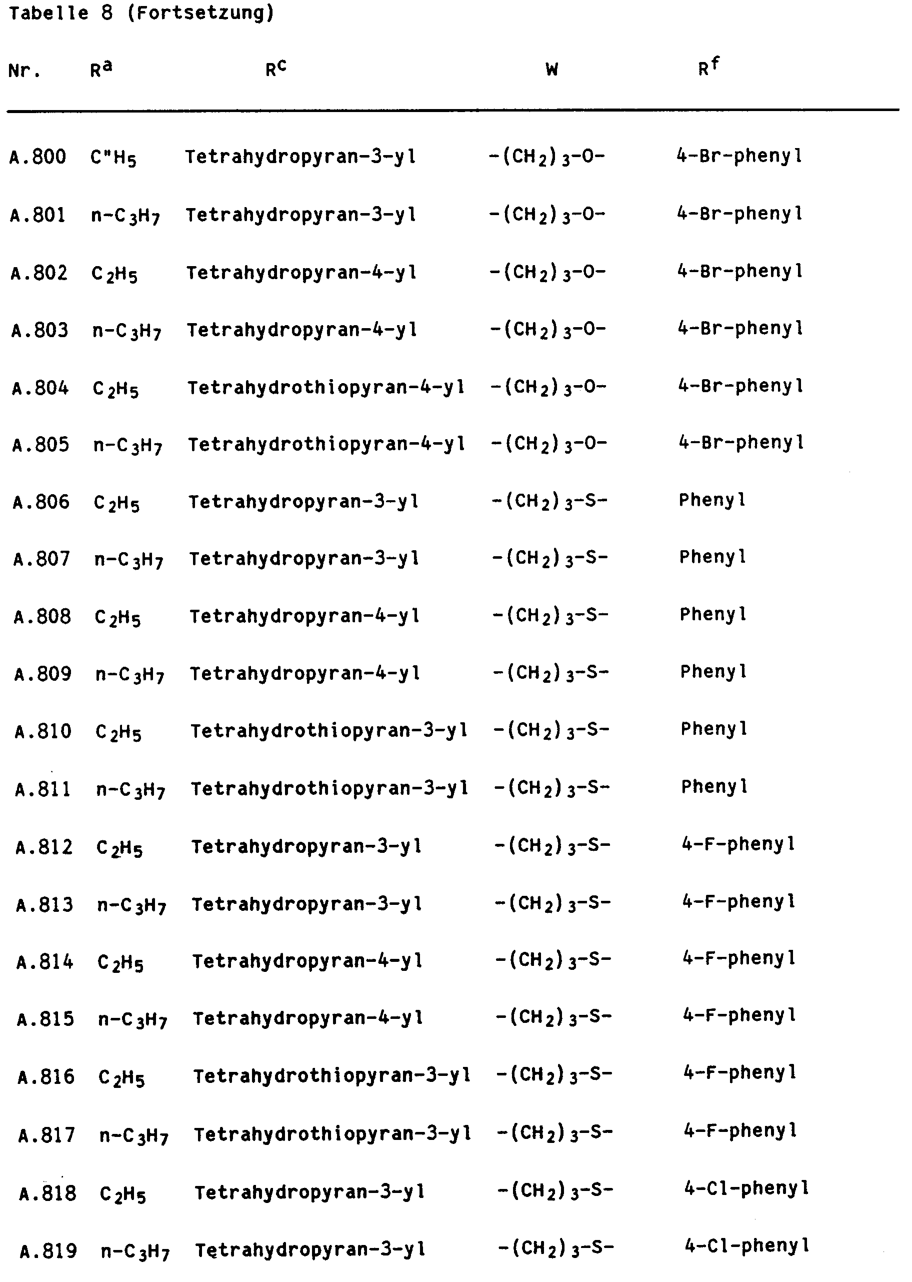

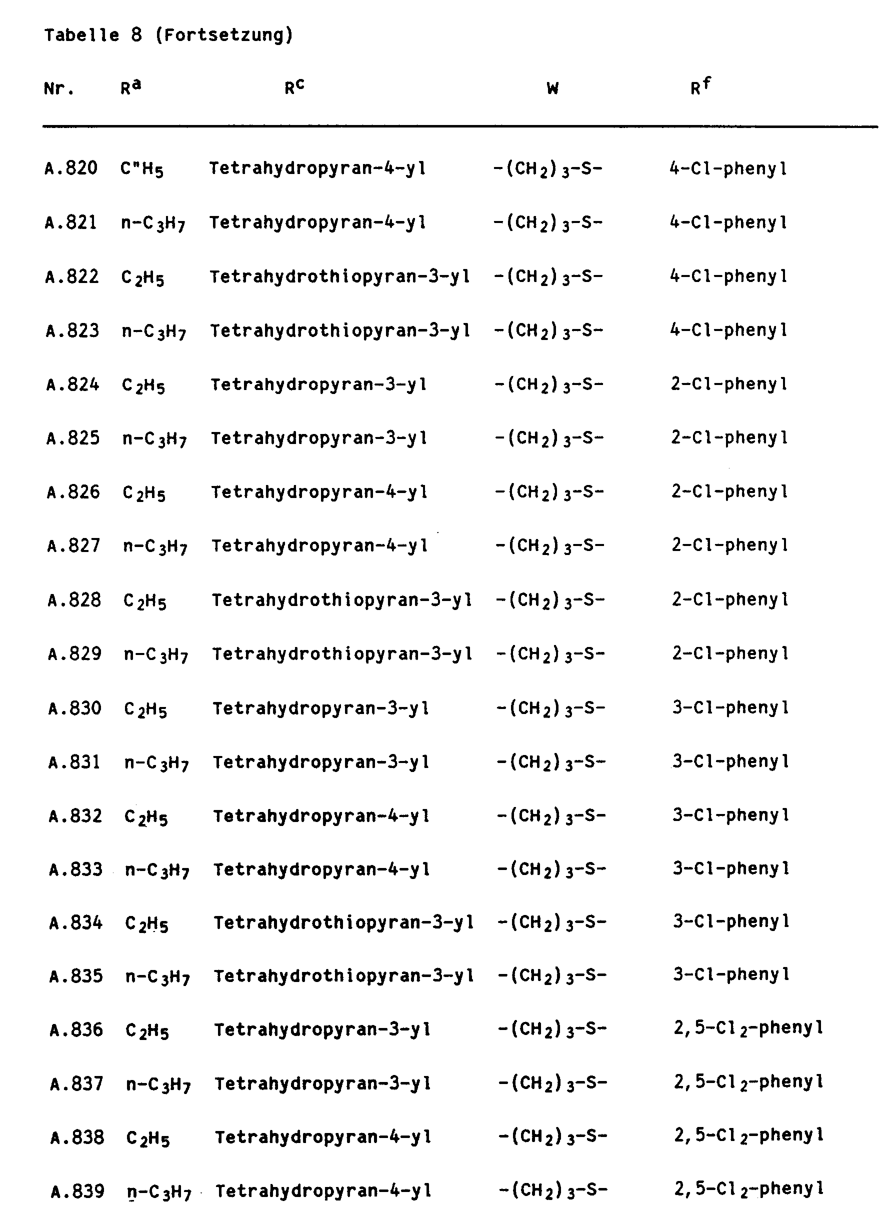

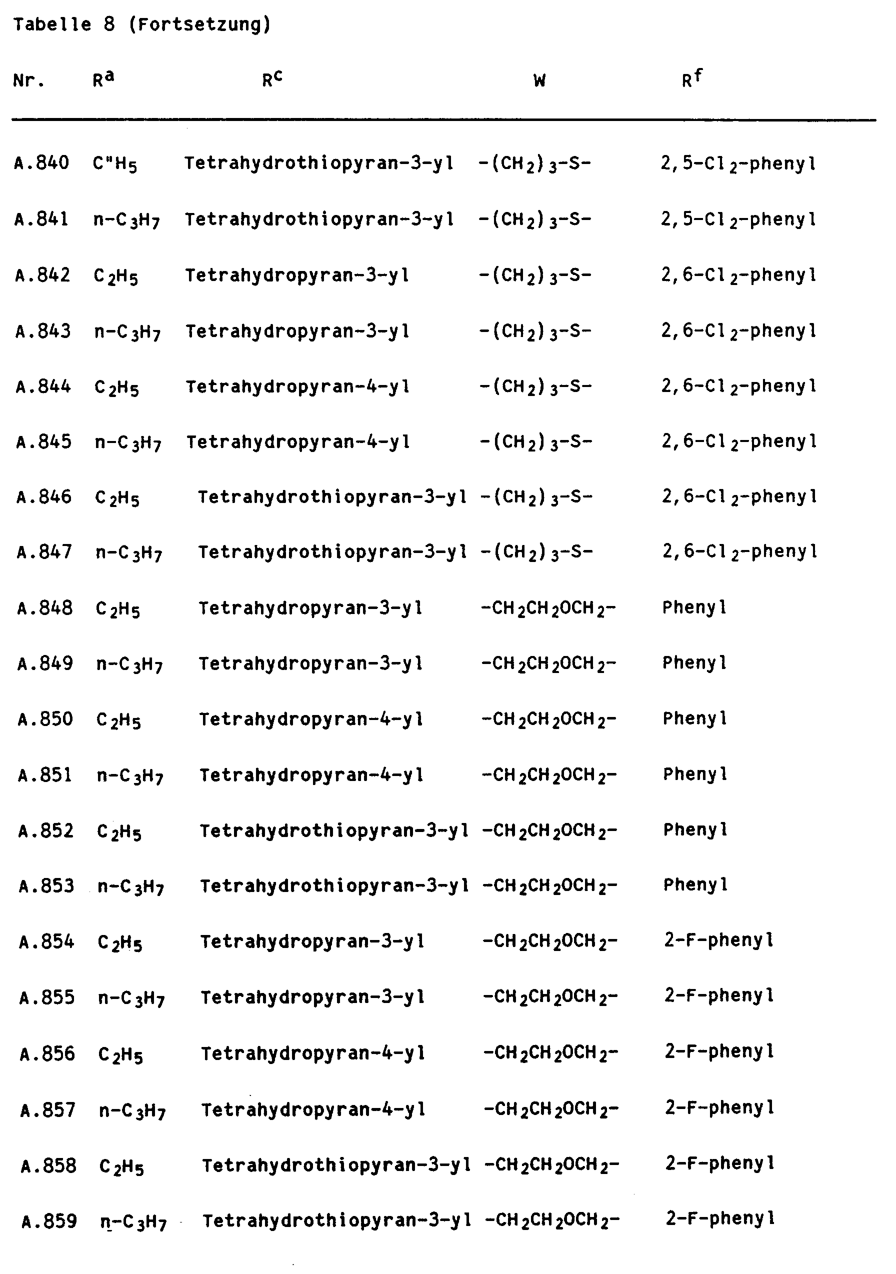

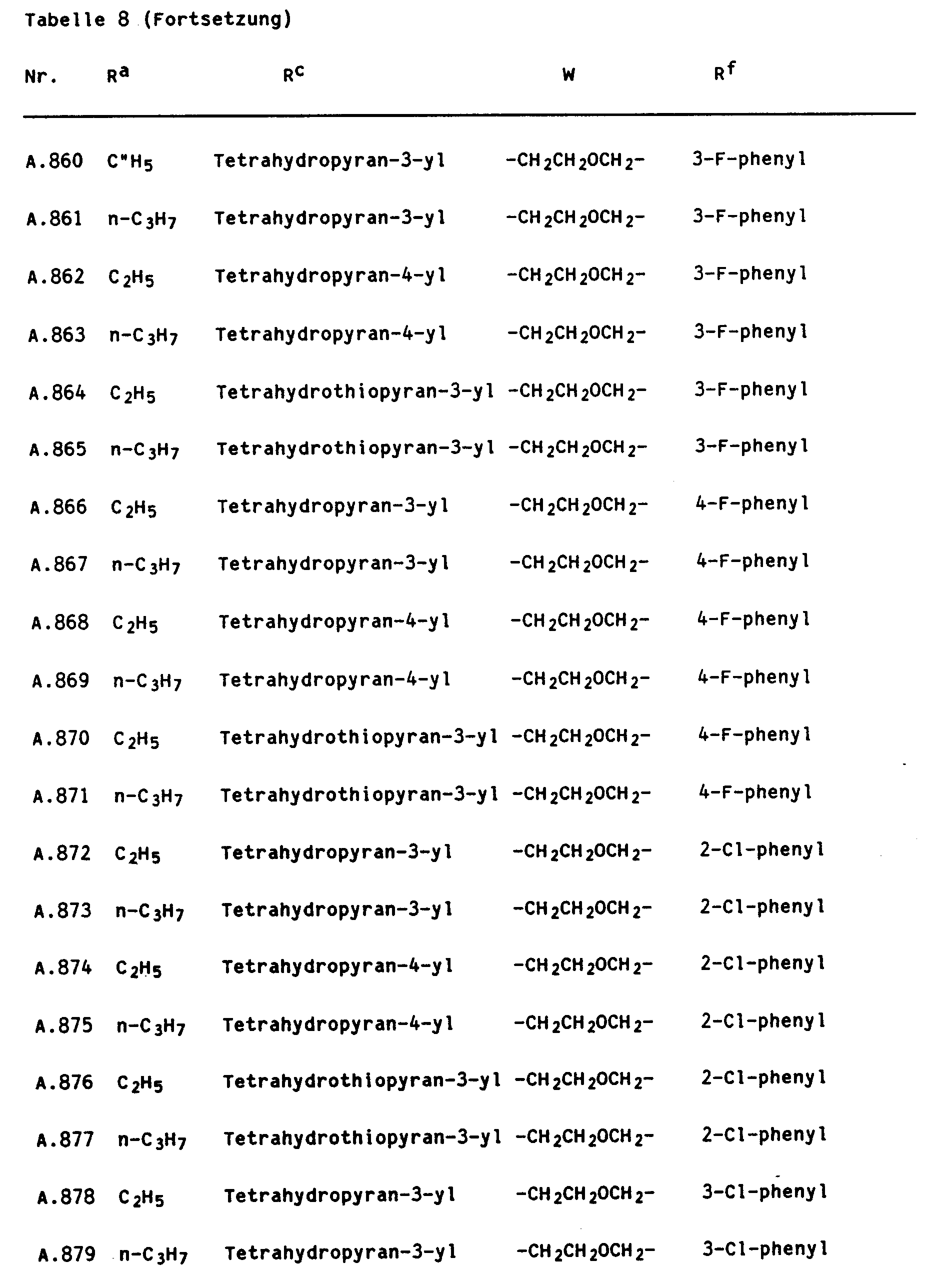

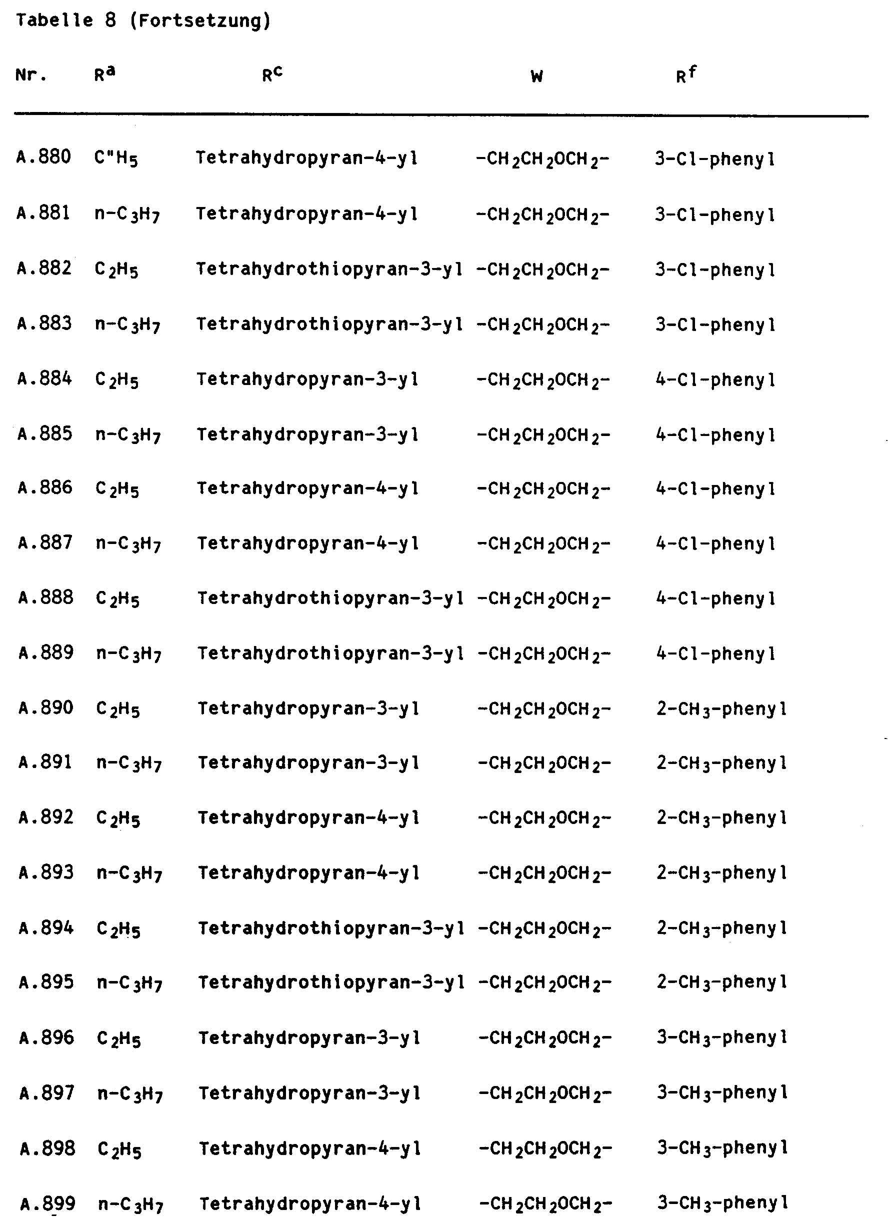

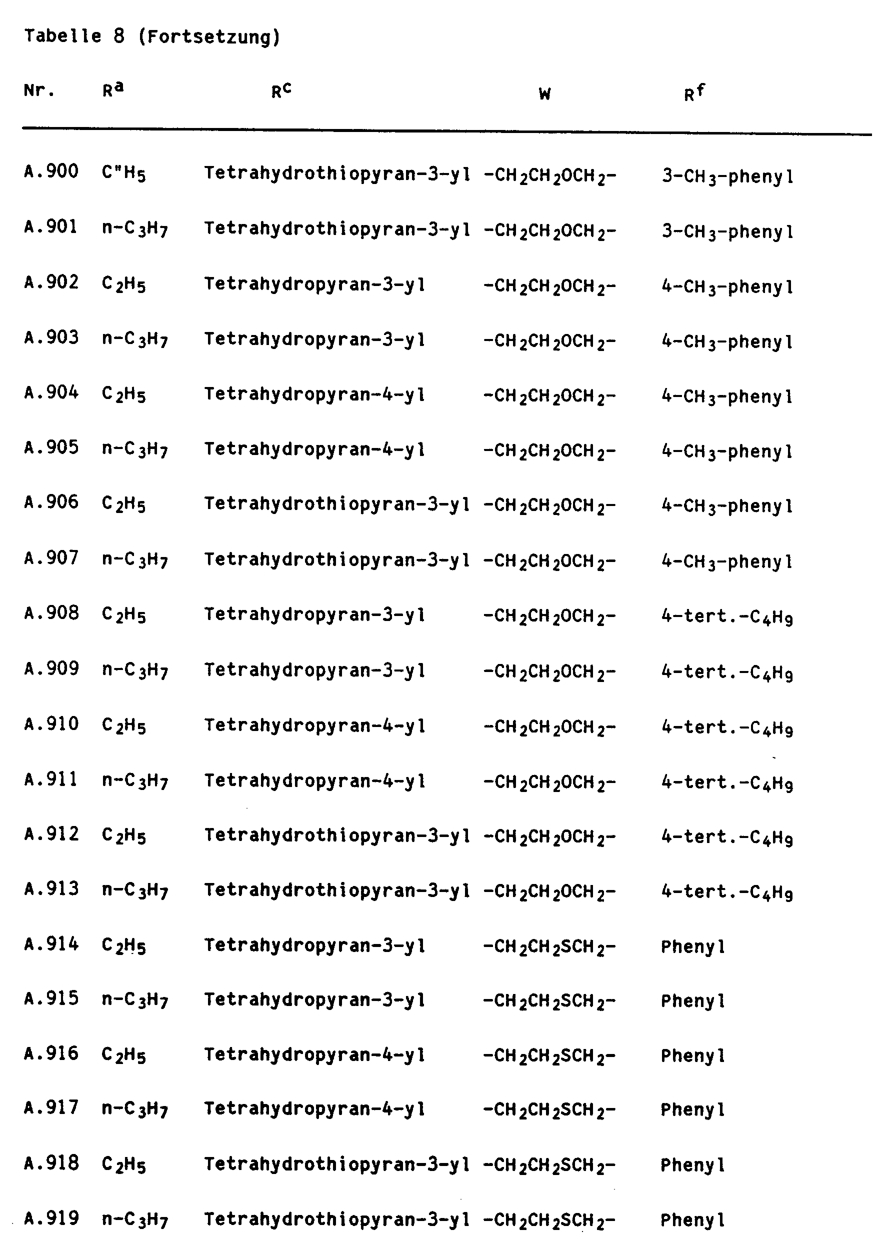

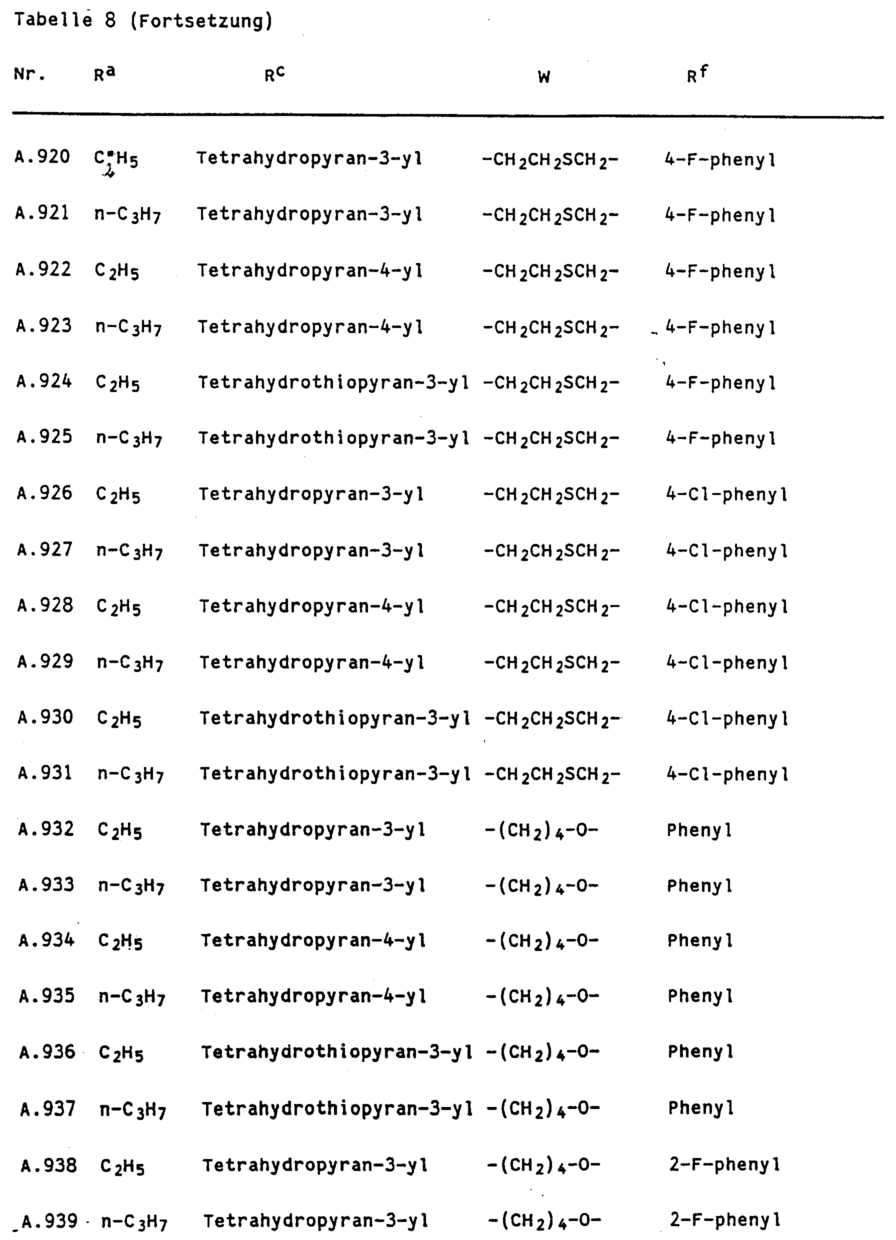

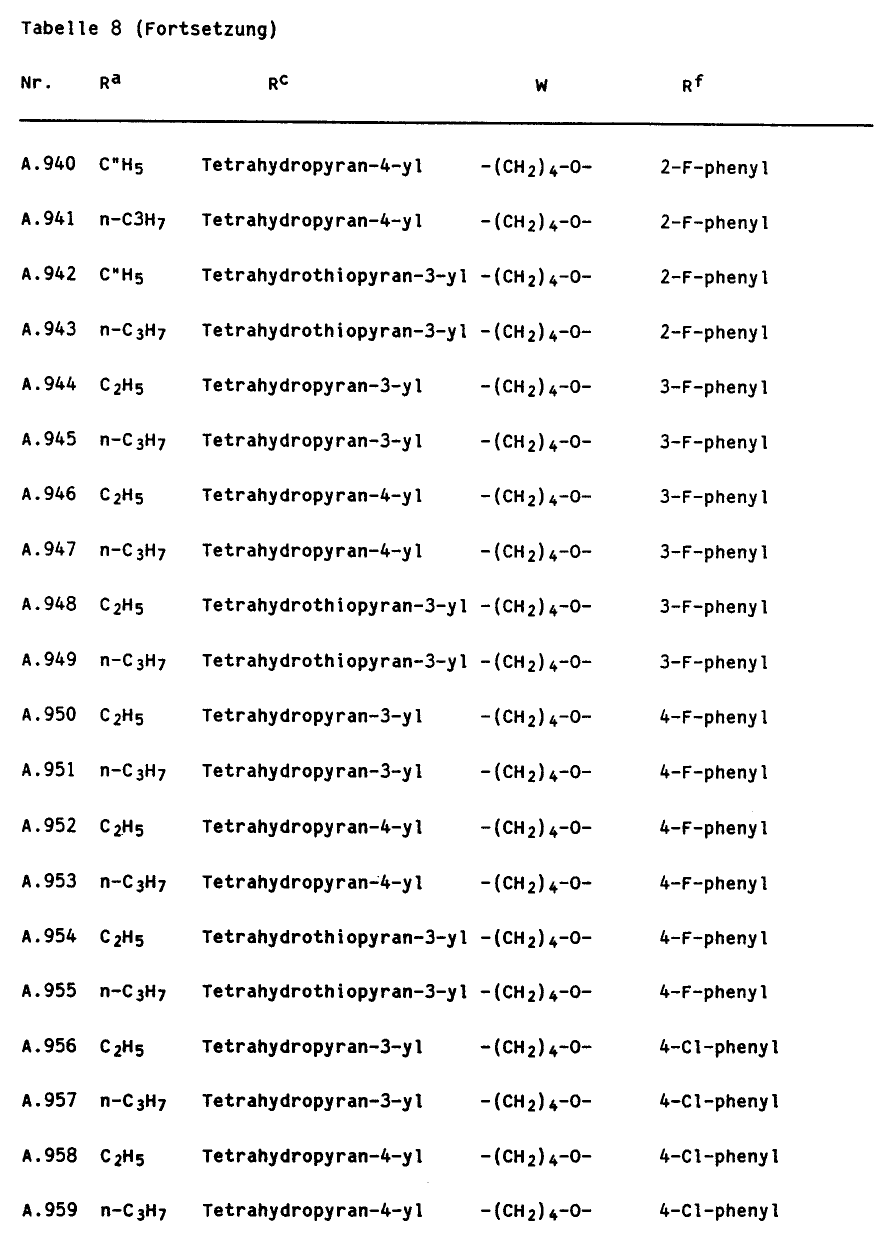

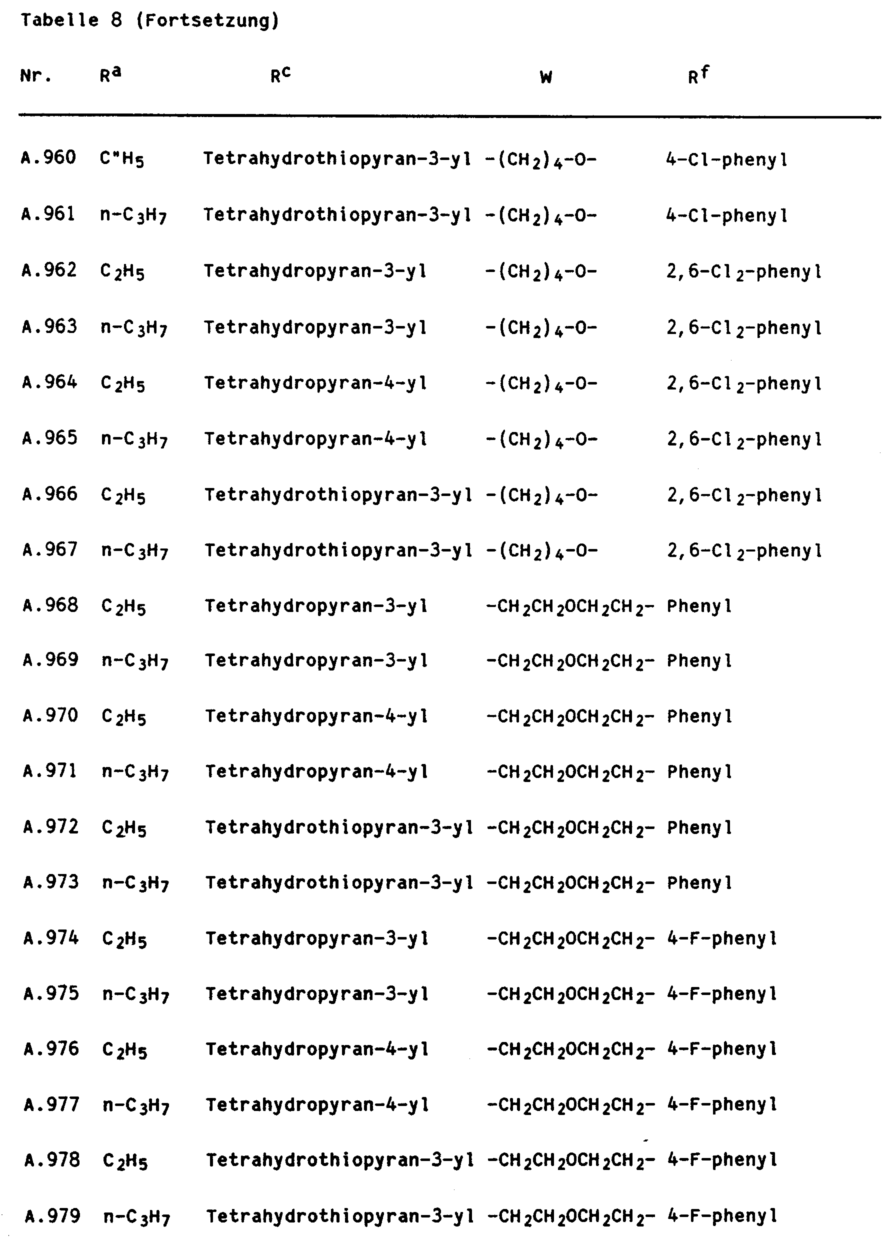

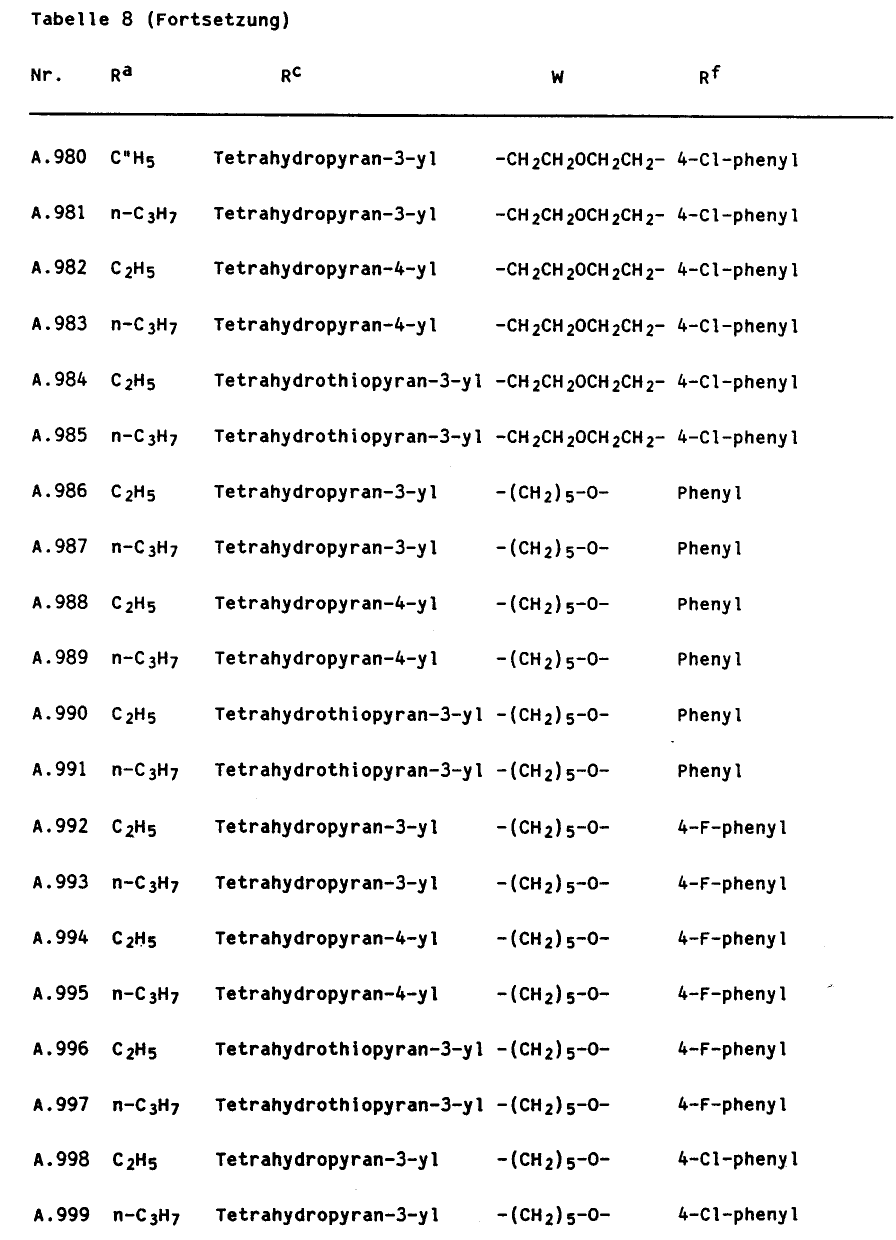

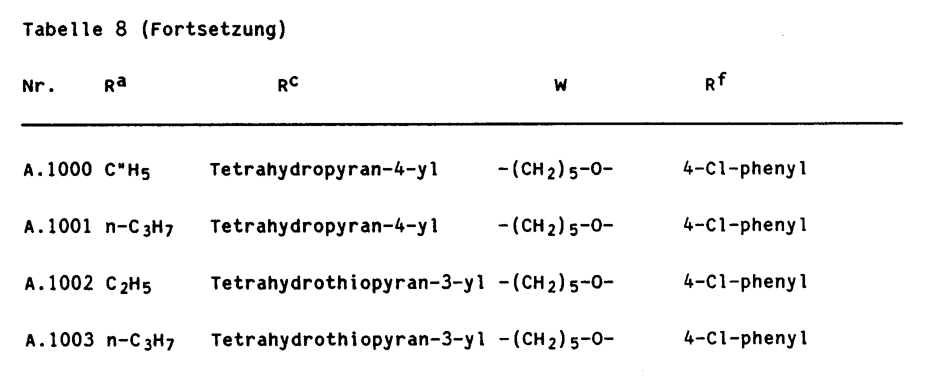

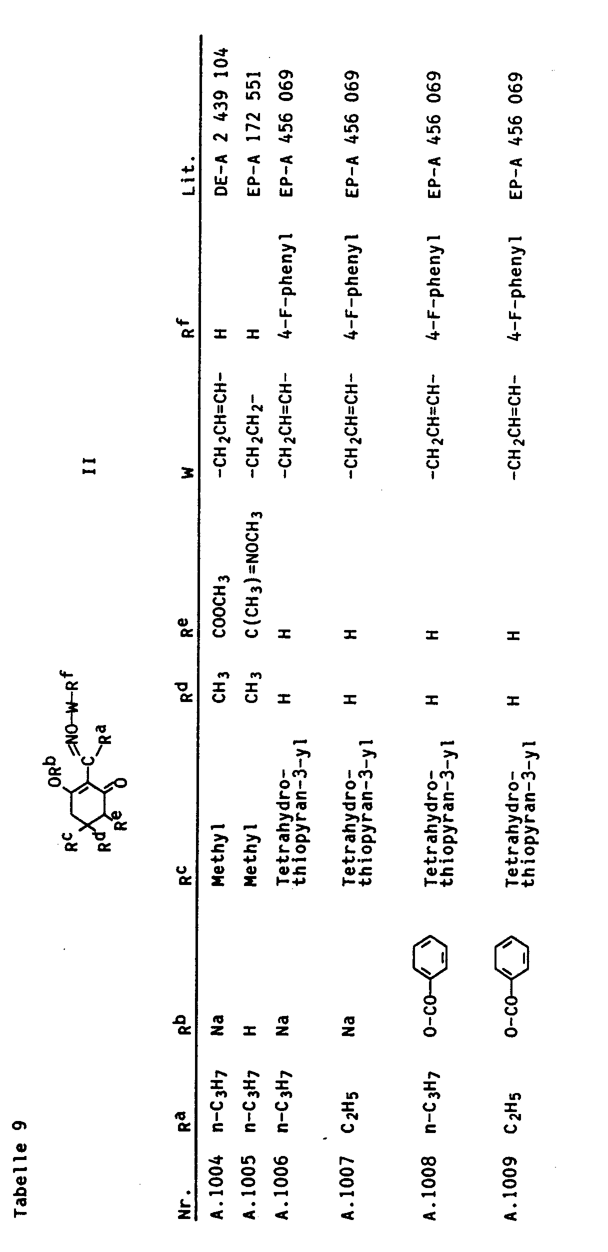

Die herbiziden Wirkstoffe aus der Gruppe der 2-(4-Heteroaryloxy)- oder 2-(4-Aryloxy)-phenoxycarbonsäurederivate der Formel III 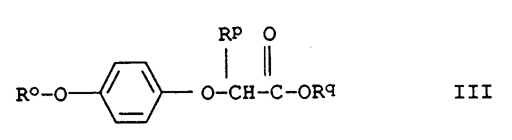 in der die Substituenten im einzelnen die folgende Bedeutung haben:
R^{o}
   die Phenylgruppe, die Pyridylgruppe, eine Benzoxazylgruppe, eine Benzthiazylgruppe oder eine Benzpyrazinylgruppe, wobei diese aromatischen Ringsysteme noch einen bis zwei Reste tragen können, ausgewählt aus einer Gruppe bestehend aus: Nitro, Halogen wie Fluor, Chlor, Brom und Jod, insbesondere Fluor und Chlor,
   unverzweigtem oder verzweigtem C₁-C₄-Alkyl wie Methyl, Ethyl, n-Propyl, Isopropyl, n-Butyl und tert.-Butyl, partiell oder vollständig halogeniertem C₁-C₄-Alkyl, insbesondere C₁-C₂-Halogenalkyl wie Chlormethyl, Dichlormethyl, Trichlormethyl, Fluormethyl, Difluormethyl, Trifluormethyl,
   Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, vorzugsweise Trifluormethyl, partiell oder vollständig halogeniertes C₁-C₄-Alkoxy, insbesondere C₁-C₂-Halogenalkoxy wie Chlormethoxy, Dichlormethoxy, Trichlormethoxy, Fluormethoxy, Difluormethoxy, Trifluormethoxy, Chlorfluormethoxy, Dichlorfluormethoxy, Chlordifluormethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy und Pentafluorethoxy, vorzugsweise Trifluormethoxy;
R^{p}
   Wasserstoff oder Methyl, bevorzugt Methyl,
R^{q}
   Wasserstoff, eine unverzweigte oder verzweigte C₁-C₄-Alkylgruppe wie vorstehend genannt, insbesondere Methyl, Ethyl, n-Propyl und n-Butyl, eine C₃-C₄-Alkenylgruppe wie Prop-2-en-1-yl, But-2-en-1-yl, But-3-en-1-yl, 1-Methyl-prop-2-en-1-yl, 2-Methyl-prop-2-en-1-yl, insbesondere Prop-2-en-1-yl, eine C3-C4-Alkinylgruppe wie Prop-2-in-1-yl, But-2-in-1-yl, But-3-in-1-yl, 1-Methyl-prop-2-in-1-yl, 2-Methyl-prop-2-in-1-yl, vorzugsweise Prop-2-in-1-yl, eine C1-C4-Alkoxy-C1-C4-alkylgruppe wie Methoxymethyl, Ethoxymethyl, n-Propoxymethyl, (1-Methylethoxy)methyl, n-Butoxymethyl, (1-Methylpropoxy)methyl, (2-Methylpropoxy)methyl, (1,1-Dimethylethoxy)methyl, Methoxyethyl, Ethoxyethyl, n-Propoxyethyl, (1-Methylethoxy)ethyl, n-Butoxyethyl, (1-Methylpropoxy)ethyl, (2-Methylpropoxy)ethyl, (1,1-Dimethylethoxy)ethyl, 3-(Methoxy)propyl, 2-(Methoxy)propyl und 2-(Ethoxy)propyl, vorzugsweise Methoxymethyl und Ethoxyethyl,
   eine C₃-C₄-Alkylideniminooxy-C₂-C₃-alkylgruppe, insbesondere 2-Propylideniminooxy-ethyl,
   eine Tetrahydrofuranylmethylgruppe, eine Isoxazolidingruppe oder das Äquivalent eines landwirtschaftlich brauchbaren Kations, beispielweise ei Alkalimetallkation wie Natrium oder Kalium, ein Äquivalent eines Erdalkalimetallkations wie Calzium, Magnesium und Barium, Mangan-, Kupfer-, Zink- oder Eisenkationen, Ammoniumkationen mit gewünschtenfalls einem bis drei Substituenten, ausgewählt aus einer Gruppe bestehend aus C₁-C₄-Alkylresten, Hydroxy-C₁-C₄-alkylresten, und einem Phenyl- oder Benzylrest, wie Tetraalkyl- und Benzyltrialkylammoniumkationen, Phosphoniumkationen, Sulfoniumkationen wie Trialkylsulfoniumkationen oder Sulfoxoniumkationen,
   sind aus der Literatur bekannt (vgl. z.B. DE-A 22 23 894, DE-A 24 33 067 DE-A 25 76 251, DE-A 30 04 770, DE-A 32 46 847, BE-A 868 875, BE-A 858 618, EP-A 054 715, EP-A 248 968, EP-A 323 127 und US 4,753,673).

Die 2-(4-Heteroaryloxy)- und 2-(4-Aryloxy-phenoxycarbonsäurederivate III können ein oder mehrere Asymmetriezentren enthalten. Sie wirken als Racemate, wie sie bei den meisten Herstellungsverfahren anfallen, können gewünschtenfalls aber auch nach den hierfür üblichen Methoden, als reine Isomere dargestellt oder aufgetrennt werden.

Sowohl die Racemate als auch die reinen Isomeren dienen zur Bekämpfung von unerwünschten Pflanzen aus der Familie der Gramineen. Die Verträglichkeit dieser Substanzen für Kulturpflanzen variiert jedoch zwischen kommerziell akzeptabel und unverträglich, je nach Substituenten und Aufwandmenge.

Spezielle Beispiele für herbizide 2-(4-Heteroaryloxy)- und 2-(4-Aryloxy)-phenoxycarbonsäurederivate der Formel III, deren Kulturpflanzenverträglichkeit durch substituierte Thiochromenone I verbessert werden kann, sind in der folgenden Tabelle 10 aufgeführt:

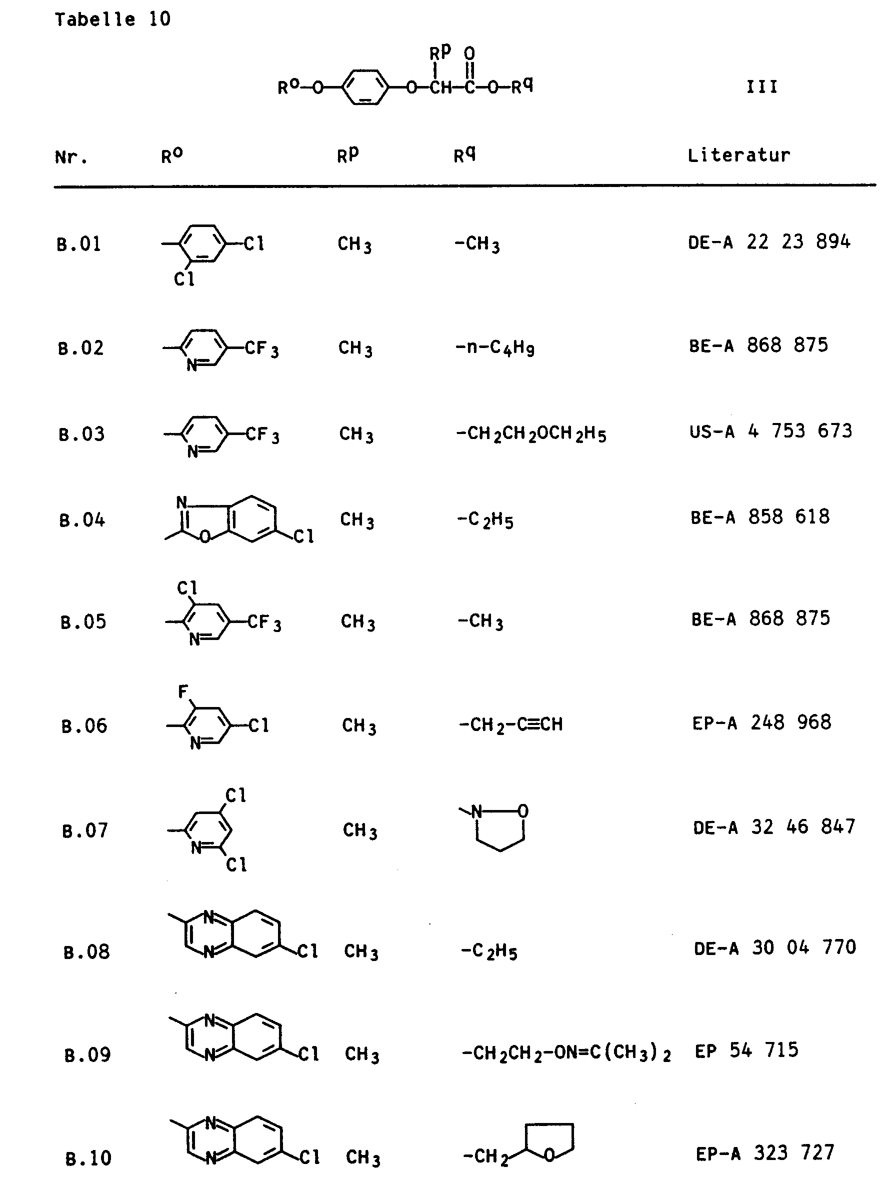

Die herbiziden Wirkstoffe und die antidotisch wirkenden Verbindungen können gemeinsam oder getrennt nach dem Auflaufen auf die Blätter und Sprossen der Kulturpflanzen und unerwünschten Gräser ausgebracht werden. Bevorzugt bringt man jedoch die herbiziden und antidotischen Wirkstoffe gleichzeitig auf das Feld. Bei getrennter Ausbringung von Antidot und herbizidem Wirkstoff wird vorzugsweise das Antidot zuerst ausgebracht.

Der antidotische und der herbizide Wirkstoff können gemeinsam oder getrennt formuliert werden und dann in suspendierbarer, emulgierbarer oder löslicher Form zur Bereitung von Spritzmitteln vorliegen.

Antidotische Effekte werden auch durch Bahandlung der Kulturpflanzensamen oder der Stecklinge mit dem Antidot vor der Aussaat bzw. vor dem Auspflanzen erzielt. Der herbizide Wirkstoff wird dann allein in der üblichen Weise appliziert.

Bei der Saatgutbehandlung werden im allgemeinen Wirkstoffmengen von 0,1 bis 10 g, vorzugsweise 1 bis 2 g, je Kilogramm Saatgut benötigt.

Bei der Applikation des Antidots durch Samenquellung oder bei der Stecklingsbehandlung werden bevorzugt Lösungen eingesetzt, die den antagonistischen Wirkstoff in einer Konzentration von 1 bis 10000 ppm, insbesondere von 100 bis 10000 ppm, enthalten.

In den verschiedenen Pflanzenkulturen benötigt man üblicherweise unterschiedliche Mengen an antidotisch wirksamer Verbindung I und herbizider Verbindung II oder III, wobei die Mengenverhältnisse in breiten Bereichen variabel sind. Sie sind abhängig von der Struktur der Cyclohexenon-Derivate II bzw. der Heteroaryloxy- und Aryloxyphenoxyessigsäurederivate III, der substituierten Thiochromenone I und der jeweiligen Pflanzenkultur, auf die die Verbindungen ausgebracht werden. Geeignete Anteilsverhältnisse von herbizidem Wirkstoff zu antidotisch wirksamen substituierten Thiochromenone I liegen zwischen 1:10 und 1:0,01, vorzugsweise zwischen 1:4 und 1:0,1.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentigen wäßrigen, öligen oder sonstigen Suspensionen, Dispersionen, Emulsionen, Öldispersionen, Pasten, Stäubemitteln, Streumitteln oder Granulaten durch Versprühen, Vernebeln, Verstäuben, Verstreuen oder Gießen angewendet. Die Anwendungsform richtet sich hierbei ganz nach dem jeweiligen Verwendungszweck.

Zur Herstellung von direkt versprühbaren Lösungen, Emulsionen, Pasten und Öldispersionen kommen Mineralölfraktionen von mittlerem bis hohem Siedepunkt, wie Kerosin und Dieselöl, ferner Kohlenteeröle, sowie Öle und Fette pflanzlichen oder tierischen Ursprungs, aliphatische, cyclische oder aromatische Kohlenwasserstoffe, beispielsweise Methanol, Ethanol, Isopropanol, Butanol, Chloroform, Tetrachlorkohlenstoff, Cyclohexanol, Cyclohexanon, Chlorbenzol, Toluol, Xylole, Paraffin, Tetrahydronaphthalin, alkylierte Naphthaline oder deren Derivate oder Isophoron, sowie stark polare Lösungsmittel wie Dimethylformamid, Dimethylsulfoxid, N-Methylpyrrolidon und vorzugsweise Wasser, in Betracht.

Wäßrige Anwendungsformen können aus Emulsionskonzentraten, Pasten, netzbaren Pulvern (Spritzpulvern) oder Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mittels Netz-, Haft-, Dispergier- oder Emulgiermittel mit Wasser homogenisiert werden. Es können aber auch aus herbizidem Wirkstoff und/oder Antidot Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und gewünschtenfalls Lösungsmittel oder Öl bestehende Konzentrate hergestellt werden, die zur Verdünnung mit Wasser geeignet sind.

Als oberflächenaktive Salze kommen Alkalimetall-, Erdalkalimetall-, Ammoniumsalze von Ligninsulfonsäure, Naphthalinsulfonsäure, Phenolsulfonsäure, Alkylarylsulfonate, Alkylsulfate, Alkylsulfonate, Alkalimetall- und Erdalkalimetallsalze der Dibutylnaphthalinsulfonsäure, Laurylethersulfat, Fettalkoholsulfate, fettsaure Alkalimetall- und Erdalkalimetallsalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctyl-phenolether, ethoxyliertes Isooctylphenol, Octylphenol oder Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether ethoxyliertes Polyoxypropylen, Laurylalkoholpolyglykoletheracetal, Sorbitester, Lignin-Sulfitablaugen und Methylcellulose in Betracht.

Pulver, Streu- und Stäubemittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogenisierungsgranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Lös, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe, und pflanzliche Produkte wie Getreidemehl, Baumrinden-, Holz- und Nußschalenmehl, Cellulosepulver und andere feste Trägerstoffe.

Die Formulierungen enthalten 0,02 bis 95 Gew.%, vorzugsweise 0,5 bis 9 Gew.% an herbizidem Wirkstoff und Antidot. Die Aufwandmengen an herbizidem Wirkstoff betragen 0,05 bis 5 kg/ha.

Die herbiziden Mittel können neben den antagonistisch wirksamen substituierten Thiochromenone I und dem Herbizid aus der Gruppe der Cyclohexenone II oder der (Heteroaryloxy)- bzw. Aryloxyphenoxycarbonsäuren III weitere herbizide oder wachstumsregulierende Wirkstoffe anderer chemischer Struktur enthalten, wobei der antagonistische Effekt der substituierten Thiochromenone I erhalten bleibt.

### Herstellungsbeispiele (erfindungsgemäße substituierte Thiochromenone):

### Beispiel 1

### 2-Methylthiochromen-4(1H)-on

100 g Polyphosphorsäure werden bei 80°C vorgelegt. Innerhalb von 30 min tropft man ein Gemisch aus 11 g (100 mmol) Thiophenol und 13 g (100 mmol) Acetessigester zu. Es wird 15 min nachgerührt, auf 200 g Eis gegeben und abgesaugt; Ausbeute: 17,0 g (97 %) farblose Kristalle mit Schmp. 70°C, Wirkstoffbeispiel 1.1.

In analoger Weise wurden hergestellt:

**Tabelle A.1**

| Nr. | Verb. I | | R³ | Schmp. [°C] |
|---|---|---|---|---|
| | R¹ | R² | | |
| 1.1 | Me | H | H | 70 |
| 1.2 | Me | H | 8-Cl | 95 |
| 1.3 | Me | H | 8-CO₂H | 74 |
| 1.4 | Me | H | 6-CMe₃ | Öl |
| 1.5 | Me | H | 6-Cl | 118 |
| 1.6 | Me | Me | 8-Cl | 123 |
| 1.7 | Me | Me | 8-CO₂H | >250 |
| 1.8 | Me | Me | H | 103 |
| 1.9 | Me | Me | 6-Cl | 116 |
| 1.10 | Me | Me | 6-CMe₃ | Öl |
| 1.11 | Me | Et | H | 57 |
| 1.12 | Me | Et | 8-Cl | 87 |
| 1.13 | Me | Et | 8-CO₂H | 195 |
| 1.14 | Me | Et | 6-Cl | 132 |
| 1.15 | Me | Et | 6-CMe₃ | Öl |
| 1.16 | Ph | H | H | Öl |

### Beispiel 2

### 6-Chlor-4(1H)-oxo-thiochromen-2-carbonsäure

12,9 g (50 mmol) 4-Chlorphenylmercaptofumarsäure werden zu 50 ml konz. Schwefelsäure gegeben, so daß die Innentemperatur 35°C nicht übersteigt. Nach 30 min bei Raumtemperatur wird auf Eis gegeben und abgesaugt; Ausbeute: 6,7 g (56 %) farbloser Feststoff mit Schmp. 140-145°C, Wirkstoffbeispiel 2.3.

Das Edukt ist z.B. wie folgt herstellbar:

### 4-Chlorphenylmercaptofumarsäure

Eine Lösung von 16,8 g (0,3 mol) Kaliumhydroxid und 14,5 g (0,1 g) 4-Chlorthiophenol wird in 40 ml Wasser bei Raumtemperatur vorgelegt. Man tropft eine Suspension von 21 g (0,14 mol) Acetylendicarbonsäure-monokaliumsalz in 60 ml Wasser zu und erhitzt die Mischung auf 100°C. Nach 45 min bei dieser Temperatur wird auf 0°C abgekühlt und durch Zugabe von konz. Salzsäure pH = 2 eingestellt. Es wird abgesaugt, der Feststoff mit Wasser gewaschen und i.Vak. getrocknet; Ausbeute: 17,6 g (68 %) farblose Nadeln mit Schmp. 222-226°C.

Analog diesem 2-stufigen Verfahren sind erhältlich:

**Tabelle A.2**

| Nr. | Verb. I | | R³ | Schmp. [°C] |
|---|---|---|---|---|
| | R¹ | R² | | |
| 2.1 | H | H | H | 198 |
| 2.2 | CO₂H | H | H | 233-236 |
| 2.3 | CO₂H | H | 6-Cl | 140-145 |
| 2.4 | CO₂H | H | 6-CMe₃ | <35 |
| 2.5 | CO₂H | H | 8-CO₂H | 150 |
| 2.6 | H | H | 6-CMe₃ | Öl |

### Beispiel 3

### 2-Amino-4(1H)-oxothiochromen-3-carbonitril

10 g (47 mmol) 2-Acetylmercaptobenzoesäure und 20 ml Oxalylchlorid werden in 50 ml absol. Toluol 30 min bei 100°C gerührt. Die Lösung wird eingeengt, bis ein zähfließendes Öl verbleibt. Das Öl wird in 1 Portion zu einer gerührten Mischung aus 7 g (106 mmol) Malodinitril in 100 ml 1 % Natronlauge gegeben. Nach 5 min gibt man erneut 7 g (106 mmol) Malodinitril und 5 ml 20 % Natronlauge zu. Es wird 20 min nachgerührt, auf 50°C Innentemperatur gebracht und mit 5 g Kaliumhydroxid (als 50 % wäßrige Lösung) versetzt. Die homogene Mischung wird bei 0°C mit konz. Salzsäure auf pH = 4 gebracht, der Feststoff abgesaugt und i.Vak. getrocknet; Ausbeute: 6,5 g (68 %) beiges Thiochromenon mit Schmp. >240°C, Wirkstoffbeispiel 3.1.

In ähnlicher Weise wurden Thiochromenone mit anderen Substitutionsmustern im heterocyclischen Teil hergestellt:

**Tabelle A.3**

| Nr. | Verb. I | | R³ | Schmp. [°C] |
|---|---|---|---|---|
| | R¹ | R² | | |
| 3.1 | NH₂ | CN | H | >240 |
| 3.2 | NH₂ | CO₂Et | H | 235 |
| 3.3 | OH | CO₂Me | H | 217 |

Als Beispiel für eine Funktionalisierung eines Thiochromenons sei genannt:

### Beispiel 4

### 2(4'-Chlorbenzoyl)amino-4(1H)-oxothiochromen-3-carbonitril

Eine Mischung aus 5 g (25 mmol) 2-Amino-4(1H)-oxothiochromen-3-carbonitril und 4 g (25 mmol) 4-Chlorbenzoylchlorid werden in 60 ml absol. Pyridin 8 h bei Rückflußtemperatur gerührt. Die Mischung wird einrotiert, der Rückstand mit Wasser digeriert und abgesaugt; Ausbeute: 4 g (47 %) beiger Feststoff mit Schmp. >250°C, Wirkstoffbeispiel 4.5.

Ähnliche Acylierungen führen zu:

**Tabelle A.4**

| Nr. | Verb. I | | R³ | Schmp. [°C] |
|---|---|---|---|---|
| | R¹ | R² | | |
| 4.1 | -NHCO-Me | CN | H | 130 (subst.) |
| 4.2 | -NHCO-CHMe₂ | CN | H | 180 |
| 4.3 | -NHCO-CMe₃ | CN | H | 155-160 |
| 4.4 | -NHCO-(CH₂)₃-Me | CN | H | 115 |
| 4.5 | -NHCO-p-Cl-C₆H₄ | CN | H | >250 |
| 4.6 | -NHCO-p-NO₂-C₆H₄ | CN | H | >250 |
| 4.7 | -NHCO-p-Me-C₆H₄ | CN | H | >238 |

### Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:
Bei Gewächshausversuchen dienten als Kulturgefäße Plastikblumentöpfe mit rund 300 cm³ Inhalt und lehmigem Sand mit etwa 3,0 Gew.-% Humus als Substrat. Die Samen der Testpflanzen wurden nach Arten getrennt, flach eingesät und befeuchtet. Danach wurden die Gefäße mit durchsichtigen Plastikhauben abgedeckt, bis die Samen gleichmäßig gekeimt und die Pflanzen angewachsen waren.

### Liste der Testpflanzen

| Lateinischer Name | Deutscher Name | Englischer Name |
|---|---|---|
| Setaria viridis | Grüne Borstenhirse | green foxtail |
| Triticum aestivum | Sommerweizen | spring wheat |
| Zea mays | Mais | corn |

Für die Nachauflaufbehandlung wurden die Testpflanzen je nach Wuchsform erst bis zu einer Wuchshöhe von 3 bis 20 cm angezüchtet und erst dann behandelt. Die herbiziden Mittel wurden hierbei in Wasser als Verteilungsmittel suspendiert oder emulgiert und mittels fein verteilender Düsen gespritzt.

Als Beispielherbizid der Cyclohexenon-Derivate II diente

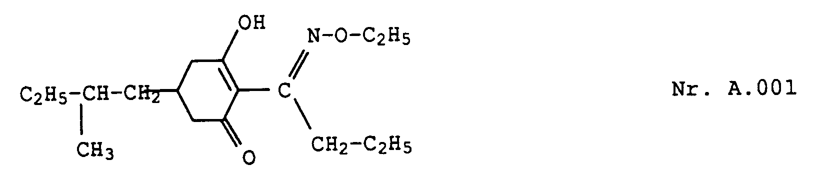

### (Handelsname: Sethoxydim)

Sämtliche antidotisch wirkenden Verbindungen wurden für die Nachauflaufbehandlung in einem Gemisch, bestehend aus 80 Gew.-% Cyclohexanon als Verdünnungsmittel und 20 Gew.-% Tensid (Emulphor EL*)) mit 10 Gew.-% Wirkstoff aufbereitet.
*) ethoxyliertes Rizinusöl (caster oil)

Zum Vergleich wurde der herbizide Wirkstoff als 10 bis 20 gew.-%iges Emulsionskonzentrat formuliert und jeweils unter Zugabe von derjenigen Menge an Lösungsmittelsystem in der Spritzbrühe eingesetzt, mit welcher die antidotisch wirkenden Verbindungen in den angegebenen Aufwandmengen ausgebracht wurden. Die Herstellung der Lösung erfolgte durch Einmischen des Wirkstoffs in eine Lösung aus 93 Gew.-% Xylol und 7 Gew.-% Lutensol AP-8**.
**) nichtionisches oberflächenaktives Mittel auf Basis von Alkylphenolpolyethylenglykolether

Nach Applikation der jeweiligen Wirkstoffmischung wurden die Testpflanzen im Gewächshaus kultiviert, und zwar wärmeliebende Arten bei etwa 18 bis 30°C, solche gemäßigterer Klimate bei ca. 10 bis 25°C.

Die Versuchsperiode erstreckte sich über 3 bis 5 Wochen. Während dieser Zeit wurden die Pflanzen gepflegt, wobei ihre Reaktionen auf die Wirkstoff-Behandlungen erfaßt wurden.

Bewertet wurde die Schädigung durch die chemischen Mittel anhand einer Skala von 0 bis 100 % im Vergleich zu den unbehandelten Kontrollpflanzen. Dabei bedeutet 0 keine Schädigung und 100 eine völlige Zerstörung der Pflanzen.

Die Verbesserung der Verträglichkeit von herbiziden Cyclohexenon-Derivaten II für Kulturpflanzen aus der Familie der Gramineen (Gräser) wie Weizen und Mais durch die Pyrido[2,3-d]pyrimidine I ist der folgenden Tabelle B.1 zu entnehmen:

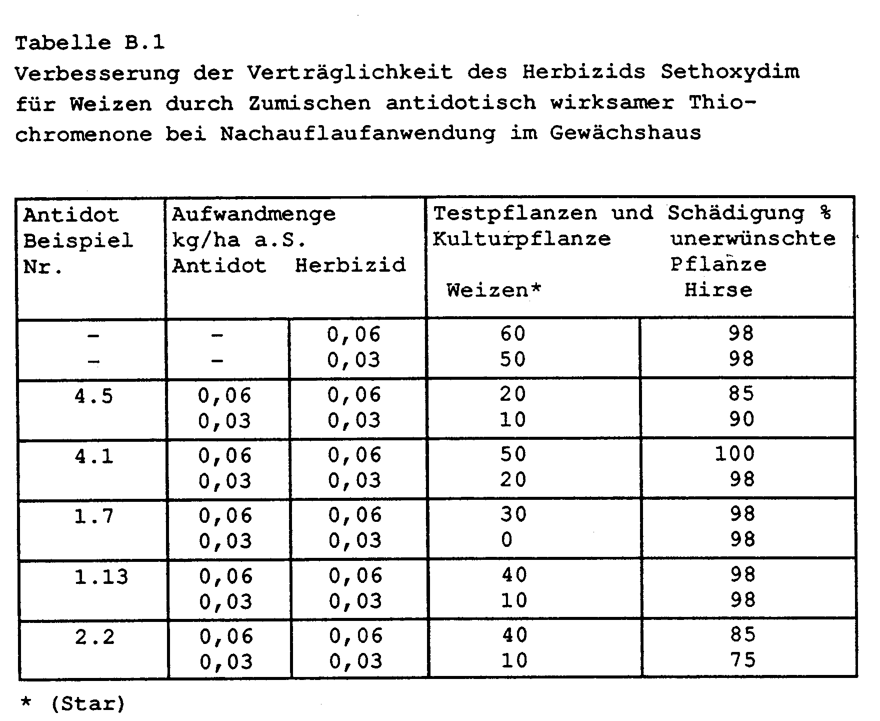

## Patentansprüche

1. Herbizide Mittel, enthaltend mindestens ein Thiochromenon der Formel I 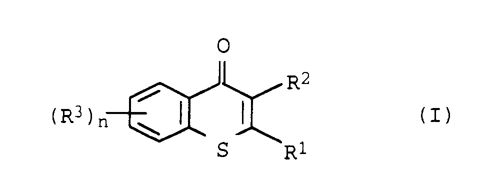 in der die Variablen folgende Bedeutung haben:
n
1, 2, 3 oder 4, wobei die Reste R³ verschiedene Bedeutungen haben können, wenn n > 1 ist;
R¹
Wasserstoff; Cyano; Halogen; C₅-C₁₆-Alkyl;
C₁-C₄-Alkyl, das ein bis fünf Halogenatome oder einen der folgenden Reste tragen kann: Hydroxy, Mercapto, C₁-C₄-Alkoxy, C₁-C₄-Alkylthio, Phenyl, Naphthyl, Thienyl oder Pyridyl, wobei die aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
eine Gruppe -XR⁴ oder eine Gruppe -COYR⁴, wobei
X für Sauerstoff, Schwefel oder -NR⁵- steht,
Y für Sauerstoff oder -NR⁵- steht,
R⁴ eine der folgenden Gruppen bedeutet:
Wasserstoff; Formyl; C₁-C₁₆-Alkyl; C₃-C₇-Cycloalkyl; C₁-C₁₆-Alkylcarbonyl; C₃-C₇-Cycloalkylcarbonyl; C₁-C₁₆-Alkylsulfonyl; C₃-C₇-Cycloalkylsulfonyl; Phenyl, Naphthyl, Thienyl, Pyridyl, Phenylcarbonyl, Naphthylcarbonyl, Thienylcarbonyl, Pyridylcarbonyl, Phenylsulfonyl, Naphthylsulfonyl, Thienylsulfonyl oder Pyridylsulfonyl, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
und
R⁵ für Wasserstoff;
C₁-C₁₆-Alkyl, welches eine Hydroxy- oder C₁-C₄-Alkoxygruppe tragen kann, oder für
Phenyl, Naphthyl, Thienyl oder Pyridyl steht, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
R²
Wasserstoff; Cyano; Nitroso; Nitro; Halogen; C₅-C₁₆-Alkyl;
C₁-C₄-Alkyl, C₁-C₄-Alkoxy oder C₁-C₄-Alkylthio, wobei diese Gruppen ein bis fünf Halogenatome oder einen der folgenden Reste tragen können: Hydroxy, Mercapto, C₁-C₄-Alkoxy, C₁-C₄-Alkylthio, Phenyl, Naphthyl, Thienyl oder Pyridyl, und wobei die aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
eine Gruppe -NR⁴R⁵ oder eine Gruppe -COYR⁴, wobei
Y, R⁴ und R⁵ die vorstehend gegebene Bedeutung haben;
R³
Wasserstoff; Cyano; Halogen; C₅-C₁₆-Alkyl;
C₁-C₄-Alkyl, das ein bis fünf Halogenatome oder einen der folgenden Reste tragen kann: Hydroxy, Mercapto, C₁-C₄-Alkoxy, C₁-C₄-Alkylthio, Phenyl, Naphthyl, Thienyl oder Pyridyl, wobei die aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
eine Gruppe -YR⁴, eine Gruppe -COYR⁴, eine Gruppe -COR⁶ oder eine Gruppe -SO₂R⁷, wobei
Y und R⁴ die vorstehend gegebene Bedeutung haben;
R⁶ eine der folgenden Gruppen bedeutet:
Wasserstoff; C₁-C₁₆-Alkyl; C₃-C₇-Cycloalkyl;
Phenyl, Naphthyl, Thienyl oder Pyridyl, wobei diese aromatischen Reste ihrerseits ein bis fünf Halogenatome und einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio, und
R⁷ für Wasserstoff; C₁-C₁₆-Alkyl; C₃-C₇-Cycloalkyl;
Phenyl, Naphthyl, Thienyl oder Pyridyl steht, wobei diese aromatischen Reste ihrerseits ein bis fünf Halogenatome und/oder einen bis drei der folgenden Substituenten tragen können: C₁-C₄-Alkyl, C₁-C₄-Halogenalkyl, C₁-C₄-Alkoxy, C₁-C₄-Halogenalkoxy, C₁-C₄-Alkylthio und C₁-C₄-Halogenalkylthio;
oder für eine Gruppe -NR⁴R⁵ steht, in der die Reste R⁴ und R⁵ die vorstehend gegebene Bedeutung haben,
und mindestens einen herbiziden Wirkstoff aus
A) der Gruppe der Cyclohexenon-Derivate der allgemeinen Formel II 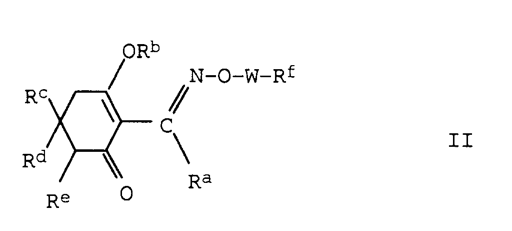 in der die Substituenten folgende Bedeutung haben:
R^{a}
eine C₁-C₆-Alkylgruppe;
R^{b}
Wasserstoff, das Äquivalent eines landwirtschaftlich brauchbaren Kations, eine C₂-C₈-Alkylcarbonylgruppe, eine C₁-C₁₀-Alkylsulfonylgruppe, eine C₁-C₁₀-Alkylphosphonylgruppe oder die Benzoyl-, Benzolsulfonyl- oder Benzolphosphonylgruppe, wobei die drei letztgenannten Gruppen noch je 1 bis 5 Halogenatome tragen können;
R^{c}
Wasserstoff, die Cyanogruppe, die Formylgruppe, eine C₁-C₆-Alkylgruppe, eine C₁-C₄-Alkoxy-C₁-C₆-alkyl- oder C₁-C₄-Alkylthio-C₁-C₆-alkylgruppe, eine Phenoxy-C₁-C₆-alkyl-, Phenylthio-C₁-C₆-alkyl-, Pyridyloxy-C₁-C₆-alkyl- oder Pyridylthio-C₁-C₆-alkylgruppe, wobei die Phenyl- und Pyridylringe jeweils noch einen bis drei Reste tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Alkenyl, C₃-C₆-Alkenyloxy, C₃-C₆-Alkinyl, C₃-C₆-Alkinyloxy und -NR^{g}R^{h}, wobei
R^{g} Wasserstoff, C₁-C₄-Alkyl, C₃-C₆-Alkenyl, C₃-C₆-Alkinyl, C₁-C₆-Acyl oder Benzoyl, das einen bis drei Reste tragen kann, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy und C₁-C₄-Alkylthio und
R^{h} Wasserstoff, C₁-C₄-Alkyl, C₃-C₆-Alkenyl oder C₃-C₆-Alkinyl
bedeuten;
eine C₃-C₇-Cycloalkyl- oder eine C₅-C₇-Cycloalkenylgruppe, wobei diese Gruppen noch einen bis drei Reste tragen können, ausgewählt aus einer Gruppe bestehend aus Hydroxyl, Halogen, C₁-C₄-Alkyl, partiell oder vollständig halogeniertem C₁-C₄-Alkyl, C₁-C₄-Alkoxy, C₁-C₄-Alkylthio, Benzylthio, C₁-C₄-Alkylsulfonyl, C₁-C₄-Alkylsulfenyl und C₁-C₄-Alkylsulfinyl,
ein 5-gliedriger gesättigter Heterocyclus, der ein oder zwei Sauerstoff- oder Schwefelatome oder ein Sauerstoff- und ein Schwefelatom als Heteroatome enthält, und der noch einen bis drei Reste tragen kann, ausgewählt aus einer Gruppe bestehend aus C₁-C₄-Alkyl, partiell oder vollständig halogeniertem C₁-C₄-Alkyl, C₁-C₄-Alkoxy und C₁-C₄-Alkylthio,
ein 6- oder 7-gliedriger gesättigter oder ein- oder zweifach ungesättigter Heterocyclus, der ein oder zwei Sauerstoff- oder Schwefelatome oder ein Sauerstoff und ein Schwefelatom als Heteroatome enthält, und der noch einen bis drei Reste tragen kann, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy, C₁-C₄-Alkylthio,
ein 5-gliedriger Heteroaromat, enthaltend ein bis drei Heteroatome, ausgewählt aus einer Gruppe bestehend aus ein oder zwei Stickstoffatomen und einem Sauerstoff- oder Schwefelatom, wobei der Heteroaromat noch einen bis drei Reste tragen kann, ausgewählt aus einer Gruppe bestehend aus Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₂-C₆-Alkenyl, C₂-C₆-Alkenyloxy, C₃-C₆-Alkinyloxy und C₁-C₄-Alkoxy-C₁-C₄-alkyl,
eine Phenyl- oder Pyridylgruppe, die jeweils noch einen bis drei Reste tragen kann, ausgewählt aus einer Gruppe bestehend aus Nitro, Cyano, Formyl, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Alkenyl, C₃-C₆-Alkenyloxy, C₃-C₆-Alkinyl, C₃-C₆-Alkinyloxy und -NR^{k}R^{l}, wobei R^{k} und R^{l} die oben genannte Bedeutung haben;
R^{d}
Wasserstoff, die Hydroxylgruppe oder, wenn R^{c} für eine C₁-C₆-Alkylgruppe steht, eine C₁-C₆-Alkylgruppe;
R^{e}
Wasserstoff, Halogen, die Cyanogruppe, eine C₁-C₄-Alkoxycarbonylgruppe oder eine C₁-C₄-Alkylketoximgruppe;
W
eine C₁-C₆-Alkylen-, C₃-C₆-Alkenylen- oder C₃-C₆-Alkinylenkette, die jeweils noch ein bis drei Reste tragen können, ausgewählt aus einer Gruppe bestehend aus ein bis drei C₁-C₃-Alkylsubstituenten, ein bis drei Halogenatomen und einem Methylensubstituenten;
eine C₃-C₆-Alkylen- oder C₄-C₆-Alkenylenkette, die beide noch eine bis drei C₁-C₃-Alkylreste tragen können, wobei jeweils eine Methylengruppe der Ketten durch ein Sauerstoff- oder Schwefelatom, eine Sulfoxid-oder Sulfongruppe oder eine Gruppe -N(Rⁱ)- substituiert sein kann, wobei
Rⁱ für Wasserstoff, C₁-C₄-Alkyl, C₃-C₆-Alkenyl oder C₃-C₆-Alkinyl steht;
R^{f} Wasserstoff; die Vinylgruppe;
eine Gruppe -CH=CH-Z, wobei Z für Cyano, Halogen, einen C₁-C₄-Alkylrest, einen partiell oder vollständig halogenierten C₁-C₄-Alkylrest, einen C₃-C₆-Cycloalkylrest, der seinerseits noch einen bis drei Substituenten tragen kann, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy;
den Carboxylrest, einen C₁-C₈-Alkoxycarbonylrest, den Benzyloxycarbonylrest, den Phenyl-, Thienyl- oder Pyridylrest, wobei diese drei aromatischen Reste jeweils noch einen bis drei Substituenten tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy, C₁-C₄-Alkylthio, C₃-C₆-Cycloalkyl wobei der Cycloalkylsubstituent seinerseits noch einen bis drei Reste tragen kann, ausgewählt aus einer Gruppe bestehend aus Halogen, C₁-C₄-Alkyl, partiell oder vollständig halogeniertem C₁-C₄-Alkyl und C₁-C₄-Alkoxy, steht;
die Ethinylgruppe, die einen der folgenden Reste tragen kann: einen C₁-C₄-Alkyl- oder C₃-C₆-Cycloalkylrest, die beide noch einen bis drei Substituenten tragen können, ausgewählt aus einer Gruppe bestehend aus Hydroxyl, Halogen, C₁-C₄-Alkyl, partiell oder vollständig halogeniertem C₁-C₄-Alkyl und C₁-C₄-Alkoxy, oder den Phenyl-, Thienyl- oder Pyridylrest, wobei die aromatischen Reste jeweils noch einen bis drei Substituenten tragen können, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy, partiell oder vollständig halogeniertem C₁-C₄-Alkoxy und C₁-C₄-Alkylthio;
die Phenylgruppe, eine Halogenphenylgruppe, eine Dihalogenphenylgruppe, eine 5-gliedrige heteroaromatische Gruppe mit ein bis drei Heteroatomen, ausgewählt aus einer Gruppe bestehend aus ein bis drei Stickstoffatomen und einem Sauerstoff- oder Schwefelatom, oder eine 6-gliedrige heteroaromatische Gruppe mit ein bis vier Stickstoffatomen, die nicht alle gleichzeitig benachbart sein können, wobei die Phenyl- und Heteroarylgruppen noch einen bis drei Reste tragen können, ausgewählt aus einer Gruppe bestehend aus Nitro, C₁-C₄-Alkoxyresten, C₁-C₄-Alkylthioresten, partiell oder vollständig halogenierten C₁-C₄-Alkoxyresten, Resten Z oder einem Rest -NR^{k}R^{l}, wobei
R^{k} Wasserstoff, C₁-C₄-Alkyl, C₃-C₆-Alkenyl oder C₃-C₆-Alkinyl und
R^{l} Wasserstoff, C₁-C₄-Alkyl, C₃-C₆-Alkenyl, C₃-C₆-Alkinyl, C₁-C₆-Acyl oder Benzoyl, das noch einen bis drei Substituenten tragen kann, ausgewählt aus einer Gruppe bestehend aus Nitro, Cyano, Halogen, C₁-C₄-Alkyl, partiell oder vollständig halogeniertem C₁-C₄-Alkyl, C₁-C₄-Alkoxy und C₁-C₄-Alkylthio, bedeuten,
oder
B) der Gruppe der 2-(4-Heteroaryloxy)- oder 2-(4-Aryloxy)-phenoxycarbonsäurederivate der Formel III 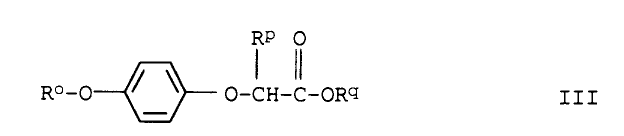 in der die Substituenten folgende Bedeutung haben:
R^{o}
die Phenylgruppe, die Pyridylgruppe, eine Benzoxazylgruppe, eine Benzthiazylgruppe oder eine Benzpyrazinylgruppe, wobei diese aromatischen Ringsysteme bis zu zwei der folgenden Reste tragen können: Halogen, Nitro, C₁-C₄-Alkyl, partiell oder vollständig halogeniertes C₁-C₄-Alkyl und/oder partiell oder vollständig halogeniertes C₁-C₄-Alkoxy;
R^{p}
Wasserstoff oder die Methylgruppe;
R^{q}
Wasserstoff, eine C₁-C₄-Alkylgruppe, eine C₃-C₄-Alkenylgruppe, eine C₃-C₄-Alkinylgruppe, eine C₁-C₄-Alkoxy-C₁-C₄-alkylgruppe, eine C₃-C₄-Alkylideniminooxy-C₂-C₃-alkylgruppe, eine Tetrahydrofuranylmethylgruppe, eine Isoxazolidingruppe oder das Äquivalent eines landwirtschaftlich brauchbaren Kations.

2. Herbizide Mittel nach Anspruch 1, enthaltend mindestens ein Thiochromenon der Formel I, wobei n den Wert 1 oder 2 hat.

3. Herbizide Mittel nach Anspruch 1, enthaltend mindestens ein Thiochromenon der Formel I, wobei R¹ die folgende Bedeutung hat:
Wasserstoff; Halogen;
C₁-C₂-Alkyl, das ein bis fünf Halogenatome oder einen der folgenden Reste tragen kann: C₁-C₂-Alkoxy, C₁-C₂-Alkylthio, Phenyl, Naphthyl, Thienyl oder Pyridyl, wobei die aromatischen Reste ihrerseits ein bis fünf Halogenatome und/oder einen bis drei der folgenden Substituenten tragen können: C₁-C₂-Alkyl, C₁-C₂-Halogenalkyl, C₁-C₂-Alkoxy, C₁-C₂-Halogenalkoxy, C₁-C₂-Alkylthio und C₁-C₂-Halogenalkylthio;
Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₂-Alkyl, C₁-C₂-Halogenalkyl, C₁-C₂-Alkoxy, C₁-C₂-Halogenalkoxy, C₁-C₂-Alkylthio und C₁-C₂-Halogenalkylthio;
eine Gruppe -XR⁴ oder eine Gruppe -COYR⁴, wobei
X und Y für Sauerstoff und -NR⁵- stehen,
R⁴ eine der folgenden Gruppen bedeutet:
Wasserstoff; C₁-C₆-Alkyl; C₃-C₇-Cycloalkyl;
Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₂-Alkyl, C₁-C₂-Halogenalkyl, C₁-C₂-Alkoxy, C₁-C₂-Halogenalkoxy, C₁-C₂-Alkylthio und C₁-C₂-Halogenalkylthio;
und
R⁵ für Wasserstoff oder für
C₁-C₆-Alkyl steht, welches eine Hydroxy- oder C₁-C₄-Alkoxygruppe tragen kann.

4. Herbizide Mittel nach Anspruch 1, enthaltend mindestens ein Thiochromenon der Formel I, wobei R² die folgende Bedeutung hat:
Wasserstoff; Halogen;
C₁-C₂-Alkyl, C₁-C₂-Alkoxy oder C₁-C₂-Alkylthio, wobei diese Gruppen ein bis fünf Halogenatome oder einen der folgenden Reste tragen können: C₁-C₂-Alkoxy, C₁-C₂-Alkylthio, Phenyl, Naphthyl, Thienyl oder Pyridyl, und wobei die aromatischen Reste ihrerseits ein bis fünf Halogenatome und/oder einen bis drei der folgenden Substituenten tragen können: C₁-C₂-Alkyl, C₁-C₂-Halogenalkyl, C₁-C₂-Alkoxy, C₁-C₂-Halogenalkoxy, C₁-C₂-Alkylthio und C₁-C₂-Halogenalkylthio;
Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₂-Alkyl, C₁-C₂-Halogenalkyl, C₁-C₂-Alkoxy, C₁-C₂-Halogenalkoxy, C₁-C₂-Alkylthio und C₁-C₂-Halogenalkylthio;
eine Gruppe -NR⁴R⁵ oder eine Gruppe -COYR⁴, wobei
Y für Sauerstoff oder -NR⁵- steht,
R⁴ eine der folgenden Gruppen bedeutet:
Wasserstoff; C₁-C₆-Alkyl; C₃-C₇-Cycloalkyl;
Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₂-Alkyl, C₁-C₂-Halogenalkyl, C₁-C₂-Alkoxy, C₁-C₂-Halogenalkoxy, C₁-C₂-Alkylthio und C₁-C₂-Halogenalkylthio;
und
R⁵ für Wasserstoff oder für C₁-C₆-Alkyl steht, welches eine Hydroxy- oder C₁-C₄-Alkoxygruppe tragen kann.

5. Herbizide Mittel nach Anspruch 1, enthaltend mindestens ein Thiochromenon der Formel I, wobei R³ die folgende Bedeutung hat:
Wasserstoff; Halogen;
C₁-C₄-Alkyl, das ein bis fünf Halogenatome oder einen der folgenden Reste tragen kann: C₁-C₂-Alkoxy, C₁-C₂-Alkylthio, Phenyl, Naphthyl, Thienyl oder Pyridyl, wobei die aromatischen Reste ihrerseits ein bis fünf Halogenatome und/oder einen bis drei der folgenden Substituenten tragen können: C₁-C₂-Alkyl, C₁-C₂-Halogenalkyl, C₁-C₂-Alkoxy, C₁-C₂-Halogenalkoxy, C₁-C₂-Alkylthio und C₁-C₂-Halogenalkylthio;
eine Gruppe -YR⁴, eine Gruppe -COYR⁴, eine Gruppe -COR⁶ oder eine Gruppe -SO₂R⁷, wobei
Y für Sauerstoff oder -NR⁵- steht;
R⁴ eine der folgenden Gruppen bedeutet:
Wasserstoff; C₁-C₆-Alkyl; C₃-C₇-Cycloalkyl;
Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₂-Alkyl, C₁-C₂-Halogenalkyl, C₁-C₂-Alkoxy, C₁-C₂-Halogenalkoxy, C₁-C₂-Alkylthio und C₁-C₂-Halogenalkylthio;
R⁵ für Wasserstoff oder für C₁-C₆-Alkyl steht, welches eine Hydroxy- oder C₁-C₄-Alkoxygruppe tragen kann;
R⁶ eine der folgenden Gruppen bedeutet:
Wasserstoff; C₁-C₆-Alkyl; C₃-C₇-Cycloalkyl;
Phenyl, Naphthyl, Thienyl oder Pyridyl, wobei diese aromatischen Reste ihrerseits ein bis fünf Halogenatome und/oder einen bis drei der folgenden Substituenten tragen können: C₁-C₂-Alkyl, C₁-C₂-Halogenalkyl, C₁-C₂-Alkoxy, C₁-C₂-Halogenalkoxy, C₁-C₂-Alkylthio und C₁-C₂-Halogenalkylthio;
und
R⁷ für Wasserstoff; C₁-C₆-Alkyl; C₃-C₇-Cycloalkyl;
Phenyl, Naphthyl, Thienyl oder Pyridyl steht, wobei diese aromatischen Reste ihrerseits ein bis fünf Halogenatome und/oder einen bis drei der folgenden Substituenten tragen können: C₁-C₂-Alkyl, C₁-C₂-Halogenalkyl, C₁-C₂-Alkoxy, C₁-C₂-Halogenalkoxy, C₁-C₂-Alkylthio und C₁-C₂-Halogenalkylthio;
oder für eine Gruppe -NR⁴R⁵ steht, in der die Reste R⁴ und R⁵ die vorstehend gegebene Bedeutung haben.

6. Herbizide Mittel nach Anspruch 1, enthaltend mindestens ein Thiochromenon der Formel I, wobei der Index n den Wert 1 oder 2 hat und die Substituenten die folgende Bedeutung haben:
R¹
Wasserstoff; Halogen;
C₁-C₂-Alkyl, das ein bis fünf Halogenatome oder eine C₁-C₂-Alkoxygruppe tragen kann;
Phenyl, welches ein bis fünf Halogenatome und/oder einen bis drei der folgenden Substituenten tragen kann: C₁-C₂-Alkyl, C₁-C₂-Alkoxy und C₁-C₂-Alkylthio;
eine Gruppe -XR⁴ oder eine Gruppe -COYR⁴, wobei
X und Y für Sauerstoff oder -NR⁵- stehen,
R⁴ eine der folgenden Gruppen bedeutet:
Wasserstoff; C₁-C₆-Alkyl; C₃-C₇-Cycloalkyl;
Phenyl, welches ein bis fünf Halogenatome und/oder einen bis drei der folgenden Substituenten tragen kann: C₁-C₂-Alkyl, C₁-C₂-Alkoxy und C₁-C₂-Alkylthio;
und
R⁵ für Wasserstoff oder für C₁-C₆-Alkyl steht;
R²
Wasserstoff; Halogen;
C₁-C₂-Alkyl oder C₁-C₂-Alkoxy, wobei diese Gruppen ein bis fünf Halogenatome oder einen C₁-C₂-Alkoxyrest tragen können;
Phenyl, welches ein bis fünf Halogenatome und/oder einen bis drei der folgenden Substituenten tragen kann: C₁-C₂-Alkyl, C₁-C₂-Alkoxy und C₁-C₂-Alkylthio;
eine Gruppe -NR⁴R⁵ oder eine Gruppe -COYR⁴, wobei
Y, R⁴ und R⁵ die vorstehend gegebene Bedeutung haben,
R³
Wasserstoff; Halogen;
C₁-C₄-Alkyl, das ein bis fünf Halogenatome oder einen C₁-C₂-Alkoxyrest tragen kann;
eine Gruppe -YR⁴, eine Gruppe -COYR⁴, eine Gruppe -COR⁶ oder eine Gruppe -SO₂R⁷, wobei
Y und R⁴ die vorstehend gegebene Bedeutung haben, und
R⁶ und R⁷ unabhängig voneinander für die folgenden Gruppen stehen:
C₁-C₆-Alkyl; C₃-C₇-Cycloalkyl;
Phenyl, welches ein bis fünf Halogenatome und/oder einen bis drei der folgenden Substituenten tragen kann: C₁-C₂-Alkyl, C₁-C₂-Alkoxy und C₁-C₂-Alkylthio.

7. Verfahren zur Bekämpfung unerwünschten Pflanzenwuchses, dadurch gekennzeichnet, daß man mindestens ein substituiertes Thiochromenon der Formel I gemäß Anspruch 1 und mindestens
A) ein Cyclohexenon-Derivat der Formel II gemäß Anspruch 1 oder
B) ein 2-(4-Heteroaryloxy)- oder 2-(4-Aryloxy)-phenoxycarbon- säure-Derivat der Formel III gemäß Anspruch 1
vor, bei oder nach der Aussaat der Kulturpflanzen, vor oder während des Auflaufens der Kulturpflanzen gleichzeitig oder nacheinander ausbringt.

8. Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit mindestens einem substituiertes Thiochromenon der Formel I gemäß Anspruch 1 und mindestens
A) einem Cyclohexenon-Derivat der Formel II gemäß Anspruch 1 oder
B) einem 2-(4-Heteroaryloxy)- oder 2-(4-Aryloxy)-phenoxycarbonsäure-Derivat der Formel III gemäß Anspruch 1
gleichzeitig oder nacheinander behandelt.

9. Verfahren zur Verhinderung der Schädigung von Kulturpflanzen durch
A) herbizide Cyclohexenon-Derivate der Formel II gemäß Anspruch 1 oder
B) herbizide 2-(4-Heteroaryloxy)- oder 2-(4-Aryloxy)-phenoxycarbon-säure-Derivat der Formel III gemäß Anspruch 1,
dadurch gekennzeichnet, daß man das Saatgut der Kulturpflanzen mit einer antagonistisch wirksamen Menge eines Thiochromenons der Formel I gemäß Anspruch 1 behandelt.

10. Verfahren nach den Ansprüchen 7 bis 9, dadurch gekennzeichnet, daß die Kulturpflanzen Gerste, Weizen, Mais, Kultursorghum und Reis sind.

## Claims

1. A herbicide containing at least one thiochromenone of the formula I 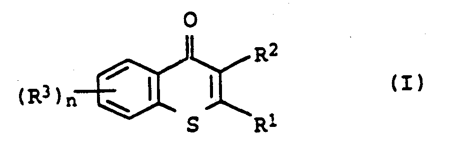 where
n is 1, 2, 3 or 4, and the radicals R³ may have different meanings when n is > 1;
R¹ is hydrogen; cyano; halogen; C₅-C₁₆-alkyl;
C₁-C₄-alkyl which may carry from one to five halogen atoms or one of the following radicals: hydroxyl, mercapto, C₁-C₄-alkoxy, C₁-C₄-alkylthio, phenyl, naphthyl, thienyl or pyridyl, where the aromatic radicals in turn may carry from one to five halogen atoms and/or from one to three of the following substituents: C₁-C₄-alkyl, C₁-C₄-haloalkyl, C₁-C₄-alkoxy, C₁-C₄-haloalkoxy, C₁-C₄-alkylthio and C₁-C₄-haloalkylthio;
phenyl, naphthyl, thieny or pyridyl, where these aromatic radicals in turn may carry from one to five halogen atoms and/or from one to three of the following substituents: C₁-C₄-alkyl, C₁-C₄-haloalkyl, C₁-C₄-alkoxy, C₁-C₄-haloalkoxy, C₁-C₄-alkylthio and C₁-C₄-haloalkylthio;
-XR⁴ or -COYR⁴, in which
X is oxygen, sulfur or -NR⁵-,
Y is oxygen or -NR⁵-,
R⁴ is one of the following groups: hydrogen; formyl; C₁-C₁₆-alkyl; C₃-C₇-cycloalkyl; C₁-C₁₆-alkylcarbonyl; C₃-C₇-cycloalkylcarbonyl; C₁-C₁₆-alkylsulfonyl; C₃-C₇-cycloalkylsulfonyl; phenyl, naphthyl, thienyl, pyridyl, phenylcarbonyl, naphthylcarbonyl, thienylcarbonyl, pyridylcarbonyl, phenylsulfonyl, naphthylsulfonyl, thienylsulfonyl or pyridylsulfonyl, where these aromatic radicals in turn may carry from one to five halogen atoms and/or from one to three of the following substituents: C₁-C₄-alkyl, C₁-C₄-haloalkyl, C₁-C₄-alkoxy, C₁-C₄-haloalkoxy, C₁-C₄-alkylthio and C₁-C₄-haloalkylthio;
and
R⁵ is hydrogen or
C₁-C₁₆-alkyl which may carry a hydroxyl or C₁-C₄-alkoxy group, or is
phenyl, naphthyl, thienyl or pyridyl, where these aromatic radicals in turn may carry from one to five halogen atoms and/or from one to three of the following substituents: C₁-C₄-alkyl, C₁-C₄-haloalkyl, C₁-C₄-alkoxy, C₁-C₄-haloalkoxy, C₁-C₄-alkylthio and C₁-C₄-haloalkylthio; R² is hydrogen; cyano; nitroso; nitro; halogen; C₅-C₁₆-alkyl;
C₁-C₄-alkyl, C₁-C₄-alkoxy or C₁-C₄-alkylthio, where these groups may carry from one to five halogen atoms or one of the following radicals: hydroxyl, mercapto, C₁-C₄-alkoxy, C₁-C₄-alkylthio, phenyl, naphthyl, thienyl or pyridyl, where the aromatic radicals in turn may carry from one to five halogen atoms and/or from one to three of the following substituents: C₁-C₄-alkyl, C₁-C₄-haloalkyl, C₁-C₄-alkoxy, C₁-C₄-haloalkoxy, C₁-C₄-alkylthio and C₁-C₄-haloalkylthio;
phenyl, naphthyl, thienyl or pyridyl, where these aromatic radicals in turn may carry from one to five halogen atoms and/or from one to three of the following substituents: C₁-C₄-alkyl, C₁-C₄-haloalkyl, C₁-C₄-alkoxy, C₁-C₄-haloalkoxy, C₁-C₄-alkylthio and C₁-C₄-haloalkylthio; -NR⁴R⁵ or -COYR⁴, in which
Y, R⁴ and R⁵ have the abovementioned meanings;
R³ is hydrogen; cyano; halogen; C₅-C₁₆-alkyl;
C₁-C₄-alkyl which may carry from one to five halogen atoms or one of the following radicals: hydroxyl, mercapto, C₁-C₄-alkoxy, C₁-C₄-alkylthio, phenyl, naphthyl, thienyl or pyridyl, where the aromatic radicals in turn may carry from one to five halogen atoms and/or from one to three of the following substiutents: C₁-C₄-alkyl, C₁-C₄-haloalkyl, C₁-C₄-alkoxy, C₁-C₄-haloalkoxy, C₁-C₄-alkylthio and C₁-C₄-haloalkylthio;
phenyl, naphthyl, thienyl or pyridyl, where these aromatic radicals in turn may carry from one to five halogen atoms and/or from one to three of the following substituents: C₁-C₄-alkyl, C₁-C₄-haloalkyl, C₁-C₄-alkoxy, C₁-C₄-haloalkoxy, C₁-C₄-alkylthio and C₁-C₄-haloalkylthio; -YR⁴, -COYR⁴, -COR⁶ or -SO₂R⁷, in which
Y and R⁴ have the abovementioned meanings;
R⁶ is one of the following groups:
hydrogen; C₁-C₁₆-alkyl; C₃-C₇-cycloalkyl;
phenyl, naphthyl, thienyl or pyridyl, where these aromatic radicals in turn may carry from one to five halogen atoms and/or from one to three of the following substituents: C₁-C₄-alkyl, C₁-C₄-haloalkyl, C₁-C₄-alkoxy, C₁-C₄-haloalkoxy, C₁-C₄-alkylthio and C₁-C₄-haloalkylthio; and
R⁷ is hydrogen; C₁-C₁₆-alkyl; C₃-C₇-cycloalkyl;
phenyl, naphthyl, thienyl or pyridyl, where these aromatic radicals in turn may carry from one to five halogen atoms and/or from one to three of the following substituents: C₁-C₄-alkyl, C₁-C₄-haloalkyl, C₁-C₄-alkoxy, C₁-C₄-haloalkoxy, C₁-C₄-alkylthio and C₁-C₄-haloalkylthio; or -NR⁴R⁵, where R⁴ and R⁵ have the abovementioned meanings,
and at least one herbicidal active ingredient selected from
A) the group consisting of the cyclohexenone derivatives of the formula II 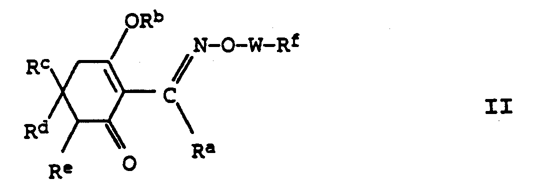 where
R^{a} is C₁-C₆-alkyl;
R^{b} is hydrogen, one equivalent of an agriculturally suitable cation, C₂-C₈-alkylcarbonyl, C₁-C₁₀-alkylsulfonyl,
C₁-C₁₀-alkylphosphonyl or benzoyl, benzenesulfonyl or benzenephosphonyl, where the three last-mentioned groups may furthermore each carry from 1 to 5 halogen atoms;
R^{c} is hydrogen, cyano, formyl, C₁-C₆-alkyl, C₁-C₄-alkoxy-C₁-C₆-alkyl, C₁-C₄-alkylthio-C₁-C₆-alkyl, phenoxy-C₁-C₆-alkyl, phenylthio-C₁-C₆-alkyl, pyridyloxy-C₁-C₆-alkyl or pyridylthio-C₁-C₆-alkyl, where the phenyl and pyridyl rings may each furthermore carry from one to three radicals selected from the group consisting of nitro, cyano, halogen, C₁-C₄-alkyl, partially or completely halogenated C₁-C₄-alkyl, C₁-C₄-alkoxy, partially or completely halogenated C₁-C₄-alkoxy, C₁-C₄-alkylthio, C₃-C₆-alkenyl, C₃-C₆-alkenyloxy, C₃-C₆-alkynyl, C₃-C₆-alkynyloxy and -NR^{g}R^{h}, in which
R^{g} is hydrogen, C₁-C₄-alkyl, C₃-C₆-alkenyl, C₃-C₆-alkynyl,
C₁-C₆-acyl or benzoyl which may carry from one to three radicals selected from the group consisting of nitro, cyano, halogen, C₁-C₄-alkyl, partially or completely halogenated C₁-C₄-alkyl, C₁-C₄-alkoxy and C₁-C₄-alkylthio, and
R^{h} is hydrogen, C₁-C₄-alkyl, C₃-C₆-alkenyl or C₃-C₆-alkynyl;
C₃-C₇-cycloalkyl or C₅-C₇-cycloalkenyl, where these groups may furthermore carry from one to three radicals selected from the group consisting of hydroxyl, halogen, C₁-C₄-alkyl, partially or completely halogenated C₁-C₄-alkyl, C₁-C₄-alkoxy, C₁-C₄-alkylthio, benzylthio, C₁-C₄-alkylsulfonyl, C₁-C₄-alkylsulfenyl and C₁-C₄-alkylsulfinyl,
a 5-membered saturated heterocyclic structure which contains one or two oxygen or sulfur atoms or one oxygen and one sulfur atom as hetero atoms and which may furthermore carry from one to three radicals selected from the group consisting of C₁-C₄-alkyl, partially or completely halogenated C₁-C₄-alkyl, C₁-C₄-alkoxy and C₁-C₄-alkylthio,
a 6-membered or 7-membered saturated or mono- or diunsaturated heterocyclic structure which contains one or two oxygen or sulfur atoms or one oxygen and one sulfur atom as hetero atoms and which may furthermore carry from one to three radicals selected from the group consisting of hydroxyl, halogen, C₁-C₄-alkyl, partially or completely halogenated C₁-C₄-alkyl, C₁-C₄-alkoxy and C₁-C₄-alkylthio, a 5-membered heteroaromatic structure containing from one to three hetero atoms selected from the group consisting of one or two nitrogen atoms and one oxygen or sulfur atom, where the heteroaromatic structure may furthermore carry from one to three radicals selected from the group consisting of cyano, halogen, C₁-C₄-alkyl, partially or completely halogenated C₁-C₄-alkyl, C₁-C₄-alkoxy, partially or completely halogenated C₁-C₄-alkoxy, C₁-C₄-alkylthio, C₂-C₆-alkenyl, C₂-C₆-alkenyloxy, C₃-C₆-alkynyloxy and C₁-C₄-alkoxy-C₁-C₄-alkyl,
phenyl or pyridyl, each of which may furthermore carry from one to three radicals selected from the group consisting of nitro, cyano, formyl, halogen, C₁-C₄-alkyl, partially or completely halogenated C₁-C₄-alkyl, C₁-C₄-alkoxy, partially or completely halogenated C₁-C₄-alkoxy, C₁-C₄-alkylthio, C₃-C₆-alkenyl, C₃-C₆-alkenyloxy, C₃-C₆-alkynyl, C₃-C₆-alkynyloxy and -NR^{k}R^{l}, where R^{k} and R^{l} have the abovementioned meanings;
R^{d} is hydrogen or hydroxyl or, when R^{c} is C₁-C₆-alkyl, R^{d} is C₁-C₆-alkyl;
R^{e} is hydrogen, halogen, cyano, C₁-C₄-alkoxycarbonyl or a C₁-C₄-alkylketoxime group;
W is a C₁-C₆-alkylene, C₃-C₆-alkenylene or C₃-C₆-alkynylene chain, each of which may furthermore carry from one to three radicals selected from the group consisting of from one to three C₁-C₃-alkyl substituents, from one to three halogen atoms and one methylene substituent;
a C₃-C₆-alkylene or C₄-C₆-alkenylene chain, both of which may furthermore carry from one to three C₁-C₃-alkyl radicals and in each case a methylene group of the chains may be substituted by oxygen, sulfur, sulfoxyl, sulfonyl or a group -N(Rⁱ)-, in which
Rⁱ is hydrogen, C₁-C₄-alkyl, C₃-C₆-alkenyl or C₃-C₆-alkynyl;
R^{f} is hydrogen; vinyl;
a group -CH=CH-Z, in which Z is cyano, halogen, C₁-C₄-alkyl, partially or completely halogenated C₁-C₄-alkyl, C₃-C₆-cycloalkyl which in turn may furthermore carry from one to three substituents selected from the group consisting of hydroxyl, halogen, C₁-C₄-alkyl, partially or completely halogenated C₁-C₄-alkyl and C₁-C₄-alkoxy;
carboxyl, C₁-C₈-alkoxycarbonyl, benzyloxycarbonyl, phenyl, thienyl or pyridyl, where these three aromatic radicals may each furthermore carry from one to three substituents selected from the group consisting of nitro, cyano, halogen, C₁-C₄-alkyl, partially or completely halogenated C₁-C₄-alkyl, C₁-C₄-alkoxy, partially or completely halogenated C₁-C₄-alkoxy, C₁-C₄-alkylthio and C₃-C₆-cycloalkyl, where the cycloalkyl substituent in turn may furthermore carry from one to three radicals selected from the group consisting of halogen, C₁-C₄-alkyl, partially or completely halogenated C₁-C₄-alkyl and C₁-C₄-alkoxy;
ethynyl which may carry one of the following radicals: C₁-C₄-alkyl or C₃-C₆-cycloalkyl, both of which nay furthermore carry from one to three substituents selected from the group consisting of hydroxyl, halogen, C₁-C₄-alkyl, partially or completely halogenated C₁-C₄-alkyl and C₁-C₄-alkoxy, or phenyl, thienyl or pyridyl, where each of the aromatic radicals may furthermore carry from one to three substituents selected from the group consisting of nitro, cyano, halogen, C₁-C₄-alkyl, partially or completely halogenated C₁-C₄-alkyl, C₁-C₄-alkoxy, partially or completely halogenated C₁-C₄-alkoxy and C₁-C₄-alkylthio;
phenyl, halophenyl, dihalophenyl, a 5-membered heteroaromatic group having from one to three hetero atoms selected from the group consisting of from one to three nitrogen atoms and one oxygen or sulfur atom, or a 6-membered heteroaromatic group having from one to four nitrogen atoms, all of which may not be simultaneously adjacent to one another, where the phenyl and hetaryl groups may furthermore carry from one to three radicals selected from the group consisting of nitro, C₁-C₄-alkoxy, C₁-C₄-alkylthio, partially or completely halogenated C₁-C₄-alkoxy, radicals Z or a radical -NR^{k}R^{l}, in which
R^{k} is hydrogen, C₁-C₄-alkyl, C₃-C₆-alkenyl or C₃-C₆-alkynyl and
R^{l} is hydrogen, C₁-C₄-alkyl, C₃-C₆-alkenyl, C₃-C₆-alkynyl, C₁-C₆-acyl or benzoyl which may furthermore carry from one to three substituents selected from the group consisting of nitro, cyano, halogen, C₁-C₄-alkyl, partially or completely halogenated C₁-C₄-alkyl, C₁-C₄-alkoxy and C₁-C₄-alkylthio,
or
B) the group consisting of the 2-(4-hetaryloxy)- or 2-(4-aryloxy)-phenoxycarboxylic acid derivatives of the formula III 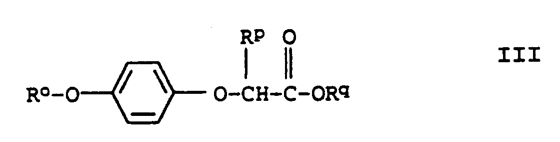 where
R^{o} is phenyl, pyridyl, benzoxazyl, benzothiazyl or benzopyrazinyl, where these aromatic ring systems may carry up to two of the following radicals: halogen, nitro, C₁-C₄-alkyl, partially or completely halogenated C₁-C₄-alkyl and/or partially or completely halogenated C₁-C₄-alkoxy;
R^{p} is hydrogen or methyl,
and
R^{q} is hydrogen, C₁-C₄-alkyl, C₃- or C₄-alkenyl, C₃- or C₄-alkynyl, C₁-C₄-alkoxy-C₁-C₄-alkyl, C₃- or C₄-alkylideneiminoxy-C₂- or - C₃-alkyl, tetrahydrofuranylmethyl, isoxazolidinyl or one equivalent of an agriculturally suitable cation.

2. A herbicide as claimed in claim 1, containing at least one thiochromenone of the formula I where n is 1 or 2.

3. A herbicide as claimed in claim 1, containing at least one thiochromenone of the formula I where R¹ is hydrogen; halogen;
C₁- or C₂-alkyl which may carry from one to five halogen atoms or one of the following radicals: C₁- or C₂-alkoxy, C₁- or C₂-alkylthio, phenyl, naphthyl, thienyl or pyridyl, where the aromatic radicals in turn may carry from one to five halogen atoms and/or from one to three of the following substituents: C₁- or C₂-alkyl, C₁- or C₂-haloalkyl, C₁- or C₂-alkoxy, C₁- or C₂-haloalkoxy, C₁- or C₂-alkylthio and C₁- or C₂-haloalkylthio;
phenyl, naphthyl, thienyl or pyridyl, where these aromatic radicals in turn may carry from one to five halogen atoms and/or from one to three of the following substituents: C₁- or C₂-alkyl, C₁- or C₂-haloalkyl, C₁- or C₂-alkoxy, C₁- or C₂-haloalkoxy, C₁- or C₂-alkylthio and C₁- or C₂-haloalkylthio;
-XR⁴ or -COYR⁴, in which
X and Y are each oxygen or -NR⁵-,
R⁴ is one of the following groups:
hydrogen; C₁-C₆-alkyl; C₃-C₇-cycloalkyl;
phenyl, naphthyl, thienyl or pyridyl, where these aromatic radicals in turn may carry from one to five halogen atoms and/or from one to three of the following substituents: C₁- or C₂-alkyl, C₁- or C₂-haloalkyl, C₁- or C₂-alkoxy, C₁- or C₂-haloalkoxy, C₁- or C₂-alkylthio and C₁- or C₂-haloalkylthio;
and
R⁵ is hydrogen or
C₁-C₆-alkyl which may carry a hydroxyl or a C₁-C₄-alkoxy group.

4. A herbicide as claimed in claim 1, containing at least one thiochromenone of the formula I where R² has the following meanings:
hydrogen; halogen;
C₁- or C₂-alkyl, C₁- or C₂-alkoxy or C₁- or C₂-alkylthio, where these groups may carry from one to five halogen atoms or one of the following radicals: C₁- or C₂-alkoxy, C₁- or C₂-alkylthio, phenyl, naphthyl, thienyl or pyridyl, where the aromatic radicals in turn may carry from one to five halogen atoms and/or from one to three of the following substituents: C₁- or C₂-alkyl, C₁- or C₂-haloalkyl, C₁- or C₂-alkoxy, C₁- or C₂-haloalkoxy, C₁- or C₂-alkylthio and C₁- or C₂-haloalkylthio;
phenyl, naphthyl, thienyl or pyridyl, where these aromatic radicals in turn may carry from one to five halogen atoms and/or from one to three of the following substituents: C₁- or C₂-alkyl, C₁- or C₂-haloalkyl, C₁- or C₂-alkoxy, C₁- or C₂-haloalkoxy, C₁- or C₂-alkylthio and C₁- or C₂-haloalkylthio;
-NR⁴R⁵ or -COYR⁴, in which
Y is oxygen or -NR⁵-,
R⁴ is one of the following groups:
hydrogen; C₁-C₆-alkyl; C₃-C₇-cycloalkyl;
phenyl, naphthyl, thienyl or pyridyl, where these aromatic radicals in turn may carry from one to five halogen atoms and/or from one to three of the following substituents: C₁- or C₂-alkyl, C₁- or C₂-haloalkyl, C₁- or C₂-alkoxy, C₁- or C₂-haloalkoxy, C₁- or C₂-alkylthio and C₁- or C₂-haloalkylthio;
and
R⁵ is hydrogen or C₁-C₆-alkyl which may carry a hydroxyl or a C₁-C₄-alkoxy group.

5. A herbicide as claimed in claim 1, containing at least one thiochromenone of the formula I where R³ has the following meanings:
hydrogen; halogen;
C₁-C₄-alkyl which may carry from one to five halogen atoms or one of the following radicals: C₁- or C₂-alkoxy, C₁- or C₂-alkylthio, phenyl, naphthyl, thienyl or pyridyl, where the aromatic radicals in turn nay carry from one to five halogen atoms and/or from one to three of the following substituents: C₁- or C₂-alkyl, C₁- or C₂-haloalkyl, C₁- or C₂-alkoxy, C₁- or C₂-haloalkoxy, C₁- or C₂-alkylthio and C₁- or C₂-haloalkylthio;
-YR⁴, -COYR⁴, -COR⁶ or -SO₂R⁷, in which
Y is oxygen or -NR⁵-,
R⁴ is one of the following groups:
hydrogen; C₁-C₆-alkyl; C₃-C₇-cycloalkyl;
phenyl, naphthyl, thienyl or pyridyl, where these aromatic radicals in turn may carry from one to five halogen atoms and/or from one to three of the following substituents: C₁- or C₂-alkyl, C₁- or C₂-haloalkyl, C₁- or C₂-alkoxy, C₁- or C₂-haloalkoxy, C₁- or C₂-alkylthio and C₁- or C₂-haloalkylthio;
R⁵ is hydrogen or C₁-C₆-alkyl which may carry a hydroxyl or a C₁-C₄-alkoxy group;
R⁶ is one of the following groups:
hydrogen; C₁-C₆-alkyl; C₃-C₇-cycloalkyl;
phenyl, naphthyl, thienyl or pyridyl, where these aromatic radicals in turn may carry from one to five halogen atoms and/or from one to three of the following substituents: C₁- or C₂-alkyl, C₁- or C₂-haloalkyl, C₁- or C₂-alkoxy, C₁- or C₂-haloalkoxy, C₁- or C₂-alkylthio and C₁- or C₂-haloalkylthio;
and
R⁷ is hydrogen; C₁-C₆-alkyl; C₃-C₇-cycloalkyl;
phenyl, naphthyl, thienyl or pyridyl, where these aromatic radicals in turn may carry from one to five halogen atoms and/or from one to three of the following substituents: C₁- or C₂-alkyl, C₁- or C₂-haloalkyl, C₁- or C₂-alkoxy, C₁- or C₂-haloalkoxy, C₁- or C₂-alkylthio and C₁- or C₂-haloalkylthio;
or -NR⁴R⁵, where R⁴ and R⁵ have the abovementioned meanings.

6. A herbicide as claimed in claim 1, containing at least one thiochromenone of the formula I where
n is 1 or 2;
R¹ is hydrogen; halogen;
C₁- or C₂-alkyl which may carry from one to five halogen atoms or a C₁- or C₂-alkoxy group; phenyl which may carry from one to five halogen atoms and/or from one to three of the following substituents: C₁- or C₂-alkyl, C₁- or C₂-alkoxy and C₁- or C₂-alkylthio;
-XR⁴ and -COYR⁴, in which
X and Y are each oxygen or -NR⁵-,
R⁴ is one of the following groups:
hydrogen; C₁-C₆-alkyl; C₃-C₇-cycloalkyl;
phenyl which may carry from one to five halogen atoms and/or from one to three of the following substituents: C₁- or C₂-alkyl, C₁- or C₂-alkoxy and C₁- or C₂-alkylthio;
and
R⁵ is hydrogen or C₁-C₆-alkyl;
R² is hydrogen; halogen;
C₁- or C₂-alkyl or C₁- or C₂-alkoxy where these groups may carry from one to five halogen atoms or a C₁- or C₂-alkoxy radical;
phenyl which may carry from one to five halogen atoms and/or from one to three of the following substituents:
C₁- or C₂-alkyl, C₁- or C₂-alkoxy and C₁- or C₂-alkylthio;
-NR⁴R⁵ or -COYR⁴, in which
Y, R⁴ and R⁵ have the abovementioned meanings,
R³ is hydrogen; halogen;
C₁-C₄-alkyl which may carry from one to five halogen atoms or a C₁- or C₂-alkoxy radical;
-YR⁴, -COYR⁴, -COR⁶ or -SO₂R⁷, in which
Y and R⁴ have the abovementioned meanings and
R⁶ and R⁷ independently of one another are each C₁-C₆-alkyl; C₃-C₇-cycloalkyl;
phenyl which may carry from one to five halogen atoms and/or from one to three of the following substituents: C₁- or C₂-alkyl, C₁- or C₂-alkoxy and C₁- or C₂-alkylthio.

7. A method for controlling undesirable plant growth, wherein at least one substituted thiochromenone of the formula I as claimed in claim 1 and at least
A) one cyclohexenone derivative of the formula II as claimed in claim 1 or
B) one 2-(4-hetaryloxy)- or 2-(4-aryloxy)-phenoxycarboxylic acid derivative of the formula III as claimed in claim 1
are applied simultaneously or in succession, before, during or after sowing of the crops or before or during emergence of the crops.

8. A method for selectively controlling undesirable plant growth, wherein the leaves of the crops and the undesirable plants are treated simultaneously or in succession, by the postemergence method, with at least one substituted thiochromenone of the formula I as claimed in claim 1 and at least
A) one cyclohexenone derivative of the formula II as claimed in claim 1 or
B) one 2-(4-hetaryloxy)- or 2-(4-aryloxy)-phenoxycarboxylic acid derivative of the formula III as claimed in claim 1.

9. A method for preventing damage to crops by
A) herbicidal cyclohexenone derivatives of the formula II as claimed in claim 1 or
B) herbicidal 2-(4-hetaryloxy)- or 2-(4-aryloxy)-phenoxycarboxylic acid derivatives of the formula III as claimed in claim 1,
wherein the seed of the crops is treated with a thiochromenone of the formula I as claimed in claim 1 in an amount which has an antagonistic action.

10. A method as claimed in any of claims 7 to 9, wherein the crops are barley, wheat, corn, millet and rice.

## Revendications

1. Agent herbicide, contenant au moins une thiochroménone de formule I 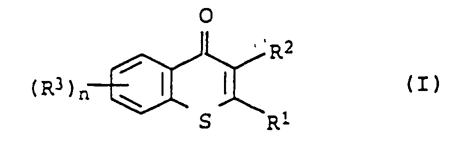 où les variables ont la signification suivante:
n
est 1, 2, 3 ou 4, tandis que les restes R³ peuvent avoir diverses significations, quand n est > 1;
R¹
représente un groupe hydrogène; cyano; halogène; alkyle en C₅-C₁₆;
alkyle en C₁-C₄, qui peut porter un à cinq atomes d'halogène ou l'un des restes suivants: hydroxy, mercapto, alcoxy en C₁-C₄, alkylthio en C₁-C₄, phényle, naphtyle, thiényle ou pyridyle, tandis que les restes aromatiques peuvent à leur tour porter un à cinq atomes d'halogène et/ou un à trois des substituants suivants: alkyle en C₁-C₄, halogénoalkyle en C₁-C₄, alcoxy en C₁-C₄, halogénoalcoxy en C₁-C₄, alkylthio en C₁-C₄, et halogénoalkylthio en C₁-C₄;
phényle, naphtyle, thiényle ou pyridyle, tandis que ces restes aromatiques peuvent à leur tour porter un à cinq atomes d'halogène et/ou un à trois des substituants suivants: alkyle en C₁-C₄, halogénoalkyle en C₁-C₄, alcoxy en C₁-C₄, halogénoalcoxy en C₁-C₄, alkylthio en C₁-C₄ et halogénoalkylthio en C₁-C₄;
un groupe -XR⁴ ou un groupe -COYR⁴, tandis que
X représente l'oxygène, le soufre ou -NR⁵-,
Y désigne l'oxygène ou -NR⁵-,
R⁴ représente l'un des groupes suivants:
hydrogène; formyle; alkyle en C₁-C₁₆; cycloalkyle en C₃-C₇; alkylcarbonyle en C₁-C₁₆; cycloalkylcarbonyle en C₃-C₇; alkylsulfonyle en C₁-C₁₆; cycloalkylsulfonyle en C₃-C₇; phényle, naphtyle, thiényle, pyridyle, phénylcarbonyle, naphtylcarbonyle, thiénylcarbonyle, pyridylcarbonyle, phénylsulfonyle, naphtylsulfonyle, thiénylsulfonyle ou pyridylsulfonyle, tandis que ces restes peuvent à leur tour porter un à cinq atomes d'halogène et/ou un à trois des substituants suivants: alkyle en C₁-C₄, halogénoalkyle en C₁-C₄, alcoxy en C₁-C₄, halogénoalcoxy en C₁-C₄, alkylthio en C₁-C₄ et halogénoalkylthio en C₁-C₄;
et
R⁵ désigne l'hydrogène ou un groupe
alkyle en C₁-C₁₆, qui peut porter un groupe hydroxy ou alcoxy en C₁-C₄, ou
phényle, naphtyle, thiényle ou pyridyle, tandis que ces restes aromatiques peuvent à leur tour porter un à cinq atomes d'halogène et/ou un à trois des substituants suivants: alkyle en C₁-C₄, halogénoalkyle en C₁-C₄, alcoxy en C₁-C₄, halogénoalcoxy en C₁-C₄, alkylthio en C₁-C₄ et halogénoalkylthio en C₁-C₄;
R²
représente un atome ou un groupe hydrogène; cyano; nitroso; nitro; halogène; alkyle en C₅-C₁₆;
alkyle en C₁-C₄, alcoxy en C₁-C₄ ou alkylthio en C₁-C₄, tandis que ces groupes peuvent porter un à cinq atomes d'halogène ou l'un des restes suivants: hydroxy, mercapto, alcoxy en C₁-C₄, alkylthio en C₁-C₄, phényle, naphtyle, thiényle ou pyridyle, et tandis que les restes aromatiques peuvent à leur tour porter un à cinq atomes d'halogène et/ou un à trois des substituants suivants: alkyle en C₁-C₄, halogénoalkyle en C₁-C₄, alcoxy en C₁-C₄, halogénoalcoxy en C₁-C₄, alkylthio en C₁-C₄ et halogénoalkylthio en C₁-C₄;
phényle, naphtyle, thiényle ou pyridyle, tandis que ces restes aromatiques peuvent à leur tour porter un à cinq atomes d'halogène et/ou un à trois des substituants suivants: alkyle en C₁-C₄, halogénoalkyle en C₁-C₄, alcoxy en C₁-C₄, halogénoalcoxy en C₁-C₄, alkylthio en C₁-C₄ et halogénoalkylthio en C₁-C₄;
un groupe -NR⁴R⁵ ou un groupe -COYR⁴, tandis que
Y, R⁴ et R⁵ ont la signification indiquée plus haut;
R³
représente un groupe hydrogène; cyano; halogène; alkyle en C₅-C₁₆;
alkyle en C₁-C₄, qui peut porter un à cinq atomes d'halogène ou l'un des restes suivants: hydroxy, mercapto, alcoxy en C₁-C₄, alkylthio en C₁-C₄, phényle, naphtyle, thiényle ou pyridyle, tandis que les restes aromatiques peuvent à leur tour porter un à cinq atomes d'halogène et/ou un à trois des substituants suivants: alkyle en C₁-C₄, halogénoalkyle en C₁-C₄, alcoxy en C₁-C₄, halogénoalcoxy en C₁-C₄, alkylthio en C₁-C₄ et halogénoalkylthio en C₁-C₄;
phényle, naphtyle, thiényle ou pyridyle, tandis que ces restes aromatiques peuvent porter à leur tour un à cinq atomes d'halogène et/ou un à trois des substituants suivants: alkyle en C₁-C₄, halogénoalkyle en C₁-C₄, alcoxy en C₁-C₄, halogénoalcoxy en C₁-C₄, alkylthio en C₁-C₄ et halogénoalkylthio en C₁-C₄;
un groupe -YR⁴, un groupe -COYR⁴, un groupe -COR⁶ ou un groupe -SO₂R⁷, tandis que
Y et R⁴ ont la signification indiquée plus haut;
R⁶ représente l'un des groupes suivants:
hydrogène; alkyle en C₁-C₁₆; cycloalkyle en C₃-C₇;
phényle, naphtyle, thiényle ou pyridyle, tandis que ces restes aromatiques peuvent à leur tour porter un à cinq atomes d'halogène et/ou un à trois des substituants suivants: alkyle en C₁-C₄, halogénoalkyle en C₁-C₄, alcoxy en C₁-C₄, halogénoalcoxy en C₁-C₄, alkylthio en C₁-C₄ et halogénoalkylthio en C₁-C₄,
et
R⁷ représente un groupe hydrogène; alkyle en C₁-C₁₆; cycloalkyle en C₃-C₇;
phényle, naphtyle, thiényle ou pyridyle, tandis que ces restes aromatiques peuvent à leur tour porter un à cinq atomes d'halogène et/ou un à trois des substituants suivants: alkyle en C₁-C₄, halogénoalkyle en C₁-C₄, alcoxy en C₁-C₄, halogénoalcoxy en C₁-C₄, alkylthio en C₁-C₄ et halogénoalkylthio en C₁-C₄;
ou un groupe -NR⁴R⁵, dans lequel les restes R⁴ et R⁵ peuvent avoir la signification indiquée plus haut,
et au moins un agent à activité herbicide choisi
A) dans le groupe des dérivés de cyclohexénone de formule générale II 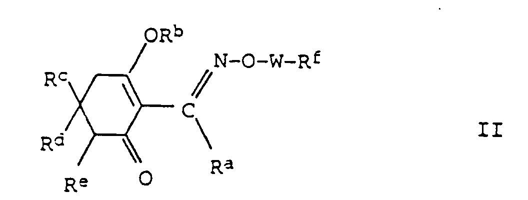 où les substituants ont la signification suivante:
R^{a}
un groupe alkyle en C₁-C₆;
R^{b}
l'hydrogène, l'équivalent d'un cation utilisable en agriculture, un groupe alkyl(C₂-C₈)carbonyle, un groupe alkyl(C₁-C₁₀)sulfonyle, un groupe alkyl (C₁-C₁₀)phosphonyle ou le groupe benzoyle, benzènesulfonyle ou benzènephosphonyle, tandis que les trois groupes mentionnés en dernier peuvent encore porter chacun 1 à 5 atomes d'halogène;
R^{c}
l'hydrogène, le groupe cyano, le groupe formyle, un groupe alkyle en C₁-C₆, un groupe alcoxy(C₁-C₄)alkyle(C₁-C₆) ou un groupe alkylthio(C₁-C₄)alkyle(C₁-C₆), un groupe phénoxy-alkyle(C₁-C₆), phénylthio-alkyle(C₁-C₆), pyridyloxy-alkyle(C₁-C₆) ou pyridylthio-alkyle(C₁-C₆), tandis que les cycles phényle et pyridyle peuvent encore porter chacun un à trois restes choisis parmi les groupes nitro, cyano, halogène, alkyle en C₁-C₄, alkyle en C₁-C₄ partiellement ou totalement halogéné, alcoxy en C₁-C₄, alcoxy en C₁-C₄ partiellement ou totalement halogéné, alkylthio en C₁-C₄, alcényle en C₃-C₆, alcényloxy en C₃-C₆, alcynyle en C₃-C₆, alcynyloxy en C₃-C₆ et -NR^{g}R^{h}, tandis que
R^{g} représente un atome ou un groupe hydrogène, alkyle en C₁-C₄, alcényle en C₃-C₆, alcynyle en C₃-C₆, acyle en C₁-C₆ ou benzoyle, qui peut porter un à trois restes choisis parmi les groupes nitro, cyano, halogène, alkyle en C₁-C₄, alkyle en C₁-C₄ partiellement ou totalement halogéné, alcoxy en C₁-C₄ et alkylthio en C₁-C₄ et
R^{h} représente un atome ou un groupe hydrogène, alkyle en C₁-C₄, alcényle en C₃-C₆ ou alcynyle en C₃-C₆;
un groupe cycloalkyle en C₃-C₇ ou un groupe cycloalcényle en C₅-C₇, tandis que ces groupes peuvent encore porter un à trois restes choisis parmi les groupes hydroxyle, halogène, alkyle en C₁-C₄, alkyle en C₁-C₄, partiellement ou totalement halogéné, alcoxy en C₁-C₄, alkylthio en C₁-C₄, benzylthio, alkyl(C₁-C₄)sulfonyle, alkyl(C₁-C₄)sulfényle et alkyl(C₁-C₄)sulfynyle, un hétérocycle saturé à 5 chaînons, qui contient comme hétéroatomes un ou deux atomes d'oxygène ou de soufre ou un atome d'oxygène et un atome de soufre, et qui peut encore porter un à trois restes choisis parmi les groupes alkyle en C₁-C₄, alkyle en C₁-C₄ partiellement ou totalement halogéné, alcoxy en C₁-C₄ et alkylthio en C₁-C₄,
un hétérocycle à 6 ou 7 chaînons, sature ou ayant une ou deux insaturations, qui contient comme hétéroatomes un ou deux atome d'oxygène ou de soufre ou un atome d'oxygène et un atome de soufre, et qui peut encore porter un à trois restes choisis parmi les groupes hydroxyle, halogène, alkyle en C₁-C₄, alkyle en C₁-C₄ partiellement ou totalement halogéné et alcoxy en C₁-C₄, alkylthio en C₁-C₄,
un groupe hétéroaromatique à 5 chaînons, contenant un à trois hétéroatomes, choisis dans le groupe comprenant un ou deux atomes d'azote et un atome d'oxygène ou un atome de soufre, tandis que le groupe hétéroaromatique peut encore porter un à trois restes, choisis parmi les groupes cyano, halogène, alkyle en C₁-C₄, alkyle en C₁-C₄ partiellement ou totalement halogéné, alcoxy en C₁-C₄, alcoxy en C₁-C₄ partiellement ou totalement halogéné, alkylthio en C₁-C₄, alcényle en C₂-C₆, alcényloxy en C₂-C₆, alcynyloxy en C₃-C₆ et alcoxy(C₁-C₄)alcoxy(C₁-C₄),
un groupe phényle ou pyridyle, qui peut encore porter dans chaque cas un à trois restes, choisis parmi les groupes nitro, cyano, formyle, halogène, alkyle en C₁-C₄, alkyle en C₁-C₄ partiellement ou totalement halogéné, alcoxy en C₁-C₄, alcoxy en C₁-C₄ partiellement ou totalement halogéné, alkylthio en C₁-C₄, alcényle en C₃-C₆, alcényloxy en C₃-C₆, alcynyle en C₃-C₆, alcynyloxy en C₃-C₆ et -NR^{k}R^{l}, tandis que R^{k} et R^{l} ont la signification indiquée plus haut;
R^{d}
l'hydrogène, le groupe hydroxyle ou, lorsque R^{c} représente un groupe alkyle en C₁-C₆, un groupe alkyle en C₁-C₆;
R^{e}
l'hydrogène, un halogène, le groupe cyano, un groupe alcoxy(C₁-C₄)carbonyle ou un groupe alkyl(C₁-C₄)cétoxime;
W
une chaîne alkylène en C₁-C₆, alcénylène en C₃-C₆ ou alcynylène en C₃-C₆, qui peuvent encore porter dans chaque cas un à trois restes choisis dans le groupe comportant un à trois substituants alkyle en C₁-C₃, un à trois atomes d'halogène et un substituant méthylène;
une chaîne alkylène en C₃-C₆ ou alcénylène en C₄-C₆, qui peuvent encore porter toutes deux un à trois restes alkyle en C₁-C₃, tandis que dans chaque cas un groupe méthylène des chaînes peut être substitué par un atome d'oxygène ou de soufre, un groupe sulfoxyde ou sulfone ou un groupe -N(Rⁱ)-, tandis que
Rⁱ représente un groupe hydrogène, alkyle en C₁-C₄, alcényle en C₃-C₆ ou alcynyle en C₃-C₆;
R^{f} représente l'hydrogène; le groupe vinyle;
un groupe -CH=CH-Z, tandis que Z désigne un groupe cyano, halogène, un reste alkyle en C₁-C₄, un reste alkyle en C₁-C₄ partiellement ou totalement halogéné, un reste cycloalkyle en C₃-C₆, qui peut à son tour porter encore un à trois substituants, choisis parmi les groupes hydroxyle, halogène, alkyle en C₁-C₄, alkyle en C₁-C₄ partiellement ou totalement halogénés et alcoxy en C₁-C₄;
le reste carbonyle, un reste alcoxy(C₁-C₈)carbonyle, le reste benzyloxycarbonyle, le reste phényle, thiényle ou pyridyle, tandis que ces trois restes aromatiques peuvent encore dans chaque cas porter un à trois substituants, choisis parmi les groupes nitro, cyano, halogène, alkyle en C₁-C₄, alkyle en C₁-C₄ partiellement ou totalement halogéné, alcoxy en C₁-C₄, alcoxy en C₁-C₄ partiellement ou totalement halogéné, alkylthio en C₁-C₄, cycloalkyle en C₃-C₆, tandis que le substituant cycloalkyle peut à son tour encore porter un à trois restes, choisis parmi les groupes halogène, alkyle en C₁-C₄, alkyle en C₁-C₄ partiellement ou totalement halogéné et alcoxy en C₁-C₄;
le groupe éthynyle, qui peut porter l'un des restes suivants: un reste alkyle en C₁-C₄ ou cycloalkyle en C₃-C₆, qui peuvent tous deux porter encore un à trois substituants, choisis parmi les groupes hydroxyle, halogène, alkyle en C₁-C₄, alkyle en C₁-C₄ partiellement ou totalement halogéné et alcoxy en C₁-C₄, ou le reste phényle, thiényle ou pyridyle, tandis que les restes aromatiques peuvent dans chaque cas porter encore un à trois substituants, choisis parmi les groupes nitro, cyano, halogène, alkyle en C₁-C₄, alkyle en C₁-C₄ partiellement ou totalement halogéné, alcoxy en C₁-C₄, alcoxy en C₁-C₄ partiellement ou totalement halogéné et alkylthio en C₁-C₄;
le groupe phényle, un groupe halogénophényle, un groupe dihalogénophényle, un groupe hétéroaromatique à 5 chaînons ayant un à trois hétéroatomes, choisi dans un groupe comprenant un à trois atomes d'azote, et un atome d'oxygène ou un atome de soufre, ou un groupe hétéroaromatique à 6 chaînons ayant un à quatre atomes d'azote, qui ne peuvent pas être simultanément tous voisins, tandis que les groupes phényle et hétéroaryle peuvent encore porter un à trois restes, choisis dans le groupe comportant un groupe nitro, des restes alcoxy en C₁-C₄, des restes alkylthio en C₁-C₄, des restes alcoxy en C₁-C₄ partiellement ou totalement halogénés, des restes Z ou un reste -NR^{k}R^{l}, tandis que
R^{k} représente un groupe hydrogène, alkyle en C₁-C₄, alcényle en C₃-C₆ ou alcynyle en C₃-C₆ et
R^{l} représente un groupe hydrogène, alkyle en C₁-C₄,alcényle en C₃-C₆, alcynyle en C₃-C₆, acyle en C₁-C₆ ou benzoyle, qui peut encore porter un à trois substituants, choisis parmi les groupes nitro, cyano, halogène, alkyle en C₁-C₄, alkyle en C₁-C₄ partiellement ou totalement halogéné, alcoxy en C₁-C₄, et alkylthio en C₁-C₄,
ou
B) le groupe des dérivés d'acide 2-(4-hétéroaryloxy)- ou 2-(4-aryloxy)-phénoxycarboxylique de formule III 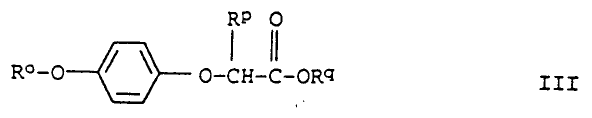 où les substituants ont la signification suivante:
R^{o}
le groupe phényle, le groupe pyridyle, un groupe benzoxazyle, un groupe benzothiazyle ou un groupe benzopyrazinyle, tandis que ces systèmes cycliques aromatiques peuvent porter jusqu'à deux des restes suivants: halogène, nitro, alkyle en C₁-C₄, alkyle en C₁-C₄ partiellement ou totalement halogéné et/ou alcoxy en C₁-C₄ partiellement ou totalement halogéné;
R^{p}
l'hydrogène ou le groupe méthyle;
R^{q}
l'hydrogène, un groupe alkyle en C₁-C₄, un groupe alcényle en C₃-C₄, un groupe alcynyle en C₃-C₄, un groupe alcoxy(C₁-C₄)alkyle(C₁-C₄), un groupe alkylidène(C₃-C₄)iminooxy-alkyle(C₂-C₃), un groupe tétrahydrofurannylméthyle, un groupe isoxazolidine ou l'équivalent d'un cation utilisable en agriculture.

2. Agent herbicide selon la revendication 1, contenant au moins une thiochroménone de formule I, tandis que n a la valeur 1 ou 2.

3. Agent herbicide selon la revendication 1, contenant au moins une thiochroménone de formule I, tandis que R¹ a la signification suivante:
hydrogène; halogène;
alkyle en C₁-C₄, qui peut porter un à cinq atomes d'halogène ou l'un des restes suivants: alcoxy en C₁-C₂, alkylthio en C₁-C₂, phényle, naphtyle, thiényle ou pyridyle, tandis que les restes aromatiques peuvent à leur tour porter un à cinq atomes d'halogène et/ou un à trois des substituants suivants: alkyle en C₁-C₂, halogénoalkyle en C₁-C₂, alcoxy en C₁-C₂, halogénoalcoxy en C₁-C₂, alkylthio en C₁-C₂ et halogénoalkylthio en C₁-C₂;
phényle, naphtyle, thiényle ou pyridyle, tandis que ces restes aromatiques peuvent à leur tour porter un à cinq atomes d'halogène et/ou un à trois des substituants suivants: alkyle en C₁-C₂, halogénoalkyle en C₁-C₂, alcoxy en C₁-C₂, halogénoalcoxy en C₁-C₂, alkylthio en C₁-C₂ et halogénoalkylthio en C₁-C₂;
un groupe -XR⁴ ou un groupe -COYR⁴, tandis que
X et Y représentent l'oxygène et -NR⁵-,
R⁴ représente l'un des groupes suivants:
hydrogène; alkyle en C₁-C₆; cycloalkyle en C₃-C₇;
phényle, naphtyle, thiényle ou pyridyle, tandis que ces restes aromatiques peuvent à leur tour porter un à cinq atomes d'halogène et/ou un à trois des substituants suivants: alkyle en C₁-C₂, halogénoalkyle en C₁-C₂, alcoxy en C₁-C₂, halogénoalcoxy en C₁-C₂, alkylthio en C₁-C₂ et halogénoalkylthio en C₁-C₂;
et
R⁵ représente l'hydrogène ou
un groupe alkyle en C₁-C₆, qui peut porter un groupe hydroxy ou alcoxy en C₁-C₄.

4. Agent herbicide selon la revendication 1, contenant au moins une thiochroménone de formule I, tandis que R² a la signification suivante:
hydrogène; halogène;
alkyle en C₁-C₂, alcoxy en C₁-C₂ ou alkylthio en C₁-C₂, tandis que ces groupes peuvent porter un à cinq atomes d'halogène ou l'un des restes suivants: alcoxy en C₁-C₂, alkylthio en C₁-C₂, phényle, naphtyle, thiényle ou pyridyle, et tandis que les restes aromatiques peuvent à leur tour porter un à cinq atomes d'halogène et/ou un à trois des substituants suivants: alkyle en C₁-C₂, halogénoalkyle en C₁-C₂, alcoxy en C₁-C₂, halogénoalcoxy en C₁-C₂, alkylthio en C₁-C₂ et halogénoalkylthio en C₁-C₂;
phényle, naphtyle, thiényle ou pyridyle, tandis que ces restes aromatiques peuvent à leur tour porter un à cinq atomes d'halogène et/ou un à trois des substituants suivants: alkyle en C₁-C₂, halogénoalkyle en C₁-C₂, alcoxy en C₁-C₂, halogénoalcoxy en C₁-C₂, alkylthio en C₁-C₂ et halogénoalkylthio en C₁-C₂;
un groupe -NR⁴R⁵ ou un groupe -COYR⁴, tandis que
Y désigne l'oxygène ou -NR⁵-,
R⁴ représente l'un des groupes suivants:
hydrogène; alkyle en C₁-C₆; cycloalkyle en C₃-C₇;
phényle, naphtyle, thiényle ou pyridyle, tandis que ces restes aromatiques peuvent à leur tour porter un à cinq atomes d'halogène et/ou un à trois des substituants suivants: alkyle en C₁-C₂, halogénoalkyle en C₁-C₂, alcoxy en C₁-C₂, halogénoalcoxy en C₁-C₂, alkylthio en C₁-C₂ et halogénoalkylthio en C₁-C₂;
et
R⁵ représente l'hydrogène ou un groupe alkyle en C₁-C₆, qui peut porter un groupe hydroxy ou alcoxy en C₁-C₄.

5. Agent herbicide selon la revendication 1, contenant au moins une thiochroménone de formule I, tandis que R³ a la signification suivante:
hydrogène; halogène;
alkyle en C₁-C₄, qui peut porter un à cinq atomes d'halogène ou l'un des restes suivants: alcoxy en C₁-C₂, alkylthio en C₁-C₂, phényle, naphtyle, thiényle ou pyridyle, tandis que les restes aromatiques peuvent à leur tour porter un à cinq atomes d'halogène et/ou un à trois des substituants suivants: alkyle en C₁-C₂, halogénoalkyle en C₁-C₂, alcoxy en C₁-C₂, halogénoalcoxy en C₁-C₂, alkylthio en C₁-C₂ et halogénoalkylthio en C₁-C₂;
un groupe -YR⁴, un groupe -COYR⁴, un groupe -COR⁶ ou un groupe -SO₂R⁷, tandis que
Y désigne l'oxygène ou -NR⁵-;
R⁴ représente l'un des groupes suivants:
hydrogène; alkyle en C₁-C₆; cycloalkyle en C₃-C₇;
phényle, naphtyle, thiényle ou pyridyle, tandis que ces restes aromatiques peuvent à leur tour porter un à cinq atomes d'halogène et/ou un à trois des substituants suivants: alkyle en C₁-C₂, halogénoalkyle en C₁-C₂, alcoxy en C₁-C₂, halogénoalcoxy en C₁-C₂, alkylthio en C₁-C₂ et halogénoalkylthio en C₁-C₂;
R⁵ désigne l'hydrogène ou un groupe alkyle en C₁-C₆, qui peut porter un groupe hydroxy ou alcoxy en C₁-C₄;
R⁶ représente l'un des groupes suivants:
hydrogène; alkyle en C₁-C₆; cycloalkyle en C₃-C₇;
phényle, naphtyle, thiényle ou pyridyle, tandis que ces restes aromatiques peuvent à leur tour porter un à cinq atomes d'halogène et/ou un à trois des substituants suivants: alkyle en C₁-C₂, halogénoalkyle en C₁-C₂, alcoxy en C₁-C₂, halogénoalcoxy en C₁-C₂, alkylthio en C₁-C₂ et halogénoalkylthio en C₁-C₂;
et
R⁷ représente un groupe hydrogène; alkyle en C₁-C₆; cycloalkyle en C₃-C₇;
phényle, naphtyle, thiényle ou pyridyle, tandis que ces restes aromatiques peuvent à leur tour porter un à cinq atomes d'halogène et/ou un à trois des substituants suivants: alkyle en C₁-C₂, halogénoalkyle en C₁-C₂, alcoxy en C₁-C₂, halogénoalcoxy en C₁-C₂, alkylthio en C₁-C₂ et halogénoalkylthio en C₁-C₂;
ou un groupe -NR⁴R⁵, où les restes R⁴ et R⁵ ont la signification indiquée plus haut.

6. Agent herbicide selon la revendication 1, contenant au moins une thiochroménone de formule I, tandis que l'indicce n a la valeur 1 ou 2 et les substituants ont la signification suivante:
R¹
hydrogène; halogène;
alkyle en C₁-C₂, qui peut porter un à cinq atomes d'halogène ou un groupe alcoxy en C₁-C₂;
phényle, qui peut porter un à cinq atomes d'halogène et/ou un à trois des substituants suivants: alkyle en C₁-C₂, alcoxy en C₁-C₂ et alkylthio en C₁-C₂;
un groupe -XR⁴ ou un groupe -COYR⁴, tandis que
X et Y désignent l'oxygène ou -NR⁵-,
R⁴ représente l'un des groupes suivants:
hydrogène; alkyle en C₁-C₆; cycloalkyle en C₃-C₇;
phényle, qui peut porter un à cinq atomes d'halogène et/ou un à trois des substituants suivants: alkyle en C₁-C₂, alcoxy en C₁-C₂ et alkylthio en C₁-C₂;
et
R⁵ désigne l'hydrogène ou un groupe alkyle en C₁-C₆;
R²
hydrogène; halogène;
alkyle en C₁-C₂ ou alcoxy en C₁-C₂, tandis que ces groupes peuvent porter un à cinq atomes d'halogène ou un reste alcoxy en C₁-C₂;
phényle, qui peut porter un à cinq atomes d'halogène et/ou un à trois des substituants suivants: alkyle en C₁-C₂, alcoxy en C₁-C₂ et alkylthio en C₁-C₂;
un groupe -NR⁴R⁵ ou un groupe -COYR⁴, tandis que
Y, R⁴ et R⁵ ont la signification indiquée plus haut,
R³
hydrogène; halogène;
alkyle en C₁-C₄, qui peut porter un à cinq atomes d'halogène ou un reste alcoxy en C₁-C₂;
un groupe -YR⁴, un groupe -COYR⁴, un groupe -COR⁶ ou un groupe -SO₂R⁷, tandis que
Y et R⁴ ont la signification indiquée plus haut, et
R⁶ et R⁷ représentent, indépendamment l'un de l'autre, les groupes suivants:
alkyle en C₁-C₆; cycloalkyle en C₃-C₇;
phényle, qui peut porter un à cinq atomes d'halogène et/ou un à trois des substituants suivants: alkyle en C₁-C₂, alcoxy en C₁-C₂ et alkylthio en C₁-C₂.

7. Procédé pour lutter contre la croissance des plantes parasites, caractérisé par le fait qu'on met en oeuvre au moins une thiochroménone substituée de formule I selon la revendication 1 et au moins
A) un dérivé de cyclohexénone de formule II selon la revendication 1 ou
B) un dérivé d'acide 2-(4-hétéroaryloxy)- ou 2-(4-aryloxy)-phénoxycarboxylique de formule III selon la revendication 1,
avant, pendant ou après l'ensemencement des plantes cultivées, avant ou pendant la croissance des plantes cultivées, simultanément ou séparément.

8. Procédé pour lutter sélectivement contre la croissance des plantes parasites, caractérisé par le fait qu'on traite les feuilles des plantes cultivées et des plantes parasites dans le procédé en post-levée, avec au moins une thiochroménone substituée de formule I selon la revendication 1 et au moins
A) un dérivé de cyclohexénone de formule II selon la revendication 1 ou
B) un dérivé d'acide 2-(4-hétéroaryloxy)- ou 2-(4-aryloxy)-phénoxycarboxylique de formule III selon la revendication 1,
simultanément ou séparément.

9. Procédé pour empêcher que soient endommagées les plantes cultivées, par
A) des dérivés herbicides de cyclohexénone de formule II selon la revendication 1 ou
B) un dérivé herbicide d'acide 2-(4-hétéroaryloxy)- ou 2-(4-aryloxy)-phénoxycarboxylique de formule III selon la revendication 1,
caractérisé par le fait qu'on traite les semences des plantes cultivées avec une quantité à activité antagoniste d'une thiochroménone de formule I selon la revendication 1.

10. Procédé selon l'une quelconque des revendications 7 à 9, caractérisé par le fait que les plantes cultivées sont de l'orge, du blé, du mais, du sorgho de culture et du riz.
